(19) Europäisches Patentamt
European Patent Office
Office européen des brevets

(11) EP 3 783 107 A1

(12) EUROPEAN PATENT APPLICATION

(43) Date of publication:
24.02.2021 Bulletin 2021/08

(51) Int Cl.:
*C12Q 1/18* (2006.01) *G01N 33/94* (2006.01)

(21) Application number: 19193190.6

(22) Date of filing: 22.08.2019

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(71) Applicant: Université de Liège
4000 Liège (BE)

(72) Inventors:
- Joris, Bernard
4141 Louveigné (BE)
- Amoroso, Ana
4141 Louveigné (BE)
- Verlaine, Olivier
4000 Liège (BE)

(74) Representative: De Clercq & Partners
Edgard Gevaertdreef 10a
9830 Sint-Martens-Latem (BE)

# (54) METHODS FOR DETERMINING THE CONCENTRATION OF A CARBAPENEM ANTIBIOTIC IN A BIOLOGICAL SAMPLE

(57) The present invention relates to methods for determining the concentration of a carbapenem antibiotic in a biological sample. In particular, the present invention relates to methods for determining the concentration of a carbapenem antibiotic in a biological sample, comprising the steps of: (a) providing a blaOXA-48 class D beta-lactamase (blaOXA-48) or a functionally active variant or fragment thereof; (b) providing a biological sample; (c) contacting the blaOXA-48 or functionally active variant or fragment thereof with the biological sample; and (d) determining the concentration of the carbapenem antibiotic in the biological sample.

EP 3 783 107 A1

SEQUENCE LISTING

<110> Université de Liège

<120> METHODS FOR DETERMINING THE CONCENTRATION OF A CARBAPENEM ANTIBIOTIC IN A BIOLOGICAL SAMPLE

<130> ULG-025

<160> 56

<170> PatentIn version 3.5

<210> 1
<211> 265
<212> PRT
<213> Klebsiella pneumoniae

<220>
<221> PEPTIDE
<222> (1)..(22)
<223> SIGNAL PEPTIDE

<400> 1

```
Met Arg Val Leu Ala Leu Ser Ala Val Phe Leu Val Ala Ser Ile Ile
1               5                   10                  15

Gly Met Pro Ala Val Ala Lys Glu Trp Gln Glu Asn Lys Ser Trp Asn
            20                  25                  30

Ala His Phe Thr Glu His Lys Ser Gln Gly Val Val Val Leu Trp Asn
        35                  40                  45

Glu Asn Lys Gln Gln Gly Phe Thr Asn Asn Leu Lys Arg Ala Asn Gln
    50                  55                  60

Ala Phe Leu Pro Ala Ser Thr Phe Lys Ile Pro Asn Ser Leu Ile Ala
65                  70                  75                  80

Leu Asp Leu Gly Val Val Lys Asp Glu His Gln Val Phe Lys Trp Asp
                85                  90                  95

Gly Gln Thr Arg Asp Ile Ala Thr Trp Asn Arg Asp His Asn Leu Ile
            100                 105                 110

Thr Ala Met Lys Tyr Ser Val Val Pro Val Tyr Gln Glu Phe Ala Arg
        115                 120                 125

Gln Ile Gly Glu Ala Arg Met Ser Lys Met Leu His Ala Phe Asp Tyr
    130                 135                 140

Gly Asn Glu Asp Ile Ser Gly Asn Val Asp Ser Phe Trp Leu Asp Gly
```

145 150 155 160

Gly Ile Arg Ile Ser Ala Thr Glu Gln Ile Ser Phe Leu Arg Lys Leu
165 170 175

Tyr His Asn Lys Leu His Val Ser Glu Arg Ser Gln Arg Ile Val Lys
180 185 190

Gln Ala Met Leu Thr Glu Ala Asn Gly Asp Tyr Ile Ile Arg Ala Lys
195 200 205

Thr Gly Tyr Ser Thr Arg Ile Glu Pro Lys Ile Gly Trp Trp Val Gly
210 215 220

Trp Val Glu Leu Asp Asp Asn Val Trp Phe Phe Ala Met Asn Met Asp
225 230 235 240

Met Pro Thr Ser Asp Gly Leu Gly Leu Arg Gln Ala Ile Thr Lys Glu
245 250 255

Val Leu Lys Gln Glu Lys Ile Ile Pro
260 265

<210> 2
<211> 265
<212> PRT
<213> Shewanella oneidensis

<220>
<221> PEPTIDE
<222> (1)..(22)
<223> SIGNAL PEPTIDE

<400> 2

Met Arg Val Leu Ala Leu Ser Ala Val Leu Val Val Ala Ser Ile Val
1 5 10 15

Gly Met Pro Ala Met Ala Asn Glu Trp Gln Glu Lys Pro Ser Trp Asn
20 25 30

Thr His Phe Ser Glu His Lys Ala Gln Gly Val Ile Val Leu Trp Asn
35 40 45

Glu Asn Lys Gln Gln Gly Phe Thr Asn Asn Leu Lys Arg Ala Asn Gln
50 55 60

Ala Phe Leu Pro Ala Ser Thr Phe Lys Ile Pro Asn Ser Leu Ile Ala
65 70 75 80

```
Leu Asp Leu Gly Val Val Lys Asp Glu His Gln Val Phe Lys Trp Asp
                85                  90                  95

Gly Gln Thr Arg Asp Ile Ala Ala Trp Asn Arg Asp His Asp Leu Ile
            100                 105                 110

Thr Ala Met Lys Tyr Ser Val Val Pro Val Tyr Gln Glu Phe Ala Arg
        115                 120                 125

Gln Ile Gly Gln Ala Arg Met Ser Lys Met Leu His Ala Phe Asp Tyr
    130                 135                 140

Gly Asn Glu Asp Ile Ser Gly Asn Leu Asp Ser Phe Trp Leu Asp Gly
145                 150                 155                 160

Gly Ile Arg Ile Ser Ala Thr Glu Gln Val Ala Phe Leu Arg Lys Leu
                165                 170                 175

Tyr His Asn Lys Leu His Val Ser Glu Arg Ser Gln Arg Ile Val Lys
            180                 185                 190

Gln Ala Met Leu Thr Glu Ala Asn Ser Asp Tyr Ile Ile Arg Ala Lys
        195                 200                 205

Thr Gly Tyr Ser Thr Arg Ile Glu Pro Gln Ile Gly Trp Trp Val Gly
    210                 215                 220

Trp Val Glu Leu Asp Asp Asn Val Trp Phe Phe Ala Met Asn Met Asp
225                 230                 235                 240

Met Pro Thr Ala Asp Gly Leu Gly Leu Arg Gln Ala Ile Thr Lys Glu
                245                 250                 255

Val Leu Lys Gln Glu Lys Ile Ile Pro
            260                 265
```

<210> 3
<211> 265
<212> PRT
<213> Klebsiella pneumoniae

<220>
<221> PEPTIDE
<222> (1)..(22)
<223> SIGNAL PEPTIDE

<400> 3

```
Met Arg Val Leu Ala Leu Ser Ala Val Phe Leu Val Ala Ser Ile Ile
```

```
1               5                   10                  15

Gly Met Pro Ala Val Ala Lys Glu Trp Gln Glu Asn Lys Ser Trp Asn
            20                  25                  30

Ala His Phe Thr Glu His Lys Ser Gln Gly Val Val Val Leu Trp Asn
        35                  40                  45

Glu Asn Lys Gln Gln Gly Phe Thr Asn Asn Leu Lys Arg Ala Asn Gln
    50                  55                  60

Ala Phe Leu Pro Ala Ser Thr Phe Lys Ile Pro Asn Ser Leu Ile Ala
65                  70                  75                  80

Leu Asp Leu Gly Val Val Lys Asp Glu His Gln Val Phe Lys Trp Asp
                85                  90                  95

Gly Gln Thr Arg Asp Ile Ala Thr Trp Asn Arg Asp His Asn Leu Ile
            100                 105                 110

Thr Ala Met Lys Tyr Ser Val Val Pro Val Tyr Gln Glu Phe Ala Arg
        115                 120                 125

Gln Ile Gly Glu Ala Arg Met Ser Lys Met Leu His Ala Phe Asp Tyr
    130                 135                 140

Gly Asn Glu Asp Ile Ser Gly Asn Val Asp Ser Phe Trp Leu Asp Gly
145                 150                 155                 160

Gly Ile Arg Ile Ser Ala Thr Glu Gln Ile Ser Phe Leu Arg Lys Leu
                165                 170                 175

Tyr His Asn Lys Leu His Val Ser Glu Arg Ser Gln Arg Ile Val Lys
            180                 185                 190

Gln Ala Met Leu Thr Glu Ala Asn Gly Asp Tyr Ile Ile Arg Ala Lys
        195                 200                 205

Thr Gly Tyr Ser Ala Arg Ile Glu Pro Lys Ile Gly Trp Trp Val Gly
    210                 215                 220

Trp Val Glu Leu Asp Asp Asn Val Trp Phe Phe Ala Met Asn Met Asp
225                 230                 235                 240

Met Pro Thr Ser Asp Gly Leu Gly Leu Arg Gln Ala Ile Thr Lys Glu
                245                 250                 255
```

```
Val Leu Lys Gln Glu Lys Ile Ile Pro
            260                 265


<210>  4
<211>  261
<212>  PRT
<213>  Enterobacter cloacae


<220>
<221>  PEPTIDE
<222>  (1)..(22)
<223>  SIGNAL PEPTIDE

<400>  4

Met Arg Val Leu Ala Leu Ser Ala Val Phe Leu Val Ala Ser Ile Ile
1               5                   10                  15


Gly Met Pro Ala Val Ala Lys Glu Trp Gln Glu Asn Lys Ser Trp Asn
            20                  25                  30


Ala His Phe Thr Glu His Lys Ser Gln Gly Val Val Val Leu Trp Asn
        35                  40                  45


Glu Asn Lys Gln Gln Gly Phe Thr Asn Asn Leu Lys Arg Ala Asn Gln
    50                  55                  60


Ala Phe Leu Pro Ala Ser Thr Phe Lys Ile Pro Asn Ser Leu Ile Ala
65                  70                  75                  80


Leu Asp Leu Gly Val Val Lys Asp Glu His Gln Val Phe Lys Trp Asp
                85                  90                  95


Gly Gln Thr Arg Asp Ile Ala Thr Trp Asn Arg Asp His Asn Leu Ile
            100                 105                 110


Thr Ala Met Lys Tyr Ser Val Val Pro Val Tyr Gln Glu Phe Ala Arg
        115                 120                 125


Gln Ile Gly Glu Ala Arg Met Ser Lys Met Leu His Ala Phe Asp Tyr
    130                 135                 140


Gly Asn Glu Asp Ile Ser Gly Asn Val Asp Ser Phe Trp Leu Asp Gly
145                 150                 155                 160


Gly Ile Arg Ile Ser Ala Thr Glu Gln Ile Ser Phe Leu Arg Lys Leu
                165                 170                 175


Tyr His Asn Lys Leu His Val Ser Glu Arg Ser Gln Arg Ile Val Lys
            180                 185                 190
```

```
Gln Ala Met Leu Thr Glu Ala Asn Gly Asp Tyr Ile Ile Arg Ala Lys
        195                 200                 205

Thr Gly Tyr Asp Thr Lys Ile Gly Trp Trp Val Gly Trp Val Glu Leu
    210                 215                 220

Asp Asp Asn Val Trp Phe Phe Ala Met Asn Met Asp Met Pro Thr Ser
225                 230                 235                 240

Asp Gly Leu Gly Leu Arg Gln Ala Ile Thr Lys Glu Val Leu Lys Gln
                245                 250                 255

Glu Lys Ile Ile Pro
            260

<210>  5
<211>  265
<212>  PRT
<213>  Klebsiella pneumoniae

<220>
<221>  PEPTIDE
<222>  (1)..(22)
<223>  SIGNAL PEPTIDE

<400>  5

Met Arg Val Leu Ala Leu Ser Ala Val Phe Leu Val Ala Ser Ile Ile
1               5                   10                  15

Gly Met Pro Ala Val Ala Lys Glu Trp Gln Glu Asn Lys Ser Trp Asn
            20                  25                  30

Ala His Phe Thr Glu His Lys Ser Gln Gly Val Val Val Leu Trp Asn
        35                  40                  45

Glu Asn Lys Gln Gln Gly Phe Thr Asn Asn Leu Lys Arg Ala Asn Gln
    50                  55                  60

Ala Phe Leu Pro Ala Ser Thr Phe Lys Ile Pro Asn Ser Leu Ile Ala
65                  70                  75                  80

Leu Asp Leu Gly Val Val Lys Asp Glu His Gln Val Phe Lys Trp Asp
                85                  90                  95

Gly Gln Thr Arg Asp Ile Ala Ala Trp Asn Arg Asp His Asp Leu Ile
            100                 105                 110
```

Thr Ala Met Lys Tyr Ser Val Val Pro Val Tyr Gln Glu Phe Ala Arg
        115                 120                 125

Gln Ile Gly Glu Ala Arg Met Ser Lys Met Leu His Ala Phe Asp Tyr
    130                 135                 140

Gly Asn Glu Asp Ile Ser Gly Asn Val Asp Ser Phe Trp Leu Asp Gly
145                 150                 155                 160

Gly Ile Arg Ile Ser Ala Thr Gln Gln Ile Ala Phe Leu Arg Lys Leu
                165                 170                 175

Tyr His Asn Lys Leu His Val Ser Glu Arg Ser Gln Arg Ile Val Lys
            180                 185                 190

Gln Ala Met Leu Thr Glu Ala Asn Gly Asp Tyr Ile Ile Arg Ala Lys
        195                 200                 205

Thr Gly Tyr Ser Thr Arg Ile Glu Pro Lys Ile Gly Trp Trp Val Gly
    210                 215                 220

Trp Val Glu Leu Asp Asp Asn Val Trp Phe Phe Ala Met Asn Met Asp
225                 230                 235                 240

Met Pro Thr Ser Asp Gly Leu Gly Leu Arg Gln Ala Ile Thr Lys Glu
                245                 250                 255

Val Leu Lys Gln Glu Lys Ile Ile Pro
            260                 265

<210> 6
<211> 265
<212> PRT
<213> Shewanella xiamenensis

<220>
<221> PEPTIDE
<222> (1)..(22)
<223> SIGNAL PEPTIDE

<400> 6

Met Arg Val Leu Ala Leu Ser Ala Val Phe Leu Val Ala Ser Ile Ile
1               5                   10                  15

Gly Met Pro Ala Val Ala Lys Glu Trp Gln Glu Asn Lys Ser Trp Asn
            20                  25                  30

Ala His Phe Thr Glu Tyr Lys Ser Gln Gly Val Val Ala Leu Trp Asn
        35                  40                  45

Glu Asn Lys Gln Gln Gly Phe Thr Asn Asn Leu Lys Arg Ala Asn Gln
    50                  55                  60

Ala Phe Leu Pro Ala Ser Thr Phe Lys Ile Pro Asn Ser Leu Ile Ala
65                  70                  75                  80

Leu Asp Leu Gly Val Val Lys Asp Glu His Gln Val Phe Lys Trp Asp
                85                  90                  95

Gly Gln Thr Arg Asp Ile Ala Thr Trp Asn Arg Asp His Asn Leu Ile
            100                 105                 110

Thr Ala Met Lys Tyr Ser Val Val Pro Val Tyr Gln Glu Phe Ala Arg
        115                 120                 125

Gln Ile Gly Glu Ala Arg Met Ser Lys Met Leu His Ala Phe Asp Tyr
    130                 135                 140

Gly Asn Glu Asp Ile Ser Gly Asn Val Gly Ser Phe Trp Leu Asp Gly
145                 150                 155                 160

Gly Ile Arg Ile Ser Ala Thr Glu Gln Ile Ser Phe Leu Arg Lys Leu
                165                 170                 175

Tyr His Asn Lys Leu His Val Ser Glu Arg Ser Gln Arg Ile Val Lys
            180                 185                 190

Gln Ala Met Leu Thr Glu Ala Asn Gly Asp Tyr Ile Ile Arg Ala Lys
        195                 200                 205

Thr Gly Tyr Ser Thr Arg Ile Glu Pro Lys Ile Gly Trp Trp Val Gly
    210                 215                 220

Trp Val Glu Leu Asp Asp Asn Val Trp Phe Phe Ala Met Asn Met Asp
225                 230                 235                 240

Met Pro Thr Ser Asp Gly Leu Gly Leu Arg Gln Ala Ile Thr Lys Glu
                245                 250                 255

Val Leu Lys Gln Glu Lys Ile Ile Pro
            260                 265

<210> 7
<211> 265
<212> PRT
<213> Klebsiella pneumoniae

<220>
<221> PEPTIDE
<222> (1)..(22)
<223> SIGNAL PEPTIDE

<400> 7

```
Met Arg Val Leu Ala Leu Ser Ala Val Phe Leu Val Ala Ser Ile Ile
1               5                   10                  15

Gly Met Pro Ala Val Ala Lys Glu Trp Gln Glu Asn Lys Ser Trp Asn
            20                  25                  30

Ala His Phe Thr Glu His Lys Ser Gln Gly Val Val Val Leu Trp Asn
        35                  40                  45

Glu Asn Lys Gln Gln Gly Phe Thr Asn Asn Leu Lys Arg Ala Asn Gln
    50                  55                  60

Ala Phe Leu Pro Ala Ser Thr Phe Lys Ile Pro Asn Ser Leu Ile Ala
65                  70                  75                  80

Leu Asp Leu Gly Val Val Lys Asp Glu His Gln Val Phe Lys Trp Asp
                85                  90                  95

Gly His Arg Arg Asp Ile Ala Thr Trp Asn Arg Asp His Asn Leu Ile
            100                 105                 110

Thr Ala Met Lys Tyr Ser Val Val Pro Val Tyr Gln Glu Phe Ala Arg
        115                 120                 125

Gln Ile Gly Glu Ala Arg Met Ser Lys Met Leu His Ala Phe Asp Tyr
    130                 135                 140

Gly Asn Glu Asp Ile Ser Gly Asn Val Asp Ser Phe Trp Leu Asp Gly
145                 150                 155                 160

Gly Ile Arg Ile Ser Ala Thr Glu Gln Ile Ser Phe Leu Arg Lys Leu
                165                 170                 175

Tyr His Asn Lys Leu His Val Ser Glu Arg Ser Gln Arg Ile Val Lys
            180                 185                 190

Gln Ala Met Leu Thr Glu Ala Asn Gly Asp Tyr Ile Ile Arg Ala Lys
        195                 200                 205

Thr Gly Tyr Ser Thr Arg Ile Glu Pro Lys Ile Gly Trp Trp Val Gly
    210                 215                 220
```

Trp Val Glu Leu Asp Asp Asn Val Trp Phe Phe Ala Met Asn Met Asp
225 230 235 240

Met Pro Thr Ser Asp Gly Leu Gly Leu Arg Gln Ala Ile Thr Lys Glu
245 250 255

Val Leu Lys Gln Glu Lys Ile Ile Pro
260 265

<210> 8
<211> 265
<212> PRT
<213> Klebsiella pneumoniae

<220>
<221> PEPTIDE
<222> (1)..(22)
<223> SIGNAL PEPTIDE

<400> 8

Met Arg Val Leu Ala Leu Ser Ala Val Phe Leu Val Ala Ser Ile Ile
1 5 10 15

Gly Met Pro Ala Val Ala Lys Glu Trp Gln Glu Asn Lys Ser Trp Asn
20 25 30

Ala His Phe Thr Glu His Lys Ser Gln Gly Val Val Val Leu Trp Asn
35 40 45

Glu Asn Lys Gln Gln Gly Phe Thr Asn Asn Leu Lys Arg Ala Asn Gln
50 55 60

Ala Phe Leu Pro Ala Ser Thr Phe Lys Ile Pro Asn Ser Leu Ile Ala
65 70 75 80

Leu Asp Leu Gly Val Val Lys Asp Glu His Gln Val Phe Lys Trp Asp
85 90 95

Gly Gln Thr Arg Asp Ile Ala Thr Trp Asn Arg Asp His Asn Leu Ile
100 105 110

Thr Ala Met Lys Tyr Ser Val Val Pro Val Tyr Gln Glu Phe Ala Arg
115 120 125

Gln Ile Gly Glu Ala Arg Met Ser Lys Met Leu His Ala Phe Asp Tyr
130 135 140

Gly Asn Glu Asp Ile Ser Gly Asn Val Asp Ser Phe Trp Leu Asp Gly

145 150 155 160

Gly Ile Arg Ile Ser Ala Thr Glu Gln Ile Ser Phe Leu Arg Lys Leu
165 170 175

Tyr His Asn Lys Leu His Val Ser Glu Arg Ser Gln Arg Ile Val Lys
180 185 190

Gln Ala Met Leu Thr Glu Ala Asn Gly Asp Tyr Ile Ile Arg Ala Lys
195 200 205

Thr Gly Tyr Ser Thr Gly Ile Glu Pro Lys Ile Gly Trp Trp Val Gly
210 215 220

Trp Val Glu Leu Asp Asp Asn Val Trp Phe Phe Ala Met Asn Met Asp
225 230 235 240

Met Pro Thr Ser Asp Gly Leu Gly Leu Arg Gln Ala Ile Thr Lys Glu
245 250 255

Val Leu Lys Gln Glu Lys Ile Ile Pro
260 265

<210> 9
<211> 265
<212> PRT
<213> Klebsiella pneumoniae

<220>
<221> PEPTIDE
<222> (1)..(22)
<223> SIGNAL PEPTIDE

<400> 9

Met Arg Val Leu Ala Leu Ser Ala Val Phe Leu Val Ala Ser Ile Ile
1 5 10 15

Gly Met Pro Ala Val Ala Lys Glu Trp Gln Glu Asn Lys Ser Trp Asn
20 25 30

Ala His Phe Thr Glu His Lys Ser Gln Gly Val Val Val Leu Trp Asn
35 40 45

Glu Asn Lys Gln Gln Gly Phe Thr Asn Asn Leu Lys Arg Ala Asn Gln
50 55 60

Ala Phe Leu Pro Ala Ser Thr Phe Lys Ile Pro Asn Ser Leu Ile Ala
65 70 75 80

```
Leu Asp Leu Gly Val Val Lys Asp Glu His Gln Val Phe Lys Trp Asp
                85                  90                  95

Gly Gln Thr Arg Asp Ile Ala Thr Trp Asn Arg Asp His Asn Leu Ile
            100                 105                 110

Thr Ala Met Lys Tyr Ser Val Val Pro Val Tyr Gln Tyr Phe Ala Arg
        115                 120                 125

Gln Ile Gly Glu Ala Arg Met Ser Lys Met Leu His Ala Phe Asp Tyr
    130                 135                 140

Gly Asn Glu Asp Ile Ser Gly Asn Val Asp Ser Phe Trp Leu Asp Gly
145                 150                 155                 160

Gly Ile Arg Ile Ser Ala Thr Glu Gln Ile Ser Phe Leu Arg Lys Leu
                165                 170                 175

Tyr His Asn Lys Leu His Val Ser Glu Arg Ser Gln Arg Ile Val Lys
            180                 185                 190

Gln Ala Met Leu Thr Glu Ala Asn Gly Asp Tyr Ile Ile Arg Ala Lys
        195                 200                 205

Thr Gly Tyr Ser Thr Arg Ile Glu Pro Lys Ile Gly Trp Trp Val Gly
    210                 215                 220

Trp Val Glu Leu Asp Asp Asn Val Trp Phe Phe Ala Met Asn Met Asp
225                 230                 235                 240

Met Pro Thr Ser Asp Gly Leu Gly Leu Arg Gln Ala Ile Thr Lys Glu
                245                 250                 255

Val Leu Lys Gln Glu Lys Ile Ile Pro
            260                 265
```

<210> 10
<211> 261
<212> PRT
<213> Klebsiella pneumoniae

<220>
<221> PEPTIDE
<222> (1)..(22)
<223> SIGNAL PEPTIDE

<400> 10

```
Met Arg Val Leu Ala Leu Ser Ala Val Phe Leu Val Ala Ser Ile Ile
```

```
1               5                   10                  15

Gly Met Pro Ala Val Ala Lys Glu Trp Gln Glu Asn Lys Ser Trp Asn
            20                  25                  30

Ala His Phe Thr Glu His Lys Ser Gln Gly Val Val Val Leu Trp Asn
        35                  40                  45

Glu Asn Lys Gln Gln Gly Phe Thr Asn Asn Leu Lys Arg Ala Asn Gln
    50                  55                  60

Ala Phe Leu Pro Ala Ser Thr Phe Lys Ile Pro Asn Ser Leu Ile Ala
65                  70                  75                  80

Leu Asp Leu Gly Val Val Lys Asp Glu His Gln Val Phe Lys Trp Asp
                85                  90                  95

Gly Gln Thr Arg Asp Ile Ala Thr Trp Asn Arg Asp His Asn Leu Ile
            100                 105                 110

Thr Ala Met Lys Tyr Ser Val Val Pro Val Tyr Gln Glu Phe Ala Arg
        115                 120                 125

Gln Ile Gly Glu Ala Arg Met Ser Lys Met Leu His Ala Phe Asp Tyr
    130                 135                 140

Gly Asn Glu Asp Ile Ser Gly Asn Val Asp Ser Phe Trp Leu Asp Gly
145                 150                 155                 160

Gly Ile Arg Ile Ser Ala Thr Glu Gln Ile Ser Phe Leu Arg Lys Leu
                165                 170                 175

Tyr His Asn Lys Leu His Val Ser Glu Arg Ser Gln Arg Ile Val Lys
            180                 185                 190

Gln Ala Met Leu Thr Glu Ala Asn Gly Asp Tyr Ile Ile Arg Ala Lys
        195                 200                 205

Thr Gly Ser Asn Thr Lys Ile Gly Trp Trp Val Gly Trp Val Glu Leu
    210                 215                 220

Asp Asp Asn Val Trp Phe Phe Ala Met Asn Met Asp Met Pro Thr Ser
225                 230                 235                 240

Asp Gly Leu Gly Leu Arg Gln Ala Ile Thr Lys Glu Val Leu Lys Gln
                245                 250                 255
```

```
Glu Lys Ile Ile Pro
            260


<210>  11
<211>  265
<212>  PRT
<213>  Escherichia coli


<220>
<221>  PEPTIDE
<222>  (1)..(22)
<223>  SIGNAL PEPTIDE

<400>  11

Met Arg Val Leu Ala Leu Ser Ala Val Phe Leu Val Ala Ser Ile Ile
1               5                   10                  15

Gly Met Pro Ala Val Ala Lys Glu Trp Gln Glu Asn Lys Ser Trp Asn
            20                  25                  30

Ala His Phe Thr Glu His Lys Ser Gln Gly Val Val Val Leu Trp Asn
        35                  40                  45

Glu Asn Lys Gln Gln Gly Phe Thr Asn Asn Leu Lys Arg Ala Asn Gln
    50                  55                  60

Ala Phe Leu Pro Ala Ser Thr Phe Lys Ile Pro Asn Ser Leu Ile Ala
65                  70                  75                  80

Leu Asp Leu Gly Val Val Lys Asp Glu His Gln Val Phe Lys Trp Asp
                85                  90                  95

Gly Gln Thr Arg Asp Ile Ala Ala Trp Asn Arg Asp His Asp Leu Ile
            100                 105                 110

Thr Ala Met Lys Tyr Ser Val Val Pro Val Tyr Gln Glu Phe Ala Arg
        115                 120                 125

Gln Ile Gly Glu Ala Arg Met Ser Lys Met Leu His Ala Phe Asp Tyr
    130                 135                 140

Gly Asn Glu Asp Ile Ser Gly Asn Val Asp Ser Phe Trp Leu Asp Gly
145                 150                 155                 160

Gly Ile Arg Ile Ser Ala Thr Gln Gln Ile Ala Phe Leu Arg Lys Leu
                165                 170                 175

Tyr His Asn Lys Leu His Val Ser Glu Arg Ser Gln Arg Ile Val Lys
            180                 185                 190
```

Gln Ala Met Leu Thr Glu Ala Asn Gly Asp Tyr Ile Ile Arg Ala Lys
        195                 200                 205

Thr Gly Tyr Ser Thr Ser Ile Glu Pro Lys Ile Gly Trp Trp Val Gly
    210                 215                 220

Trp Val Glu Leu Asp Asp Asn Val Trp Phe Phe Ala Met Asn Met Asp
225                 230                 235                 240

Met Pro Thr Ser Asp Gly Leu Gly Leu Arg Gln Ala Ile Thr Lys Glu
                245                 250                 255

Val Leu Lys Gln Glu Lys Ile Ile Pro
            260                 265

<210> 12
<211> 265
<212> PRT
<213> Enterobacter sp. 87F-2

<220>
<221> PEPTIDE
<222> (1)..(22)
<223> SIGNAL PEPTIDE

<400> 12

Met Arg Val Leu Ala Leu Ser Ala Val Phe Leu Val Ala Ser Ile Ile
1               5                   10                  15

Gly Met Pro Ala Val Ala Lys Glu Trp Gln Glu Asn Lys Ser Trp Asn
            20                  25                  30

Ala His Phe Thr Glu His Lys Ser Gln Gly Val Val Val Leu Trp Asn
        35                  40                  45

Glu Asn Lys Gln Gln Gly Phe Thr Asn Asn Leu Lys Arg Ala Asn Gln
    50                  55                  60

Ala Phe Leu Pro Ala Ser Thr Phe Lys Ile Pro Asn Ser Leu Ile Ala
65                  70                  75                  80

Leu Asp Leu Gly Val Val Lys Asp Glu His Gln Val Phe Lys Trp Asp
                85                  90                  95

Gly Gln Thr Arg Asp Ile Ala Thr Trp Asn Arg Asp His Asn Leu Ile
            100                 105                 110

Thr Ala Met Lys Tyr Ser Val Val Pro Val Tyr Gln Glu Phe Ala Arg
        115                 120                 125

Gln Ile Gly Glu Ala Arg Met Ser Lys Met Leu His Ala Phe Asp Tyr
    130                 135                 140

Gly Asn Glu Asp Ile Ser Gly Asn Val Asp Ser Phe Trp Leu Asp Gly
145                 150                 155                 160

Gly Ile Arg Ile Ser Ala Thr Glu Gln Ile Ser Phe Leu Arg Lys Leu
                165                 170                 175

Tyr His Asn Lys Leu His Val Ser Glu Arg Ser Gln Arg Ile Val Lys
            180                 185                 190

Gln Ala Met Leu Thr Glu Ala Asn Gly Asp Tyr Ile Ile Arg Ala Lys
        195                 200                 205

Thr Gly Tyr Glu Thr Arg Ile Glu Pro Lys Ile Gly Trp Trp Val Gly
    210                 215                 220

Trp Val Glu Leu Asp Asp Asn Val Trp Phe Phe Ala Met Asn Met Asp
225                 230                 235                 240

Met Pro Thr Ser Asp Gly Leu Gly Leu Arg Gln Ala Ile Thr Lys Glu
                245                 250                 255

Val Leu Lys Gln Glu Lys Ile Ile Pro
            260                 265

<210> 13
<211> 261
<212> PRT
<213> Serratia marcescens

<220>
<221> PEPTIDE
<222> (1)..(22)
<223> SIGNAL PEPTIDE

<400> 13

Met Arg Val Leu Ala Leu Ser Ala Val Phe Leu Val Ala Ser Ile Ile
1               5                   10                  15

Gly Met Pro Ala Val Ala Lys Glu Trp Gln Glu Asn Lys Ser Trp Asn
            20                  25                  30

Ala His Phe Thr Glu His Lys Ser Gln Gly Val Val Val Leu Trp Asn
        35                  40                  45

```
Glu Asn Lys Gln Gln Gly Phe Thr Asn Asn Leu Lys Arg Ala Asn Gln
    50                  55                  60

Ala Phe Leu Pro Ala Ser Thr Phe Lys Ile Pro Asn Ser Leu Ile Ala
65                  70                  75                  80

Leu Asp Leu Gly Val Val Lys Asp Glu His Gln Val Phe Lys Trp Asp
                85                  90                  95

Gly Gln Thr Arg Asp Ile Ala Thr Trp Asn Arg Asp His Asn Leu Ile
            100                 105                 110

Thr Ala Met Lys Tyr Ser Val Val Pro Val Tyr Gln Glu Phe Ala Arg
        115                 120                 125

Gln Ile Gly Glu Ala Arg Met Ser Lys Met Leu His Ala Phe Asp Tyr
    130                 135                 140

Gly Asn Glu Asp Ile Ser Gly Asn Val Asp Ser Phe Trp Leu Asp Gly
145                 150                 155                 160

Gly Ile Arg Ile Ser Ala Thr Glu Gln Ile Ser Phe Leu Arg Lys Leu
                165                 170                 175

Tyr His Asn Lys Leu His Val Ser Glu Arg Ser Gln Arg Ile Val Lys
            180                 185                 190

Gln Ala Met Leu Thr Glu Ala Asn Gly Asp Tyr Ile Ile Arg Ala Lys
        195                 200                 205

Thr Gly Tyr Ser Pro Lys Ile Gly Trp Trp Val Gly Trp Val Glu Leu
    210                 215                 220

Asp Asp Asn Val Trp Phe Phe Ala Met Asn Met Asp Met Pro Thr Ser
225                 230                 235                 240

Asp Gly Leu Gly Leu Arg Gln Ala Ile Thr Lys Glu Val Leu Lys Gln
                245                 250                 255

Glu Lys Ile Ile Pro
            260


<210>  14
<211>  265
<212>  PRT
<213>  Shewanella xiamenensis
```

```
<220>
<221>  PEPTIDE
<222>  (1)..(22)
<223>  SIGNAL PEPTIDE

<400>  14

Met Arg Val Leu Ala Leu Ser Ala Val Phe Leu Val Ala Ser Ile Ile
1               5                   10                  15

Gly Met Pro Ala Val Ala Lys Glu Trp Gln Glu Asn Lys Ser Trp Asn
            20                  25                  30

Ala His Phe Thr Glu His Lys Ser Gln Gly Val Val Val Leu Trp Asn
        35                  40                  45

Glu Asn Lys Gln Gln Gly Phe Thr Asn Asn Leu Lys Arg Ala Asn Gln
    50                  55                  60

Ala Phe Leu Pro Ala Ser Thr Phe Lys Ile Pro Asn Ser Leu Ile Ala
65                  70                  75                  80

Leu Asp Leu Gly Val Val Lys Asp Glu His Gln Val Phe Lys Trp Asp
                85                  90                  95

Gly Gln Thr Arg Asp Ile Ala Thr Trp Asn Arg Asp His Asn Leu Ile
            100                 105                 110

Thr Ala Met Lys Tyr Ser Val Val Pro Val Tyr Gln Glu Phe Ala Arg
        115                 120                 125

Gln Ile Gly Glu Ala Arg Met Ser Lys Met Leu His Ala Phe Asp Tyr
    130                 135                 140

Gly Asn Glu Asp Ile Ser Gly Asn Leu Asp Thr Phe Trp Leu Asp Gly
145                 150                 155                 160

Gly Ile Arg Ile Ser Ala Thr Glu Gln Ile Ser Phe Leu Arg Lys Leu
                165                 170                 175

Tyr His Asn Lys Leu His Val Ser Glu Arg Ser Gln Arg Ile Val Lys
            180                 185                 190

Gln Ala Met Leu Thr Glu Ala Asn Ala Asp Tyr Ile Ile Arg Ala Lys
        195                 200                 205

Thr Gly Tyr Ser Thr Arg Ile Glu Pro Lys Ile Gly Trp Trp Val Gly
    210                 215                 220
```

Trp Val Glu Leu Asp Asp Asn Val Trp Phe Phe Ala Met Asn Met Asp
225 230 235 240

Met Pro Thr Ser Asp Gly Leu Gly Leu Arg Gln Ala Ile Thr Lys Glu
245 250 255

Val Leu Lys Gln Glu Lys Ile Ile Pro
260 265

<210> 15
<211> 263
<212> PRT
<213> Escherichia coli

<220>
<221> PEPTIDE
<222> (1)..(22)
<223> SIGNAL PEPTIDE

<400> 15

Met Arg Val Leu Ala Leu Ser Ala Val Phe Leu Val Ala Ser Ile Ile
1 5 10 15

Gly Met Pro Ala Val Ala Lys Glu Trp Gln Glu Asn Lys Ser Trp Asn
20 25 30

Ala His Phe Thr Glu His Lys Ser Gln Gly Val Val Val Leu Trp Asn
35 40 45

Glu Asn Lys Gln Gln Gly Phe Thr Asn Asn Leu Lys Arg Ala Asn Gln
50 55 60

Ala Phe Leu Pro Ala Ser Thr Phe Lys Ile Pro Asn Ser Leu Ile Ala
65 70 75 80

Leu Asp Leu Gly Val Val Lys Asp Glu His Gln Val Phe Lys Trp Asp
85 90 95

Gly Gln Thr Arg Asp Ile Ala Thr Trp Asn Arg Asp His Asn Leu Ile
100 105 110

Thr Ala Met Lys Tyr Ser Val Val Pro Val Tyr Gln Glu Phe Ala Arg
115 120 125

Gln Ile Gly Glu Ala Arg Met Ser Lys Met Leu His Ala Phe Asp Tyr
130 135 140

Gly Asn Glu Asp Ile Ser Gly Asn Val Asp Ser Phe Trp Leu Asp Gly

145 150 155 160

Gly Ile Arg Ile Ser Ala Thr Glu Gln Ile Ser Phe Leu Arg Lys Leu
165 170 175

Tyr His Asn Lys Leu His Val Ser Glu Arg Ser Gln Arg Ile Val Lys
180 185 190

Gln Ala Met Leu Thr Glu Ala Asn Gly Asp Tyr Ile Ile Arg Ala Lys
195 200 205

Thr Gly Tyr Gly Tyr Asp Thr Lys Ile Gly Trp Trp Val Gly Trp Val
210 215 220

Glu Leu Asp Asp Asn Val Trp Phe Phe Ala Met Asn Met Asp Met Pro
225 230 235 240

Thr Ser Asp Gly Leu Gly Leu Arg Gln Ala Ile Thr Lys Glu Val Leu
245 250 255

Lys Gln Glu Lys Ile Ile Pro
260

<210> 16
<211> 261
<212> PRT
<213> Escherichia coli

<220>
<221> PEPTIDE
<222> (1)..(22)
<223> SIGNAL PEPTIDE

<400> 16

Met Arg Val Leu Ala Leu Ser Ala Val Phe Leu Val Ala Ser Ile Ile
1 5 10 15

Gly Met Pro Ala Val Ala Lys Glu Trp Gln Glu Asn Lys Ser Trp Asn
20 25 30

Ala His Phe Thr Glu His Lys Ser Gln Gly Val Val Val Leu Trp Asn
35 40 45

Glu Asn Lys Gln Gln Gly Phe Thr Asn Asn Leu Lys Arg Ala Asn Gln
50 55 60

Ala Phe Leu Pro Ala Ser Thr Phe Lys Ile Pro Asn Ser Leu Ile Ala
65 70 75 80

Leu Asp Leu Gly Val Val Lys Asp Glu His Gln Val Phe Lys Trp Asp
85 90 95

Gly Gln Thr Arg Asp Ile Ala Thr Trp Asn Arg Asp His Asn Leu Ile
100 105 110

Thr Ala Met Lys Tyr Ser Val Val Pro Val His Gln Glu Phe Ala Arg
115 120 125

Gln Ile Gly Glu Ala Arg Met Ser Lys Met Leu His Ala Phe Asp Tyr
130 135 140

Gly Asn Glu Asp Ile Ser Gly Asn Val Asp Ser Phe Trp Leu Asp Gly
145 150 155 160

Gly Ile Arg Ile Ser Ala Thr Glu Gln Ile Ser Phe Leu Arg Lys Leu
165 170 175

Tyr His Asn Lys Leu His Val Ser Glu Arg Ser Gln Arg Ile Val Lys
180 185 190

Gln Ala Met Leu Thr Glu Ala Asn Gly Asp Tyr Ile Ile Arg Ala Lys
195 200 205

Thr Gly Tyr Asp Thr Lys Ile Gly Trp Trp Val Gly Trp Val Glu Leu
210 215 220

Asp Asp Asn Val Trp Phe Phe Ala Met Asn Met Asp Met Pro Thr Ser
225 230 235 240

Asp Gly Leu Gly Leu Arg Gln Ala Ile Thr Lys Glu Val Leu Lys Gln
245 250 255

Glu Lys Ile Ile Pro
260

<210> 17
<211> 265
<212> PRT
<213> Klebsiella pneumoniae

<220>
<221> PEPTIDE
<222> (1)..(22)
<223> SIGNAL PEPTIDE

<400> 17

Met Arg Val Leu Ala Leu Ser Ala Val Phe Leu Val Ala Ser Ile Ile

```
1               5                   10                  15

Gly Met Pro Ala Val Ala Lys Glu Trp Gln Glu Asn Lys Ser Trp Asn
            20                  25                  30

Ala His Phe Thr Glu His Lys Ser Gln Gly Val Val Val Leu Trp Asn
        35                  40                  45

Glu Asn Lys Gln Gln Gly Phe Thr Asn Asn Leu Lys Arg Ala Asn Gln
    50                  55                  60

Ala Phe Leu Pro Ala Ser Thr Phe Lys Ile Pro Asn Ser Leu Ile Ala
65                  70                  75                  80

Leu Asp Leu Gly Val Val Lys Asp Glu His Gln Val Phe Lys Trp Asp
                85                  90                  95

Gly Gln Thr Arg Asp Ile Ala Ala Trp Asn Arg Asp His Asp Leu Ile
            100                 105                 110

Thr Ala Met Lys Tyr Ser Val Val Pro Val Tyr Gln Glu Phe Ala Arg
        115                 120                 125

Gln Ile Gly Glu Ala Arg Met Ser Lys Met Leu His Ala Phe Asp Tyr
    130                 135                 140

Gly Asn Glu Asp Ile Ser Gly Asn Val Asp Ser Phe Trp Leu Asp Gly
145                 150                 155                 160

Gly Ile Arg Ile Ser Ala Thr Gln Gln Ile Ala Phe Leu Arg Lys Leu
                165                 170                 175

Tyr His Asn Lys Leu His Val Ser Glu Arg Ser Gln Arg Ile Val Lys
            180                 185                 190

Gln Ala Met Leu Thr Glu Ala Asn Gly Asp Tyr Ile Ile Arg Ala Lys
        195                 200                 205

Thr Gly Tyr Ser Thr Gly Ile Glu Pro Lys Ile Gly Trp Trp Val Gly
    210                 215                 220

Trp Val Glu Leu Asp Asp Asn Val Trp Phe Phe Ala Met Asn Met Asp
225                 230                 235                 240

Met Pro Thr Ser Asp Gly Leu Gly Leu Arg Gln Ala Ile Thr Lys Glu
                245                 250                 255
```

Val Leu Lys Gln Glu Lys Ile Ile Pro
260 265

<210> 18
<211> 265
<212> PRT
<213> Enterobacter asburiae

<220>
<221> PEPTIDE
<222> (1)..(22)
<223> SIGNAL PEPTIDE

<400> 18

Met Arg Ala Leu Ala Leu Ser Ala Val Leu Met Val Thr Thr Met Ile
1 5 10 15

Gly Met Pro Ala Val Ala Lys Glu Trp Gln Glu Asn Lys Ser Trp Asn
20 25 30

Ala His Phe Ser Glu His Lys Thr Gln Gly Val Val Val Leu Trp Asn
35 40 45

Glu Asn Thr Gln Gln Gly Phe Thr Asn Asp Leu Lys Arg Ala Asn Gln
50 55 60

Ala Phe Leu Pro Ala Ser Thr Phe Lys Ile Pro Asn Ser Leu Ile Ala
65 70 75 80

Leu Asp Leu Gly Val Val Lys Asp Glu His Gln Val Phe Lys Trp Asp
85 90 95

Gly Gln Thr Arg Asp Ile Ala Ala Trp Asn Arg Asp His Asp Leu Ile
100 105 110

Thr Ala Met Lys Tyr Ser Val Val Pro Val Tyr Gln Glu Phe Ala Arg
115 120 125

Gln Ile Gly Glu Ala Arg Met Ser Lys Met Leu His Ala Phe Asp Tyr
130 135 140

Gly Asn Glu Asp Ile Ser Gly Asn Leu Asp Ser Phe Trp Leu Asp Gly
145 150 155 160

Gly Ile Arg Ile Ser Ala Thr Gln Gln Ile Ala Phe Leu Arg Lys Leu
165 170 175

Tyr His Asn Lys Leu His Val Ser Glu Arg Ser Gln Arg Ile Val Lys
180 185 190

Gln Ala Met Leu Thr Glu Ala Asn Ala Asp Tyr Ile Ile Arg Ala Lys
195 200 205

Thr Gly Tyr Ser Val Arg Ile Glu Pro Lys Ile Gly Trp Trp Val Gly
210 215 220

Trp Ile Glu Leu Asp Asp Asn Val Trp Phe Phe Ala Thr Asn Met Asp
225 230 235 240

Met Pro Thr Ala Glu Gly Leu Gly Leu Arg Gln Thr Ile Thr Lys Ala
245 250 255

Val Leu Lys Gln Glu Lys Ile Ile Pro
260 265

<210> 19
<211> 265
<212> PRT
<213> Klebsiella pneumoniae

<220>
<221> PEPTIDE
<222> (1)..(22)
<223> SIGNAL PEPTIDE

<400> 19

Met Arg Val Leu Ala Leu Ser Thr Val Phe Leu Val Ala Ser Ile Ile
1 5 10 15

Gly Met Pro Ala Val Ala Lys Glu Trp Gln Glu Asn Lys Ser Trp Asn
20 25 30

Ala His Phe Thr Glu His Lys Ser Gln Gly Val Val Val Leu Trp Asn
35 40 45

Glu Asn Lys Gln Gln Gly Phe Thr Asn Asn Leu Lys Arg Ala Asn Gln
50 55 60

Ala Phe Leu Pro Ala Ser Thr Phe Lys Ile Pro Asn Ser Leu Ile Ala
65 70 75 80

Leu Asp Leu Gly Val Val Lys Asp Glu His Gln Val Phe Lys Trp Asp
85 90 95

Gly Gln Thr Arg Asp Ile Ala Thr Trp Asn Arg Asp His Asn Leu Ile
100 105 110

Thr Ala Met Lys Tyr Ser Val Val Pro Val Tyr Gln Glu Phe Ala Arg
115 120 125

Gln Ile Gly Glu Ala Arg Met Ser Lys Met Leu His Ala Phe Asp Tyr
130 135 140

Gly Asn Glu Asp Ile Ser Gly Asn Val Asp Ser Phe Trp Leu Asp Gly
145 150 155 160

Gly Ile Arg Ile Ser Ala Thr Glu Gln Ile Ser Phe Leu Arg Lys Leu
165 170 175

Tyr His Asn Lys Leu His Val Ser Glu Arg Ser Gln Arg Ile Val Lys
180 185 190

Gln Ala Met Leu Thr Glu Ala Asn Gly Asp Tyr Ile Ile Arg Ala Lys
195 200 205

Thr Gly Tyr Ser Thr Arg Ile Glu Pro Lys Ile Gly Trp Trp Val Gly
210 215 220

Trp Val Glu Leu Asp Asp Asn Val Trp Phe Phe Ala Met Asn Met Asp
225 230 235 240

Met Pro Thr Ser Asp Gly Leu Gly Leu Arg Gln Ala Ile Thr Lys Glu
245 250 255

Val Leu Lys Gln Glu Lys Ile Ile Pro
260 265

<210> 20
<211> 265
<212> PRT
<213> Shewanella sp. 10A

<220>
<221> PEPTIDE
<222> (1)..(22)
<223> SIGNAL PEPTIDE

<400> 20

Met Arg Val Leu Ala Leu Ser Ala Val Phe Leu Val Ala Ser Ile Ile
1 5 10 15

Gly Met Pro Ala Val Ala Lys Glu Trp Gln Glu Asn Lys Ser Trp Asn
20 25 30

Ala His Phe Thr Glu His Lys Ser Gln Gly Val Val Val Leu Trp Asn
35 40 45

```
Glu Asn Lys Gln Gln Gly Phe Thr Asn Asn Leu Lys Arg Ala Asn Gln
    50                  55                  60

Ala Phe Leu Pro Ala Ser Thr Phe Lys Ile Pro Asn Ser Leu Ile Ala
65                  70                  75                  80

Leu Asp Leu Gly Val Val Lys Asp Glu His Gln Val Phe Lys Trp Asp
                85                  90                  95

Gly Gln Thr Arg Asp Ile Ala Thr Trp Asn Arg Asp His Asn Leu Ile
            100                 105                 110

Thr Ala Met Lys Tyr Ser Val Val Pro Val Tyr Gln Glu Phe Ala Arg
        115                 120                 125

Gln Ile Gly Glu Ala Arg Met Ser Lys Met Leu His Ala Phe Asp Tyr
    130                 135                 140

Gly Asn Glu Asp Ile Ser Gly Asn Leu Asp Thr Phe Trp Leu Asp Gly
145                 150                 155                 160

Gly Ile Arg Ile Ser Ala Thr Glu Gln Ile Ser Phe Leu Arg Lys Leu
                165                 170                 175

Tyr His Asn Lys Leu His Val Ser Glu Arg Ser Gln Arg Ile Val Lys
            180                 185                 190

Gln Ala Met Leu Thr Glu Ala Asn Gly Asp Tyr Ile Ile Arg Ala Lys
        195                 200                 205

Thr Gly Tyr Ser Thr Arg Ile Glu Pro Lys Ile Gly Trp Trp Val Gly
    210                 215                 220

Trp Val Glu Leu Asp Asp Asn Val Trp Phe Phe Ala Met Asn Met Asp
225                 230                 235                 240

Met Pro Thr Ser Asp Gly Leu Gly Leu Arg Gln Ala Ile Thr Lys Glu
                245                 250                 255

Val Leu Lys Gln Glu Lys Ile Ile Pro
            260                 265
```

<210> 21
<211> 265
<212> PRT
<213> Shewanella sp. W17

```
<220>
<221>  PEPTIDE
<222>  (1)..(22)
<223>  SIGNAL PEPTIDE

<400>  21

Met Arg Val Leu Ala Leu Ser Ala Val Phe Leu Val Ala Ser Ile Ile
1               5                   10                  15

Gly Met Pro Ala Val Ala Lys Glu Trp Gln Glu Asn Lys Ser Trp Asn
            20                  25                  30

Ala His Phe Thr Glu His Lys Ser Gln Gly Val Val Ala Leu Trp Asn
        35                  40                  45

Glu Asn Lys Gln Gln Gly Phe Thr Asn Asn Leu Lys Arg Ala Asn Gln
    50                  55                  60

Ala Phe Leu Pro Ala Ser Thr Phe Lys Ile Pro Asn Ser Leu Ile Ala
65                  70                  75                  80

Leu Asp Leu Gly Val Val Lys Asp Glu His Gln Val Phe Lys Trp Asp
                85                  90                  95

Gly Gln Thr Arg Asp Ile Ala Thr Trp Asn Arg Asp His Asn Leu Ile
            100                 105                 110

Thr Ala Met Lys Tyr Ser Val Val Pro Val Tyr Gln Glu Phe Ala Arg
        115                 120                 125

Gln Ile Gly Glu Ala Arg Met Ser Lys Met Leu His Ala Phe Asp Tyr
    130                 135                 140

Gly Asn Glu Asp Ile Ser Gly Asn Val Asp Ser Phe Trp Leu Asp Gly
145                 150                 155                 160

Gly Ile Arg Ile Ser Ala Thr Glu Gln Ile Ser Phe Leu Arg Lys Leu
                165                 170                 175

Tyr His Asn Lys Leu His Val Ser Glu Arg Ser Gln Arg Ile Val Lys
            180                 185                 190

Gln Ala Met Leu Thr Glu Ala Asn Ser Asp Tyr Ile Ile Arg Ala Lys
        195                 200                 205

Thr Gly Tyr Ser Thr Arg Ile Glu Pro Lys Ile Gly Trp Trp Val Gly
    210                 215                 220
```

Trp Val Glu Leu Asp Asp Asn Val Trp Phe Phe Ala Met Asn Met Asp
225 230 235 240

Met Pro Thr Ser Asp Gly Leu Gly Leu Arg Gln Ala Ile Thr Lys Glu
245 250 255

Val Leu Lys Gln Glu Lys Ile Ile Pro
260 265

<210> 22
<211> 263
<212> PRT
<213> Klebsiella pneumoniae

<220>
<221> PEPTIDE
<222> (1)..(22)
<223> SIGNAL PEPTIDE

<400> 22

Met Arg Val Leu Ala Leu Ser Ala Val Phe Leu Val Ala Ser Ile Ile
1 5 10 15

Gly Met Pro Ala Val Ala Lys Glu Trp Gln Glu Asn Lys Ser Trp Asn
20 25 30

Ala His Phe Thr Glu His Lys Ser Gln Gly Val Val Val Leu Trp Asn
35 40 45

Glu Asn Lys Gln Gln Gly Phe Thr Asn Asn Leu Lys Arg Ala Asn Gln
50 55 60

Ala Phe Leu Pro Ala Ser Thr Phe Lys Ile Pro Asn Ser Leu Ile Ala
65 70 75 80

Leu Asp Leu Gly Val Val Lys Asp Glu His Gln Val Phe Lys Trp Asp
85 90 95

Gly Gln Thr Arg Asp Ile Ala Thr Trp Asn Arg Asp His Asn Leu Ile
100 105 110

Thr Ala Met Lys Tyr Ser Val Val Pro Val Tyr Gln Glu Phe Ala Arg
115 120 125

Gln Ile Gly Glu Ala Arg Met Ser Lys Met Leu His Ala Phe Asp Tyr
130 135 140

Gly Asn Glu Asp Ile Ser Gly Asn Val Asp Ser Phe Trp Leu Asp Gly

145 150 155 160

Gly Ile Arg Ile Ser Ala Thr Glu Gln Ile Ser Phe Leu Arg Lys Leu
165 170 175

Tyr His Asn Lys Leu His Val Ser Glu Arg Ser Gln Arg Ile Val Lys
180 185 190

Gln Ala Met Leu Thr Glu Ala Asn Gly Asp Tyr Ile Ile Arg Ala Lys
195 200 205

Thr Gly Tyr Ser Thr Lys Pro Lys Ile Gly Trp Trp Val Gly Trp Val
210 215 220

Glu Leu Asp Asp Asn Val Trp Phe Phe Ala Met Asn Met Asp Met Pro
225 230 235 240

Thr Ser Asp Gly Leu Gly Leu Arg Gln Ala Ile Thr Lys Glu Val Leu
245 250 255

Lys Gln Glu Lys Ile Ile Pro
260

<210> 23
<211> 265
<212> PRT
<213> Klebsiella pneumoniae

<220>
<221> PEPTIDE
<222> (1)..(22)
<223> SIGNAL PEPTIDE

<400> 23

Met Arg Val Leu Ala Leu Ser Ala Val Phe Leu Val Ala Ser Ile Ile
1 5 10 15

Gly Met Pro Ala Val Ala Lys Glu Trp Gln Glu Asn Lys Ser Trp Asn
20 25 30

Ala His Phe Thr Glu His Lys Ser Gln Gly Val Val Val Leu Trp Asn
35 40 45

Glu Asn Lys Gln Gln Gly Phe Thr Asn Asn Leu Lys Arg Ala Asn Gln
50 55 60

Ala Phe Leu Pro Ala Ser Thr Phe Lys Ile Pro Asn Ser Leu Ile Ala
65 70 75 80

Leu Asp Leu Gly Val Val Lys Asp Glu His Gln Val Phe Lys Trp Asp
85 90 95

Gly Gln Thr Arg Asp Ile Ala Thr Trp Asn Arg Asp His Asn Leu Ile
100 105 110

Thr Ala Met Lys Tyr Ser Val Leu Pro Val Tyr Gln Glu Phe Ala Arg
115 120 125

Gln Ile Gly Glu Ala Arg Met Ser Lys Met Leu His Ala Phe Asp Tyr
130 135 140

Gly Asn Glu Asp Ile Ser Gly Asn Val Asp Ser Phe Trp Leu Asp Gly
145 150 155 160

Gly Ile Arg Ile Ser Ala Thr Glu Gln Ile Ser Phe Leu Arg Lys Leu
165 170 175

Tyr His Asn Lys Leu His Val Ser Glu Arg Ser Gln Arg Ile Val Lys
180 185 190

Gln Ala Met Leu Thr Glu Ala Asn Gly Asp Tyr Ile Ile Arg Ala Lys
195 200 205

Thr Gly Tyr Ser Thr Arg Ile Glu Pro Lys Ile Gly Trp Trp Val Gly
210 215 220

Trp Val Glu Leu Asp Asp Asn Val Trp Phe Phe Ala Met Asn Met Asp
225 230 235 240

Met Pro Thr Ser Asp Gly Leu Gly Leu Arg Gln Ala Ile Thr Lys Glu
245 250 255

Val Leu Lys Gln Glu Lys Ile Ile Pro
260 265

<210> 24
<211> 265
<212> PRT
<213> Shewanella xiamenensis

<220>
<221> PEPTIDE
<222> (1)..(22)
<223> SIGNAL PEPTIDE

<400> 24

Met Arg Val Leu Ala Leu Ser Ala Val Phe Leu Val Ala Ser Ile Ile

```
1               5                   10                  15

Gly Met Pro Ala Val Ala Lys Glu Trp Gln Glu Asn Lys Ser Trp Asn
            20                  25                  30

Ala His Phe Thr Glu His Lys Ser Gln Gly Val Val Val Leu Trp Asn
        35                  40                  45

Glu Asn Lys Gln Gln Gly Phe Thr Asn Asn Leu Lys Arg Ala Asn Gln
    50                  55                  60

Ala Phe Leu Pro Ala Ser Thr Phe Lys Ile Pro Asn Ser Leu Ile Ala
65                  70                  75                  80

Leu Asp Leu Gly Val Val Lys Asp Glu His Gln Val Phe Lys Trp Asp
                85                  90                  95

Gly Gln Thr Arg Asp Ile Ala Thr Trp Asn Arg Asp His Asn Leu Ile
            100                 105                 110

Thr Ala Met Lys Tyr Ser Val Val Pro Val Tyr Gln Glu Phe Ala Arg
        115                 120                 125

Gln Ile Gly Glu Ala Arg Met Ser Lys Met Leu His Ala Phe Asp Tyr
    130                 135                 140

Gly Asn Glu Asp Ile Ser Gly Asn Val Asp Ser Phe Trp Leu Asp Gly
145                 150                 155                 160

Gly Ile Arg Ile Ser Ala Thr Glu Gln Ile Ser Phe Leu Arg Lys Leu
                165                 170                 175

Tyr His Asn Lys Leu His Val Ser Glu Arg Ser Gln Arg Ile Val Lys
            180                 185                 190

Gln Ala Met Leu Thr Glu Ala Asn Ala Asp Tyr Ile Ile Arg Ala Lys
        195                 200                 205

Thr Gly Tyr Ser Gly Arg Ile Glu Pro Lys Phe Gly Trp Trp Val Gly
    210                 215                 220

Trp Val Glu Leu Asp Asp Asn Val Trp Phe Phe Ala Met Asn Met Asp
225                 230                 235                 240

Met Pro Thr Ser Asp Gly Leu Gly Leu Arg Gln Ala Ile Thr Lys Glu
                245                 250                 255
```

Val Leu Lys Gln Glu Lys Ile Ile Pro
260 265

<210> 25
<211> 265
<212> PRT
<213> Shewanella xiamenensis

<220>
<221> PEPTIDE
<222> (1)..(22)
<223> SIGNAL PEPTIDE

<400> 25

Met Arg Val Leu Ala Leu Ser Ala Val Phe Leu Val Ala Ser Ile Ile
1 5 10 15

Gly Met Pro Ala Val Ala Arg Glu Trp Gln Glu Asn Lys Ser Trp Asn
20 25 30

Val His Phe Thr Glu His Lys Ser Gln Gly Val Val Val Leu Trp Asn
35 40 45

Glu Asn Lys Gln Gln Gly Phe Thr Asn Asn Leu Lys Arg Ala Asn Gln
50 55 60

Ala Phe Leu Pro Ala Ser Thr Phe Lys Ile Pro Asn Ser Leu Ile Ala
65 70 75 80

Leu Asp Leu Gly Val Val Lys Asp Glu His Gln Val Phe Lys Trp Asp
85 90 95

Gly Gln Thr Arg Asp Ile Ala Thr Trp Asn Arg Asp His Asn Leu Ile
100 105 110

Thr Ala Met Lys Tyr Ser Val Val Pro Val Tyr Gln Glu Phe Ala Arg
115 120 125

Gln Ile Gly Glu Ala Arg Met Ser Lys Met Leu His Ala Phe Asp Tyr
130 135 140

Gly Asn Glu Asp Ile Ser Gly Asn Val Asp Ser Phe Trp Leu Asp Gly
145 150 155 160

Gly Ile Arg Ile Ser Ala Ile Glu Gln Ile Ser Phe Leu Arg Lys Leu
165 170 175

Tyr His Asn Lys Leu His Val Ser Glu Arg Ser Gln Arg Ile Val Lys
180 185 190

Gln Ala Met Leu Thr Glu Ala Asn Gly Asp Tyr Ile Ile Arg Ala Lys
195 200 205

Thr Gly Tyr Ser Thr Arg Ile Glu Pro Lys Ile Gly Trp Trp Val Gly
210 215 220

Trp Val Glu Leu Asp Asp Asn Val Trp Phe Phe Ala Met Asn Met Asp
225 230 235 240

Met Pro Thr Ser Asp Gly Leu Gly Leu Arg Gln Ala Ile Thr Lys Glu
245 250 255

Val Leu Lys Gln Glu Lys Ile Ile Pro
260 265

<210> 26
<211> 265
<212> PRT
<213> Shewanella xiamenensis

<220>
<221> PEPTIDE
<222> (1)..(22)
<223> SIGNAL PEPTIDE

<400> 26

Met Arg Val Leu Ala Leu Ser Ala Val Phe Leu Val Ala Ser Ile Ile
1 5 10 15

Gly Met Pro Ala Val Ala Lys Glu Trp Gln Glu Asn Lys Ser Trp Asn
20 25 30

Ala His Phe Thr Glu His Lys Ser Gln Gly Val Val Ala Leu Trp Asn
35 40 45

Glu Asn Lys Gln Gln Gly Phe Thr Asn Asn Leu Lys Arg Ala Asn Gln
50 55 60

Ala Phe Leu Pro Ala Ser Thr Phe Lys Ile Pro Asn Ser Leu Ile Ala
65 70 75 80

Leu Asp Leu Gly Val Val Lys Asp Glu His Gln Val Phe Lys Trp Asp
85 90 95

Gly Gln Thr Arg Asp Ile Ala Thr Trp Asn Arg Asp His Asn Leu Ile
100 105 110

Thr Ala Met Lys Tyr Ser Val Val Pro Val Tyr Gln Glu Phe Ala Arg
        115                 120                 125

Gln Ile Gly Glu Ala Arg Met Ser Lys Met Leu His Ala Phe Asp Tyr
    130                 135                 140

Gly Asn Glu Asp Ile Ser Gly Asn Val Asp Ser Phe Trp Leu Asp Gly
145                 150                 155                 160

Gly Ile Arg Ile Ser Ala Ile Glu Gln Ile Ser Phe Leu Arg Lys Leu
                165                 170                 175

Tyr His Asn Lys Leu His Val Ser Glu Arg Ser Gln Arg Ile Val Lys
            180                 185                 190

Gln Ala Met Leu Thr Glu Ala Asn Gly Asp Tyr Ile Ile Arg Ala Lys
        195                 200                 205

Thr Gly Tyr Ser Thr Arg Ile Glu Pro Lys Ile Gly Trp Trp Val Gly
    210                 215                 220

Trp Val Glu Leu Asp Asp Asn Val Trp Phe Phe Ala Met Asn Met Asp
225                 230                 235                 240

Met Pro Thr Ser Asp Gly Leu Gly Leu Arg Gln Ala Ile Thr Lys Glu
                245                 250                 255

Val Leu Lys Gln Glu Lys Ile Ile Pro
            260                 265

<210> 27
<211> 265
<212> PRT
<213> Escherichia coli

<220>
<221> PEPTIDE
<222> (1)..(22)
<223> SIGNAL PEPTIDE

<400> 27

Met Arg Val Leu Ala Leu Ser Ala Val Phe Leu Val Ala Ser Ile Ile
1               5                   10                  15

Gly Met Pro Ala Val Ala Lys Glu Trp Gln Glu Asn Lys Ser Trp Asn
            20                  25                  30

Ala His Phe Thr Glu His Lys Ser Gln Gly Val Val Val Leu Trp Asn
        35                  40                  45

```
Glu Asn Lys Gln Gln Gly Phe Thr Asn Asn Leu Lys Arg Ala Asn Gln
    50                  55                  60

Ala Phe Leu Pro Ala Ser Thr Phe Lys Ile Pro Asn Ser Leu Ile Ala
65                  70                  75                  80

Leu Asp Leu Gly Val Val Lys Asp Glu His Gln Val Phe Lys Trp Asp
                85                  90                  95

Gly Gln Thr Arg Asp Ile Ala Thr Trp Asn Arg Asp His Asn Leu Ile
            100                 105                 110

Thr Ala Met Lys Tyr Ser Val Val Pro Val Tyr Gln Glu Phe Ala Arg
        115                 120                 125

Gln Ile Gly Glu Ala Arg Met Ser Lys Met Leu His Asp Phe Asp Tyr
    130                 135                 140

Gly Asn Glu Asp Ile Ser Gly Asn Val Asp Ser Phe Trp Leu Asp Gly
145                 150                 155                 160

Gly Ile Arg Ile Ser Ala Thr Glu Gln Ile Ser Phe Leu Arg Lys Leu
                165                 170                 175

Tyr His Asn Lys Leu His Val Ser Glu Arg Ser Gln Arg Ile Val Lys
            180                 185                 190

Gln Ala Met Leu Thr Glu Ala Asn Gly Asp Tyr Ile Ile Arg Ala Lys
        195                 200                 205

Thr Gly Tyr Ser Thr Arg Ile Glu Pro Lys Ile Gly Trp Trp Val Gly
    210                 215                 220

Trp Val Glu Leu Asp Asp Asn Val Trp Phe Phe Ala Met Asn Met Asp
225                 230                 235                 240

Met Pro Thr Ser Asp Gly Leu Gly Leu Arg Gln Ala Ile Thr Lys Glu
                245                 250                 255

Val Leu Lys Gln Glu Lys Ile Ile Pro
            260                 265
```

<210> 28
<211> 265
<212> PRT
<213> Shewanella sp. FDAARGOS_354

```
<220>
<221>  PEPTIDE
<222>  (1)..(22)
<223>  SIGNAL PEPTIDE

<400>  28

Met Arg Val Leu Ala Leu Ser Ala Val Phe Leu Val Ala Ser Ile Ile
1               5                   10                  15

Gly Met Pro Ala Val Ala Lys Glu Trp Gln Glu Asn Lys Ser Trp Asn
            20                  25                  30

Ala His Phe Thr Glu His Lys Ser Gln Gly Val Val Ala Leu Trp Asn
        35                  40                  45

Glu Asn Lys Gln Gln Gly Phe Thr Asn Asn Leu Lys Arg Ala Asn Gln
    50                  55                  60

Ala Phe Leu Pro Ala Ser Thr Phe Lys Ile Pro Asn Ser Leu Ile Ala
65                  70                  75                  80

Leu Asp Leu Gly Val Val Lys Asp Glu His Gln Val Phe Lys Trp Asp
                85                  90                  95

Gly Gln Thr Arg Asp Ile Ala Thr Trp Asn Arg Asp His Asn Leu Ile
            100                 105                 110

Thr Ala Met Lys Tyr Ser Val Val Pro Val Tyr Gln Glu Phe Ala Arg
        115                 120                 125

Gln Ile Gly Glu Ala Arg Met Ser Lys Met Leu His Ala Phe Asp Tyr
    130                 135                 140

Gly Asn Glu Asp Ile Ser Gly Asn Val Asp Ser Phe Trp Leu Asp Gly
145                 150                 155                 160

Gly Ile Arg Ile Ser Ala Thr Glu Gln Ile Ser Phe Leu Arg Lys Leu
                165                 170                 175

Tyr His Asn Lys Leu His Val Ser Glu Arg Ser Gln Arg Ile Val Lys
            180                 185                 190

Gln Ala Met Leu Thr Glu Ala Asn Gly Asp Tyr Ile Ile Arg Ala Lys
        195                 200                 205

Thr Gly Tyr Ser Thr Arg Ile Glu Pro Lys Ile Gly Trp Trp Val Gly
    210                 215                 220
```

Trp Val Glu Leu Asp Asp Asn Val Trp Phe Phe Ala Met Asn Met Asp
225 230 235 240

Met Pro Thr Ser Asp Gly Leu Gly Leu Arg Gln Ala Ile Thr Lys Glu
245 250 255

Val Leu Lys Gln Glu Lys Ile Ile Pro
260 265

<210> 29
<211> 243
<212> PRT
<213> Klebsiella pneumoniae

<400> 29

Lys Glu Trp Gln Glu Asn Lys Ser Trp Asn Ala His Phe Thr Glu His
1 5 10 15

Lys Ser Gln Gly Val Val Val Leu Trp Asn Glu Asn Lys Gln Gln Gly
20 25 30

Phe Thr Asn Asn Leu Lys Arg Ala Asn Gln Ala Phe Leu Pro Ala Ser
35 40 45

Thr Phe Lys Ile Pro Asn Ser Leu Ile Ala Leu Asp Leu Gly Val Val
50 55 60

Lys Asp Glu His Gln Val Phe Lys Trp Asp Gly Gln Thr Arg Asp Ile
65 70 75 80

Ala Thr Trp Asn Arg Asp His Asn Leu Ile Thr Ala Met Lys Tyr Ser
85 90 95

Val Val Pro Val Tyr Gln Glu Phe Ala Arg Gln Ile Gly Glu Ala Arg
100 105 110

Met Ser Lys Met Leu His Ala Phe Asp Tyr Gly Asn Glu Asp Ile Ser
115 120 125

Gly Asn Val Asp Ser Phe Trp Leu Asp Gly Gly Ile Arg Ile Ser Ala
130 135 140

Thr Glu Gln Ile Ser Phe Leu Arg Lys Leu Tyr His Asn Lys Leu His
145 150 155 160

Val Ser Glu Arg Ser Gln Arg Ile Val Lys Gln Ala Met Leu Thr Glu
165 170 175

```
Ala Asn Gly Asp Tyr Ile Ile Arg Ala Lys Thr Gly Tyr Ser Thr Arg
            180                 185                 190

Ile Glu Pro Lys Ile Gly Trp Trp Val Gly Trp Val Glu Leu Asp Asp
        195                 200                 205

Asn Val Trp Phe Phe Ala Met Asn Met Asp Met Pro Thr Ser Asp Gly
    210                 215                 220

Leu Gly Leu Arg Gln Ala Ile Thr Lys Glu Val Leu Lys Gln Glu Lys
225                 230                 235                 240

Ile Ile Pro



<210>  30
<211>  243
<212>  PRT
<213>  Shewanella oneidensis

<400>  30

Asn Glu Trp Gln Glu Lys Pro Ser Trp Asn Thr His Phe Ser Glu His
1               5                   10                  15

Lys Ala Gln Gly Val Ile Val Leu Trp Asn Glu Asn Lys Gln Gln Gly
            20                  25                  30

Phe Thr Asn Asn Leu Lys Arg Ala Asn Gln Ala Phe Leu Pro Ala Ser
        35                  40                  45

Thr Phe Lys Ile Pro Asn Ser Leu Ile Ala Leu Asp Leu Gly Val Val
    50                  55                  60

Lys Asp Glu His Gln Val Phe Lys Trp Asp Gly Gln Thr Arg Asp Ile
65                  70                  75                  80

Ala Ala Trp Asn Arg Asp His Asp Leu Ile Thr Ala Met Lys Tyr Ser
                85                  90                  95

Val Val Pro Val Tyr Gln Glu Phe Ala Arg Gln Ile Gly Gln Ala Arg
            100                 105                 110

Met Ser Lys Met Leu His Ala Phe Asp Tyr Gly Asn Glu Asp Ile Ser
        115                 120                 125

Gly Asn Leu Asp Ser Phe Trp Leu Asp Gly Gly Ile Arg Ile Ser Ala
    130                 135                 140
```

Thr Glu Gln Val Ala Phe Leu Arg Lys Leu Tyr His Asn Lys Leu His
145                 150                 155                 160

Val Ser Glu Arg Ser Gln Arg Ile Val Lys Gln Ala Met Leu Thr Glu
                165                 170                 175

Ala Asn Ser Asp Tyr Ile Ile Arg Ala Lys Thr Gly Tyr Ser Thr Arg
            180                 185                 190

Ile Glu Pro Gln Ile Gly Trp Trp Val Gly Trp Val Glu Leu Asp Asp
        195                 200                 205

Asn Val Trp Phe Phe Ala Met Asn Met Asp Met Pro Thr Ala Asp Gly
    210                 215                 220

Leu Gly Leu Arg Gln Ala Ile Thr Lys Glu Val Leu Lys Gln Glu Lys
225                 230                 235                 240

Ile Ile Pro

<210> 31
<211> 243
<212> PRT
<213> Klebsiella pneumoniae

<400> 31

Lys Glu Trp Gln Glu Asn Lys Ser Trp Asn Ala His Phe Thr Glu His
1               5                   10                  15

Lys Ser Gln Gly Val Val Val Leu Trp Asn Glu Asn Lys Gln Gln Gly
            20                  25                  30

Phe Thr Asn Asn Leu Lys Arg Ala Asn Gln Ala Phe Leu Pro Ala Ser
        35                  40                  45

Thr Phe Lys Ile Pro Asn Ser Leu Ile Ala Leu Asp Leu Gly Val Val
    50                  55                  60

Lys Asp Glu His Gln Val Phe Lys Trp Asp Gly Gln Thr Arg Asp Ile
65                  70                  75                  80

Ala Thr Trp Asn Arg Asp His Asn Leu Ile Thr Ala Met Lys Tyr Ser
                85                  90                  95

Val Val Pro Val Tyr Gln Glu Phe Ala Arg Gln Ile Gly Glu Ala Arg
            100                 105                 110

Met Ser Lys Met Leu His Ala Phe Asp Tyr Gly Asn Glu Asp Ile Ser
115 120 125

Gly Asn Val Asp Ser Phe Trp Leu Asp Gly Gly Ile Arg Ile Ser Ala
130 135 140

Thr Glu Gln Ile Ser Phe Leu Arg Lys Leu Tyr His Asn Lys Leu His
145 150 155 160

Val Ser Glu Arg Ser Gln Arg Ile Val Lys Gln Ala Met Leu Thr Glu
165 170 175

Ala Asn Gly Asp Tyr Ile Ile Arg Ala Lys Thr Gly Tyr Ser Ala Arg
180 185 190

Ile Glu Pro Lys Ile Gly Trp Trp Val Gly Trp Val Glu Leu Asp Asp
195 200 205

Asn Val Trp Phe Phe Ala Met Asn Met Asp Met Pro Thr Ser Asp Gly
210 215 220

Leu Gly Leu Arg Gln Ala Ile Thr Lys Glu Val Leu Lys Gln Glu Lys
225 230 235 240

Ile Ile Pro

<210> 32
<211> 239
<212> PRT
<213> Enterobacter cloacae

<400> 32

Lys Glu Trp Gln Glu Asn Lys Ser Trp Asn Ala His Phe Thr Glu His
1 5 10 15

Lys Ser Gln Gly Val Val Val Leu Trp Asn Glu Asn Lys Gln Gln Gly
20 25 30

Phe Thr Asn Asn Leu Lys Arg Ala Asn Gln Ala Phe Leu Pro Ala Ser
35 40 45

Thr Phe Lys Ile Pro Asn Ser Leu Ile Ala Leu Asp Leu Gly Val Val
50 55 60

Lys Asp Glu His Gln Val Phe Lys Trp Asp Gly Gln Thr Arg Asp Ile
65 70 75 80

Ala Thr Trp Asn Arg Asp His Asn Leu Ile Thr Ala Met Lys Tyr Ser
85 90 95

Val Val Pro Val Tyr Gln Glu Phe Ala Arg Gln Ile Gly Glu Ala Arg
100 105 110

Met Ser Lys Met Leu His Ala Phe Asp Tyr Gly Asn Glu Asp Ile Ser
115 120 125

Gly Asn Val Asp Ser Phe Trp Leu Asp Gly Gly Ile Arg Ile Ser Ala
130 135 140

Thr Glu Gln Ile Ser Phe Leu Arg Lys Leu Tyr His Asn Lys Leu His
145 150 155 160

Val Ser Glu Arg Ser Gln Arg Ile Val Lys Gln Ala Met Leu Thr Glu
165 170 175

Ala Asn Gly Asp Tyr Ile Ile Arg Ala Lys Thr Gly Tyr Asp Thr Lys
180 185 190

Ile Gly Trp Trp Val Gly Trp Val Glu Leu Asp Asp Asn Val Trp Phe
195 200 205

Phe Ala Met Asn Met Asp Met Pro Thr Ser Asp Gly Leu Gly Leu Arg
210 215 220

Gln Ala Ile Thr Lys Glu Val Leu Lys Gln Glu Lys Ile Ile Pro
225 230 235

<210> 33
<211> 243
<212> PRT
<213> Klebsiella pneumoniae

<400> 33

Lys Glu Trp Gln Glu Asn Lys Ser Trp Asn Ala His Phe Thr Glu His
1 5 10 15

Lys Ser Gln Gly Val Val Val Leu Trp Asn Glu Asn Lys Gln Gln Gly
20 25 30

Phe Thr Asn Asn Leu Lys Arg Ala Asn Gln Ala Phe Leu Pro Ala Ser
35 40 45

Thr Phe Lys Ile Pro Asn Ser Leu Ile Ala Leu Asp Leu Gly Val Val
50 55 60

Lys Asp Glu His Gln Val Phe Lys Trp Asp Gly Gln Thr Arg Asp Ile
65 70 75 80

Ala Ala Trp Asn Arg Asp His Asp Leu Ile Thr Ala Met Lys Tyr Ser
85 90 95

Val Val Pro Val Tyr Gln Glu Phe Ala Arg Gln Ile Gly Glu Ala Arg
100 105 110

Met Ser Lys Met Leu His Ala Phe Asp Tyr Gly Asn Glu Asp Ile Ser
115 120 125

Gly Asn Val Asp Ser Phe Trp Leu Asp Gly Gly Ile Arg Ile Ser Ala
130 135 140

Thr Gln Gln Ile Ala Phe Leu Arg Lys Leu Tyr His Asn Lys Leu His
145 150 155 160

Val Ser Glu Arg Ser Gln Arg Ile Val Lys Gln Ala Met Leu Thr Glu
165 170 175

Ala Asn Gly Asp Tyr Ile Ile Arg Ala Lys Thr Gly Tyr Ser Thr Arg
180 185 190

Ile Glu Pro Lys Ile Gly Trp Trp Val Gly Trp Val Glu Leu Asp Asp
195 200 205

Asn Val Trp Phe Phe Ala Met Asn Met Asp Met Pro Thr Ser Asp Gly
210 215 220

Leu Gly Leu Arg Gln Ala Ile Thr Lys Glu Val Leu Lys Gln Glu Lys
225 230 235 240

Ile Ile Pro

<210> 34
<211> 243
<212> PRT
<213> Shewanella xiamenensis

<400> 34

Lys Glu Trp Gln Glu Asn Lys Ser Trp Asn Ala His Phe Thr Glu Tyr
1 5 10 15

Lys Ser Gln Gly Val Val Ala Leu Trp Asn Glu Asn Lys Gln Gln Gly
20 25 30

```
Phe Thr Asn Asn Leu Lys Arg Ala Asn Gln Ala Phe Leu Pro Ala Ser
        35                  40                  45

Thr Phe Lys Ile Pro Asn Ser Leu Ile Ala Leu Asp Leu Gly Val Val
    50                  55                  60

Lys Asp Glu His Gln Val Phe Lys Trp Asp Gly Gln Thr Arg Asp Ile
65                  70                  75                  80

Ala Thr Trp Asn Arg Asp His Asn Leu Ile Thr Ala Met Lys Tyr Ser
                85                  90                  95

Val Val Pro Val Tyr Gln Glu Phe Ala Arg Gln Ile Gly Glu Ala Arg
            100                 105                 110

Met Ser Lys Met Leu His Ala Phe Asp Tyr Gly Asn Glu Asp Ile Ser
        115                 120                 125

Gly Asn Val Gly Ser Phe Trp Leu Asp Gly Gly Ile Arg Ile Ser Ala
    130                 135                 140

Thr Glu Gln Ile Ser Phe Leu Arg Lys Leu Tyr His Asn Lys Leu His
145                 150                 155                 160

Val Ser Glu Arg Ser Gln Arg Ile Val Lys Gln Ala Met Leu Thr Glu
                165                 170                 175

Ala Asn Gly Asp Tyr Ile Ile Arg Ala Lys Thr Gly Tyr Ser Thr Arg
            180                 185                 190

Ile Glu Pro Lys Ile Gly Trp Trp Val Gly Trp Val Glu Leu Asp Asp
        195                 200                 205

Asn Val Trp Phe Phe Ala Met Asn Met Asp Met Pro Thr Ser Asp Gly
    210                 215                 220

Leu Gly Leu Arg Gln Ala Ile Thr Lys Glu Val Leu Lys Gln Glu Lys
225                 230                 235                 240

Ile Ile Pro
```

<210> 35
<211> 243
<212> PRT
<213> Klebsiella pneumoniae

<400> 35

```
Lys Glu Trp Gln Glu Asn Lys Ser Trp Asn Ala His Phe Thr Glu His
1               5                   10                  15

Lys Ser Gln Gly Val Val Val Leu Trp Asn Glu Asn Lys Gln Gln Gly
            20                  25                  30

Phe Thr Asn Asn Leu Lys Arg Ala Asn Gln Ala Phe Leu Pro Ala Ser
        35                  40                  45

Thr Phe Lys Ile Pro Asn Ser Leu Ile Ala Leu Asp Leu Gly Val Val
    50                  55                  60

Lys Asp Glu His Gln Val Phe Lys Trp Asp Gly His Arg Arg Asp Ile
65                  70                  75                  80

Ala Thr Trp Asn Arg Asp His Asn Leu Ile Thr Ala Met Lys Tyr Ser
                85                  90                  95

Val Val Pro Val Tyr Gln Glu Phe Ala Arg Gln Ile Gly Glu Ala Arg
            100                 105                 110

Met Ser Lys Met Leu His Ala Phe Asp Tyr Gly Asn Glu Asp Ile Ser
        115                 120                 125

Gly Asn Val Asp Ser Phe Trp Leu Asp Gly Gly Ile Arg Ile Ser Ala
    130                 135                 140

Thr Glu Gln Ile Ser Phe Leu Arg Lys Leu Tyr His Asn Lys Leu His
145                 150                 155                 160

Val Ser Glu Arg Ser Gln Arg Ile Val Lys Gln Ala Met Leu Thr Glu
                165                 170                 175

Ala Asn Gly Asp Tyr Ile Ile Arg Ala Lys Thr Gly Tyr Ser Thr Arg
            180                 185                 190

Ile Glu Pro Lys Ile Gly Trp Trp Val Gly Trp Val Glu Leu Asp Asp
        195                 200                 205

Asn Val Trp Phe Phe Ala Met Asn Met Asp Met Pro Thr Ser Asp Gly
    210                 215                 220

Leu Gly Leu Arg Gln Ala Ile Thr Lys Glu Val Leu Lys Gln Glu Lys
225                 230                 235                 240

Ile Ile Pro
```

<210> 36
<211> 243
<212> PRT
<213> Klebsiella pneumoniae

<400> 36

Lys Glu Trp Gln Glu Asn Lys Ser Trp Asn Ala His Phe Thr Glu His
1 5 10 15

Lys Ser Gln Gly Val Val Val Leu Trp Asn Glu Asn Lys Gln Gln Gly
20 25 30

Phe Thr Asn Asn Leu Lys Arg Ala Asn Gln Ala Phe Leu Pro Ala Ser
35 40 45

Thr Phe Lys Ile Pro Asn Ser Leu Ile Ala Leu Asp Leu Gly Val Val
50 55 60

Lys Asp Glu His Gln Val Phe Lys Trp Asp Gly Gln Thr Arg Asp Ile
65 70 75 80

Ala Thr Trp Asn Arg Asp His Asn Leu Ile Thr Ala Met Lys Tyr Ser
85 90 95

Val Val Pro Val Tyr Gln Glu Phe Ala Arg Gln Ile Gly Glu Ala Arg
100 105 110

Met Ser Lys Met Leu His Ala Phe Asp Tyr Gly Asn Glu Asp Ile Ser
115 120 125

Gly Asn Val Asp Ser Phe Trp Leu Asp Gly Gly Ile Arg Ile Ser Ala
130 135 140

Thr Glu Gln Ile Ser Phe Leu Arg Lys Leu Tyr His Asn Lys Leu His
145 150 155 160

Val Ser Glu Arg Ser Gln Arg Ile Val Lys Gln Ala Met Leu Thr Glu
165 170 175

Ala Asn Gly Asp Tyr Ile Ile Arg Ala Lys Thr Gly Tyr Ser Thr Gly
180 185 190

Ile Glu Pro Lys Ile Gly Trp Trp Val Gly Trp Val Glu Leu Asp Asp
195 200 205

Asn Val Trp Phe Phe Ala Met Asn Met Asp Met Pro Thr Ser Asp Gly
210 215 220

Leu Gly Leu Arg Gln Ala Ile Thr Lys Glu Val Leu Lys Gln Glu Lys
225 230 235 240

Ile Ile Pro

<210> 37
<211> 243
<212> PRT
<213> Klebsiella pneumoniae

<400> 37

Lys Glu Trp Gln Glu Asn Lys Ser Trp Asn Ala His Phe Thr Glu His
1 5 10 15

Lys Ser Gln Gly Val Val Val Leu Trp Asn Glu Asn Lys Gln Gln Gly
20 25 30

Phe Thr Asn Asn Leu Lys Arg Ala Asn Gln Ala Phe Leu Pro Ala Ser
35 40 45

Thr Phe Lys Ile Pro Asn Ser Leu Ile Ala Leu Asp Leu Gly Val Val
50 55 60

Lys Asp Glu His Gln Val Phe Lys Trp Asp Gly Gln Thr Arg Asp Ile
65 70 75 80

Ala Thr Trp Asn Arg Asp His Asn Leu Ile Thr Ala Met Lys Tyr Ser
85 90 95

Val Val Pro Val Tyr Gln Tyr Phe Ala Arg Gln Ile Gly Glu Ala Arg
100 105 110

Met Ser Lys Met Leu His Ala Phe Asp Tyr Gly Asn Glu Asp Ile Ser
115 120 125

Gly Asn Val Asp Ser Phe Trp Leu Asp Gly Gly Ile Arg Ile Ser Ala
130 135 140

Thr Glu Gln Ile Ser Phe Leu Arg Lys Leu Tyr His Asn Lys Leu His
145 150 155 160

Val Ser Glu Arg Ser Gln Arg Ile Val Lys Gln Ala Met Leu Thr Glu
165 170 175

Ala Asn Gly Asp Tyr Ile Ile Arg Ala Lys Thr Gly Tyr Ser Thr Arg
180 185 190

Ile Glu Pro Lys Ile Gly Trp Trp Val Gly Trp Val Glu Leu Asp Asp
195 200 205

Asn Val Trp Phe Phe Ala Met Asn Met Asp Met Pro Thr Ser Asp Gly
210 215 220

Leu Gly Leu Arg Gln Ala Ile Thr Lys Glu Val Leu Lys Gln Glu Lys
225 230 235 240

Ile Ile Pro

<210> 38
<211> 239
<212> PRT
<213> Klebsiella pneumoniae

<400> 38

Lys Glu Trp Gln Glu Asn Lys Ser Trp Asn Ala His Phe Thr Glu His
1 5 10 15

Lys Ser Gln Gly Val Val Val Leu Trp Asn Glu Asn Lys Gln Gln Gly
20 25 30

Phe Thr Asn Asn Leu Lys Arg Ala Asn Gln Ala Phe Leu Pro Ala Ser
35 40 45

Thr Phe Lys Ile Pro Asn Ser Leu Ile Ala Leu Asp Leu Gly Val Val
50 55 60

Lys Asp Glu His Gln Val Phe Lys Trp Asp Gly Gln Thr Arg Asp Ile
65 70 75 80

Ala Thr Trp Asn Arg Asp His Asn Leu Ile Thr Ala Met Lys Tyr Ser
85 90 95

Val Val Pro Val Tyr Gln Glu Phe Ala Arg Gln Ile Gly Glu Ala Arg
100 105 110

Met Ser Lys Met Leu His Ala Phe Asp Tyr Gly Asn Glu Asp Ile Ser
115 120 125

Gly Asn Val Asp Ser Phe Trp Leu Asp Gly Gly Ile Arg Ile Ser Ala
130 135 140

Thr Glu Gln Ile Ser Phe Leu Arg Lys Leu Tyr His Asn Lys Leu His
145 150 155 160

```
Val Ser Glu Arg Ser Gln Arg Ile Val Lys Gln Ala Met Leu Thr Glu
                165                 170                 175

Ala Asn Gly Asp Tyr Ile Ile Arg Ala Lys Thr Gly Ser Asn Thr Lys
            180                 185                 190

Ile Gly Trp Trp Val Gly Trp Val Glu Leu Asp Asp Asn Val Trp Phe
        195                 200                 205

Phe Ala Met Asn Met Asp Met Pro Thr Ser Asp Gly Leu Gly Leu Arg
    210                 215                 220

Gln Ala Ile Thr Lys Glu Val Leu Lys Gln Glu Lys Ile Ile Pro
225                 230                 235


<210>  39
<211>  243
<212>  PRT
<213>  Escherichia coli

<400>  39

Lys Glu Trp Gln Glu Asn Lys Ser Trp Asn Ala His Phe Thr Glu His
1               5                   10                  15

Lys Ser Gln Gly Val Val Val Leu Trp Asn Glu Asn Lys Gln Gln Gly
            20                  25                  30

Phe Thr Asn Asn Leu Lys Arg Ala Asn Gln Ala Phe Leu Pro Ala Ser
        35                  40                  45

Thr Phe Lys Ile Pro Asn Ser Leu Ile Ala Leu Asp Leu Gly Val Val
    50                  55                  60

Lys Asp Glu His Gln Val Phe Lys Trp Asp Gly Gln Thr Arg Asp Ile
65                  70                  75                  80

Ala Ala Trp Asn Arg Asp His Asp Leu Ile Thr Ala Met Lys Tyr Ser
                85                  90                  95

Val Val Pro Val Tyr Gln Glu Phe Ala Arg Gln Ile Gly Glu Ala Arg
            100                 105                 110

Met Ser Lys Met Leu His Ala Phe Asp Tyr Gly Asn Glu Asp Ile Ser
        115                 120                 125

Gly Asn Val Asp Ser Phe Trp Leu Asp Gly Gly Ile Arg Ile Ser Ala
    130                 135                 140
```

Thr Gln Gln Ile Ala Phe Leu Arg Lys Leu Tyr His Asn Lys Leu His
145 150 155 160

Val Ser Glu Arg Ser Gln Arg Ile Val Lys Gln Ala Met Leu Thr Glu
165 170 175

Ala Asn Gly Asp Tyr Ile Ile Arg Ala Lys Thr Gly Tyr Ser Thr Ser
180 185 190

Ile Glu Pro Lys Ile Gly Trp Trp Val Gly Trp Val Glu Leu Asp Asp
195 200 205

Asn Val Trp Phe Phe Ala Met Asn Met Asp Met Pro Thr Ser Asp Gly
210 215 220

Leu Gly Leu Arg Gln Ala Ile Thr Lys Glu Val Leu Lys Gln Glu Lys
225 230 235 240

Ile Ile Pro

<210> 40
<211> 243
<212> PRT
<213> Enterobacter sp. 87F-2

<400> 40

Lys Glu Trp Gln Glu Asn Lys Ser Trp Asn Ala His Phe Thr Glu His
1 5 10 15

Lys Ser Gln Gly Val Val Val Leu Trp Asn Glu Asn Lys Gln Gln Gly
20 25 30

Phe Thr Asn Asn Leu Lys Arg Ala Asn Gln Ala Phe Leu Pro Ala Ser
35 40 45

Thr Phe Lys Ile Pro Asn Ser Leu Ile Ala Leu Asp Leu Gly Val Val
50 55 60

Lys Asp Glu His Gln Val Phe Lys Trp Asp Gly Gln Thr Arg Asp Ile
65 70 75 80

Ala Thr Trp Asn Arg Asp His Asn Leu Ile Thr Ala Met Lys Tyr Ser
85 90 95

Val Val Pro Val Tyr Gln Glu Phe Ala Arg Gln Ile Gly Glu Ala Arg
100 105 110

Met Ser Lys Met Leu His Ala Phe Asp Tyr Gly Asn Glu Asp Ile Ser
        115                 120                 125

Gly Asn Val Asp Ser Phe Trp Leu Asp Gly Gly Ile Arg Ile Ser Ala
    130                 135                 140

Thr Glu Gln Ile Ser Phe Leu Arg Lys Leu Tyr His Asn Lys Leu His
145                 150                 155                 160

Val Ser Glu Arg Ser Gln Arg Ile Val Lys Gln Ala Met Leu Thr Glu
                165                 170                 175

Ala Asn Gly Asp Tyr Ile Ile Arg Ala Lys Thr Gly Tyr Glu Thr Arg
            180                 185                 190

Ile Glu Pro Lys Ile Gly Trp Trp Val Gly Trp Val Glu Leu Asp Asp
        195                 200                 205

Asn Val Trp Phe Phe Ala Met Asn Met Asp Met Pro Thr Ser Asp Gly
    210                 215                 220

Leu Gly Leu Arg Gln Ala Ile Thr Lys Glu Val Leu Lys Gln Glu Lys
225                 230                 235                 240

Ile Ile Pro

<210> 41
<211> 239
<212> PRT
<213> Serratia marcescens

<400> 41

Lys Glu Trp Gln Glu Asn Lys Ser Trp Asn Ala His Phe Thr Glu His
1               5                   10                  15

Lys Ser Gln Gly Val Val Val Leu Trp Asn Glu Asn Lys Gln Gln Gly
            20                  25                  30

Phe Thr Asn Asn Leu Lys Arg Ala Asn Gln Ala Phe Leu Pro Ala Ser
        35                  40                  45

Thr Phe Lys Ile Pro Asn Ser Leu Ile Ala Leu Asp Leu Gly Val Val
    50                  55                  60

Lys Asp Glu His Gln Val Phe Lys Trp Asp Gly Gln Thr Arg Asp Ile
65                  70                  75                  80

Ala Thr Trp Asn Arg Asp His Asn Leu Ile Thr Ala Met Lys Tyr Ser
85 90 95

Val Val Pro Val Tyr Gln Glu Phe Ala Arg Gln Ile Gly Glu Ala Arg
100 105 110

Met Ser Lys Met Leu His Ala Phe Asp Tyr Gly Asn Glu Asp Ile Ser
115 120 125

Gly Asn Val Asp Ser Phe Trp Leu Asp Gly Gly Ile Arg Ile Ser Ala
130 135 140

Thr Glu Gln Ile Ser Phe Leu Arg Lys Leu Tyr His Asn Lys Leu His
145 150 155 160

Val Ser Glu Arg Ser Gln Arg Ile Val Lys Gln Ala Met Leu Thr Glu
165 170 175

Ala Asn Gly Asp Tyr Ile Ile Arg Ala Lys Thr Gly Tyr Ser Pro Lys
180 185 190

Ile Gly Trp Trp Val Gly Trp Val Glu Leu Asp Asp Asn Val Trp Phe
195 200 205

Phe Ala Met Asn Met Asp Met Pro Thr Ser Asp Gly Leu Gly Leu Arg
210 215 220

Gln Ala Ile Thr Lys Glu Val Leu Lys Gln Glu Lys Ile Ile Pro
225 230 235

<210> 42
<211> 243
<212> PRT
<213> Shewanella xiamenensis

<400> 42

Lys Glu Trp Gln Glu Asn Lys Ser Trp Asn Ala His Phe Thr Glu His
1 5 10 15

Lys Ser Gln Gly Val Val Val Leu Trp Asn Glu Asn Lys Gln Gln Gly
20 25 30

Phe Thr Asn Asn Leu Lys Arg Ala Asn Gln Ala Phe Leu Pro Ala Ser
35 40 45

Thr Phe Lys Ile Pro Asn Ser Leu Ile Ala Leu Asp Leu Gly Val Val
50 55 60

Lys Asp Glu His Gln Val Phe Lys Trp Asp Gly Gln Thr Arg Asp Ile
65 70 75 80

Ala Thr Trp Asn Arg Asp His Asn Leu Ile Thr Ala Met Lys Tyr Ser
85 90 95

Val Val Pro Val Tyr Gln Glu Phe Ala Arg Gln Ile Gly Glu Ala Arg
100 105 110

Met Ser Lys Met Leu His Ala Phe Asp Tyr Gly Asn Glu Asp Ile Ser
115 120 125

Gly Asn Leu Asp Thr Phe Trp Leu Asp Gly Gly Ile Arg Ile Ser Ala
130 135 140

Thr Glu Gln Ile Ser Phe Leu Arg Lys Leu Tyr His Asn Lys Leu His
145 150 155 160

Val Ser Glu Arg Ser Gln Arg Ile Val Lys Gln Ala Met Leu Thr Glu
165 170 175

Ala Asn Ala Asp Tyr Ile Ile Arg Ala Lys Thr Gly Tyr Ser Thr Arg
180 185 190

Ile Glu Pro Lys Ile Gly Trp Trp Val Gly Trp Val Glu Leu Asp Asp
195 200 205

Asn Val Trp Phe Phe Ala Met Asn Met Asp Met Pro Thr Ser Asp Gly
210 215 220

Leu Gly Leu Arg Gln Ala Ile Thr Lys Glu Val Leu Lys Gln Glu Lys
225 230 235 240

Ile Ile Pro

<210> 43
<211> 241
<212> PRT
<213> Escherichia coli

<400> 43

Lys Glu Trp Gln Glu Asn Lys Ser Trp Asn Ala His Phe Thr Glu His
1 5 10 15

Lys Ser Gln Gly Val Val Val Leu Trp Asn Glu Asn Lys Gln Gln Gly
20 25 30

Phe Thr Asn Asn Leu Lys Arg Ala Asn Gln Ala Phe Leu Pro Ala Ser
35 40 45

Thr Phe Lys Ile Pro Asn Ser Leu Ile Ala Leu Asp Leu Gly Val Val
50 55 60

Lys Asp Glu His Gln Val Phe Lys Trp Asp Gly Gln Thr Arg Asp Ile
65 70 75 80

Ala Thr Trp Asn Arg Asp His Asn Leu Ile Thr Ala Met Lys Tyr Ser
85 90 95

Val Val Pro Val Tyr Gln Glu Phe Ala Arg Gln Ile Gly Glu Ala Arg
100 105 110

Met Ser Lys Met Leu His Ala Phe Asp Tyr Gly Asn Glu Asp Ile Ser
115 120 125

Gly Asn Val Asp Ser Phe Trp Leu Asp Gly Gly Ile Arg Ile Ser Ala
130 135 140

Thr Glu Gln Ile Ser Phe Leu Arg Lys Leu Tyr His Asn Lys Leu His
145 150 155 160

Val Ser Glu Arg Ser Gln Arg Ile Val Lys Gln Ala Met Leu Thr Glu
165 170 175

Ala Asn Gly Asp Tyr Ile Ile Arg Ala Lys Thr Gly Tyr Gly Tyr Asp
180 185 190

Thr Lys Ile Gly Trp Trp Val Gly Trp Val Glu Leu Asp Asp Asn Val
195 200 205

Trp Phe Phe Ala Met Asn Met Asp Met Pro Thr Ser Asp Gly Leu Gly
210 215 220

Leu Arg Gln Ala Ile Thr Lys Glu Val Leu Lys Gln Glu Lys Ile Ile
225 230 235 240

Pro

<210> 44
<211> 239
<212> PRT
<213> Escherichia coli

<400> 44

```
Lys Glu Trp Gln Glu Asn Lys Ser Trp Asn Ala His Phe Thr Glu His
1               5                   10                  15

Lys Ser Gln Gly Val Val Val Leu Trp Asn Glu Asn Lys Gln Gln Gly
            20                  25                  30

Phe Thr Asn Asn Leu Lys Arg Ala Asn Gln Ala Phe Leu Pro Ala Ser
        35                  40                  45

Thr Phe Lys Ile Pro Asn Ser Leu Ile Ala Leu Asp Leu Gly Val Val
    50                  55                  60

Lys Asp Glu His Gln Val Phe Lys Trp Asp Gly Gln Thr Arg Asp Ile
65                  70                  75                  80

Ala Thr Trp Asn Arg Asp His Asn Leu Ile Thr Ala Met Lys Tyr Ser
                85                  90                  95

Val Val Pro Val His Gln Glu Phe Ala Arg Gln Ile Gly Glu Ala Arg
            100                 105                 110

Met Ser Lys Met Leu His Ala Phe Asp Tyr Gly Asn Glu Asp Ile Ser
        115                 120                 125

Gly Asn Val Asp Ser Phe Trp Leu Asp Gly Gly Ile Arg Ile Ser Ala
    130                 135                 140

Thr Glu Gln Ile Ser Phe Leu Arg Lys Leu Tyr His Asn Lys Leu His
145                 150                 155                 160

Val Ser Glu Arg Ser Gln Arg Ile Val Lys Gln Ala Met Leu Thr Glu
                165                 170                 175

Ala Asn Gly Asp Tyr Ile Ile Arg Ala Lys Thr Gly Tyr Asp Thr Lys
            180                 185                 190

Ile Gly Trp Trp Val Gly Trp Val Glu Leu Asp Asp Asn Val Trp Phe
        195                 200                 205

Phe Ala Met Asn Met Asp Met Pro Thr Ser Asp Gly Leu Gly Leu Arg
    210                 215                 220

Gln Ala Ile Thr Lys Glu Val Leu Lys Gln Glu Lys Ile Ile Pro
225                 230                 235
```

<210> 45

<211> 243
<212> PRT
<213> Klebsiella pneumoniae

<400> 45

```
Lys Glu Trp Gln Glu Asn Lys Ser Trp Asn Ala His Phe Thr Glu His
1               5                   10                  15

Lys Ser Gln Gly Val Val Val Leu Trp Asn Glu Asn Lys Gln Gln Gly
            20                  25                  30

Phe Thr Asn Asn Leu Lys Arg Ala Asn Gln Ala Phe Leu Pro Ala Ser
        35                  40                  45

Thr Phe Lys Ile Pro Asn Ser Leu Ile Ala Leu Asp Leu Gly Val Val
    50                  55                  60

Lys Asp Glu His Gln Val Phe Lys Trp Asp Gly Gln Thr Arg Asp Ile
65                  70                  75                  80

Ala Ala Trp Asn Arg Asp His Asp Leu Ile Thr Ala Met Lys Tyr Ser
                85                  90                  95

Val Val Pro Val Tyr Gln Glu Phe Ala Arg Gln Ile Gly Glu Ala Arg
            100                 105                 110

Met Ser Lys Met Leu His Ala Phe Asp Tyr Gly Asn Glu Asp Ile Ser
        115                 120                 125

Gly Asn Val Asp Ser Phe Trp Leu Asp Gly Gly Ile Arg Ile Ser Ala
    130                 135                 140

Thr Gln Gln Ile Ala Phe Leu Arg Lys Leu Tyr His Asn Lys Leu His
145                 150                 155                 160

Val Ser Glu Arg Ser Gln Arg Ile Val Lys Gln Ala Met Leu Thr Glu
                165                 170                 175

Ala Asn Gly Asp Tyr Ile Ile Arg Ala Lys Thr Gly Tyr Ser Thr Gly
            180                 185                 190

Ile Glu Pro Lys Ile Gly Trp Trp Val Gly Trp Val Glu Leu Asp Asp
        195                 200                 205

Asn Val Trp Phe Phe Ala Met Asn Met Asp Met Pro Thr Ser Asp Gly
    210                 215                 220

Leu Gly Leu Arg Gln Ala Ile Thr Lys Glu Val Leu Lys Gln Glu Lys
```

225 230 235 240

Ile Ile Pro

<210> 46
<211> 243
<212> PRT
<213> Enterobacter asburiae

<400> 46

Lys Glu Trp Gln Glu Asn Lys Ser Trp Asn Ala His Phe Ser Glu His
1 5 10 15

Lys Thr Gln Gly Val Val Val Leu Trp Asn Glu Asn Thr Gln Gln Gly
20 25 30

Phe Thr Asn Asp Leu Lys Arg Ala Asn Gln Ala Phe Leu Pro Ala Ser
35 40 45

Thr Phe Lys Ile Pro Asn Ser Leu Ile Ala Leu Asp Leu Gly Val Val
50 55 60

Lys Asp Glu His Gln Val Phe Lys Trp Asp Gly Gln Thr Arg Asp Ile
65 70 75 80

Ala Ala Trp Asn Arg Asp His Asp Leu Ile Thr Ala Met Lys Tyr Ser
85 90 95

Val Val Pro Val Tyr Gln Glu Phe Ala Arg Gln Ile Gly Glu Ala Arg
100 105 110

Met Ser Lys Met Leu His Ala Phe Asp Tyr Gly Asn Glu Asp Ile Ser
115 120 125

Gly Asn Leu Asp Ser Phe Trp Leu Asp Gly Gly Ile Arg Ile Ser Ala
130 135 140

Thr Gln Gln Ile Ala Phe Leu Arg Lys Leu Tyr His Asn Lys Leu His
145 150 155 160

Val Ser Glu Arg Ser Gln Arg Ile Val Lys Gln Ala Met Leu Thr Glu
165 170 175

Ala Asn Ala Asp Tyr Ile Ile Arg Ala Lys Thr Gly Tyr Ser Val Arg
180 185 190

Ile Glu Pro Lys Ile Gly Trp Trp Val Gly Trp Ile Glu Leu Asp Asp

195 200 205

Asn Val Trp Phe Phe Ala Thr Asn Met Asp Met Pro Thr Ala Glu Gly
210 215 220

Leu Gly Leu Arg Gln Thr Ile Thr Lys Ala Val Leu Lys Gln Glu Lys
225 230 235 240

Ile Ile Pro

<210> 47
<211> 243
<212> PRT
<213> Klebsiella pneumoniae

<400> 47

Lys Glu Trp Gln Glu Asn Lys Ser Trp Asn Ala His Phe Thr Glu His
1 5 10 15

Lys Ser Gln Gly Val Val Val Leu Trp Asn Glu Asn Lys Gln Gln Gly
20 25 30

Phe Thr Asn Asn Leu Lys Arg Ala Asn Gln Ala Phe Leu Pro Ala Ser
35 40 45

Thr Phe Lys Ile Pro Asn Ser Leu Ile Ala Leu Asp Leu Gly Val Val
50 55 60

Lys Asp Glu His Gln Val Phe Lys Trp Asp Gly Gln Thr Arg Asp Ile
65 70 75 80

Ala Thr Trp Asn Arg Asp His Asn Leu Ile Thr Ala Met Lys Tyr Ser
85 90 95

Val Val Pro Val Tyr Gln Glu Phe Ala Arg Gln Ile Gly Glu Ala Arg
100 105 110

Met Ser Lys Met Leu His Ala Phe Asp Tyr Gly Asn Glu Asp Ile Ser
115 120 125

Gly Asn Val Asp Ser Phe Trp Leu Asp Gly Gly Ile Arg Ile Ser Ala
130 135 140

Thr Glu Gln Ile Ser Phe Leu Arg Lys Leu Tyr His Asn Lys Leu His
145 150 155 160

Val Ser Glu Arg Ser Gln Arg Ile Val Lys Gln Ala Met Leu Thr Glu

165 170 175

Ala Asn Gly Asp Tyr Ile Ile Arg Ala Lys Thr Gly Tyr Ser Thr Arg
180 185 190

Ile Glu Pro Lys Ile Gly Trp Trp Val Gly Trp Val Glu Leu Asp Asp
195 200 205

Asn Val Trp Phe Phe Ala Met Asn Met Asp Met Pro Thr Ser Asp Gly
210 215 220

Leu Gly Leu Arg Gln Ala Ile Thr Lys Glu Val Leu Lys Gln Glu Lys
225 230 235 240

Ile Ile Pro

<210> 48
<211> 243
<212> PRT
<213> Shewanella sp. 10A

<400> 48

Lys Glu Trp Gln Glu Asn Lys Ser Trp Asn Ala His Phe Thr Glu His
1 5 10 15

Lys Ser Gln Gly Val Val Val Leu Trp Asn Glu Asn Lys Gln Gln Gly
20 25 30

Phe Thr Asn Asn Leu Lys Arg Ala Asn Gln Ala Phe Leu Pro Ala Ser
35 40 45

Thr Phe Lys Ile Pro Asn Ser Leu Ile Ala Leu Asp Leu Gly Val Val
50 55 60

Lys Asp Glu His Gln Val Phe Lys Trp Asp Gly Gln Thr Arg Asp Ile
65 70 75 80

Ala Thr Trp Asn Arg Asp His Asn Leu Ile Thr Ala Met Lys Tyr Ser
85 90 95

Val Val Pro Val Tyr Gln Glu Phe Ala Arg Gln Ile Gly Glu Ala Arg
100 105 110

Met Ser Lys Met Leu His Ala Phe Asp Tyr Gly Asn Glu Asp Ile Ser
115 120 125

Gly Asn Leu Asp Thr Phe Trp Leu Asp Gly Gly Ile Arg Ile Ser Ala

130 135 140

Thr Glu Gln Ile Ser Phe Leu Arg Lys Leu Tyr His Asn Lys Leu His
145 150 155 160

Val Ser Glu Arg Ser Gln Arg Ile Val Lys Gln Ala Met Leu Thr Glu
165 170 175

Ala Asn Gly Asp Tyr Ile Ile Arg Ala Lys Thr Gly Tyr Ser Thr Arg
180 185 190

Ile Glu Pro Lys Ile Gly Trp Trp Val Gly Trp Val Glu Leu Asp Asp
195 200 205

Asn Val Trp Phe Phe Ala Met Asn Met Asp Met Pro Thr Ser Asp Gly
210 215 220

Leu Gly Leu Arg Gln Ala Ile Thr Lys Glu Val Leu Lys Gln Glu Lys
225 230 235 240

Ile Ile Pro

<210> 49
<211> 243
<212> PRT
<213> Shewanella sp. W17

<400> 49

Lys Glu Trp Gln Glu Asn Lys Ser Trp Asn Ala His Phe Thr Glu His
1 5 10 15

Lys Ser Gln Gly Val Val Ala Leu Trp Asn Glu Asn Lys Gln Gln Gly
20 25 30

Phe Thr Asn Asn Leu Lys Arg Ala Asn Gln Ala Phe Leu Pro Ala Ser
35 40 45

Thr Phe Lys Ile Pro Asn Ser Leu Ile Ala Leu Asp Leu Gly Val Val
50 55 60

Lys Asp Glu His Gln Val Phe Lys Trp Asp Gly Gln Thr Arg Asp Ile
65 70 75 80

Ala Thr Trp Asn Arg Asp His Asn Leu Ile Thr Ala Met Lys Tyr Ser
85 90 95

Val Val Pro Val Tyr Gln Glu Phe Ala Arg Gln Ile Gly Glu Ala Arg

100 105 110

Met Ser Lys Met Leu His Ala Phe Asp Tyr Gly Asn Glu Asp Ile Ser
115 120 125

Gly Asn Val Asp Ser Phe Trp Leu Asp Gly Gly Ile Arg Ile Ser Ala
130 135 140

Thr Glu Gln Ile Ser Phe Leu Arg Lys Leu Tyr His Asn Lys Leu His
145 150 155 160

Val Ser Glu Arg Ser Gln Arg Ile Val Lys Gln Ala Met Leu Thr Glu
165 170 175

Ala Asn Ser Asp Tyr Ile Ile Arg Ala Lys Thr Gly Tyr Ser Thr Arg
180 185 190

Ile Glu Pro Lys Ile Gly Trp Trp Val Gly Trp Val Glu Leu Asp Asp
195 200 205

Asn Val Trp Phe Phe Ala Met Asn Met Asp Met Pro Thr Ser Asp Gly
210 215 220

Leu Gly Leu Arg Gln Ala Ile Thr Lys Glu Val Leu Lys Gln Glu Lys
225 230 235 240

Ile Ile Pro

<210> 50
<211> 241
<212> PRT
<213> Klebsiella pneumoniae

<400> 50

Lys Glu Trp Gln Glu Asn Lys Ser Trp Asn Ala His Phe Thr Glu His
1 5 10 15

Lys Ser Gln Gly Val Val Val Leu Trp Asn Glu Asn Lys Gln Gln Gly
20 25 30

Phe Thr Asn Asn Leu Lys Arg Ala Asn Gln Ala Phe Leu Pro Ala Ser
35 40 45

Thr Phe Lys Ile Pro Asn Ser Leu Ile Ala Leu Asp Leu Gly Val Val
50 55 60

Lys Asp Glu His Gln Val Phe Lys Trp Asp Gly Gln Thr Arg Asp Ile

65 70 75 80

Ala Thr Trp Asn Arg Asp His Asn Leu Ile Thr Ala Met Lys Tyr Ser
85 90 95

Val Val Pro Val Tyr Gln Glu Phe Ala Arg Gln Ile Gly Glu Ala Arg
100 105 110

Met Ser Lys Met Leu His Ala Phe Asp Tyr Gly Asn Glu Asp Ile Ser
115 120 125

Gly Asn Val Asp Ser Phe Trp Leu Asp Gly Gly Ile Arg Ile Ser Ala
130 135 140

Thr Glu Gln Ile Ser Phe Leu Arg Lys Leu Tyr His Asn Lys Leu His
145 150 155 160

Val Ser Glu Arg Ser Gln Arg Ile Val Lys Gln Ala Met Leu Thr Glu
165 170 175

Ala Asn Gly Asp Tyr Ile Ile Arg Ala Lys Thr Gly Tyr Ser Thr Lys
180 185 190

Pro Lys Ile Gly Trp Trp Val Gly Trp Val Glu Leu Asp Asp Asn Val
195 200 205

Trp Phe Phe Ala Met Asn Met Asp Met Pro Thr Ser Asp Gly Leu Gly
210 215 220

Leu Arg Gln Ala Ile Thr Lys Glu Val Leu Lys Gln Glu Lys Ile Ile
225 230 235 240

Pro

<210> 51
<211> 243
<212> PRT
<213> Klebsiella pneumoniae

<400> 51

Lys Glu Trp Gln Glu Asn Lys Ser Trp Asn Ala His Phe Thr Glu His
1 5 10 15

Lys Ser Gln Gly Val Val Val Leu Trp Asn Glu Asn Lys Gln Gln Gly
20 25 30

Phe Thr Asn Asn Leu Lys Arg Ala Asn Gln Ala Phe Leu Pro Ala Ser

35 40 45

Thr Phe Lys Ile Pro Asn Ser Leu Ile Ala Leu Asp Leu Gly Val Val
50 55 60

Lys Asp Glu His Gln Val Phe Lys Trp Asp Gly Gln Thr Arg Asp Ile
65 70 75 80

Ala Thr Trp Asn Arg Asp His Asn Leu Ile Thr Ala Met Lys Tyr Ser
85 90 95

Val Leu Pro Val Tyr Gln Glu Phe Ala Arg Gln Ile Gly Glu Ala Arg
100 105 110

Met Ser Lys Met Leu His Ala Phe Asp Tyr Gly Asn Glu Asp Ile Ser
115 120 125

Gly Asn Val Asp Ser Phe Trp Leu Asp Gly Gly Ile Arg Ile Ser Ala
130 135 140

Thr Glu Gln Ile Ser Phe Leu Arg Lys Leu Tyr His Asn Lys Leu His
145 150 155 160

Val Ser Glu Arg Ser Gln Arg Ile Val Lys Gln Ala Met Leu Thr Glu
165 170 175

Ala Asn Gly Asp Tyr Ile Ile Arg Ala Lys Thr Gly Tyr Ser Thr Arg
180 185 190

Ile Glu Pro Lys Ile Gly Trp Trp Val Gly Trp Val Glu Leu Asp Asp
195 200 205

Asn Val Trp Phe Phe Ala Met Asn Met Asp Met Pro Thr Ser Asp Gly
210 215 220

Leu Gly Leu Arg Gln Ala Ile Thr Lys Glu Val Leu Lys Gln Glu Lys
225 230 235 240

Ile Ile Pro

<210> 52
<211> 243
<212> PRT
<213> Shewanella xiamenensis

<400> 52

Lys Glu Trp Gln Glu Asn Lys Ser Trp Asn Ala His Phe Thr Glu His

```
1               5                   10                  15

Lys Ser Gln Gly Val Val Val Leu Trp Asn Glu Asn Lys Gln Gln Gly
            20                  25                  30

Phe Thr Asn Asn Leu Lys Arg Ala Asn Gln Ala Phe Leu Pro Ala Ser
        35                  40                  45

Thr Phe Lys Ile Pro Asn Ser Leu Ile Ala Leu Asp Leu Gly Val Val
    50                  55                  60

Lys Asp Glu His Gln Val Phe Lys Trp Asp Gly Gln Thr Arg Asp Ile
65                  70                  75                  80

Ala Thr Trp Asn Arg Asp His Asn Leu Ile Thr Ala Met Lys Tyr Ser
                85                  90                  95

Val Val Pro Val Tyr Gln Glu Phe Ala Arg Gln Ile Gly Glu Ala Arg
            100                 105                 110

Met Ser Lys Met Leu His Ala Phe Asp Tyr Gly Asn Glu Asp Ile Ser
        115                 120                 125

Gly Asn Val Asp Ser Phe Trp Leu Asp Gly Gly Ile Arg Ile Ser Ala
    130                 135                 140

Thr Glu Gln Ile Ser Phe Leu Arg Lys Leu Tyr His Asn Lys Leu His
145                 150                 155                 160

Val Ser Glu Arg Ser Gln Arg Ile Val Lys Gln Ala Met Leu Thr Glu
                165                 170                 175

Ala Asn Ala Asp Tyr Ile Ile Arg Ala Lys Thr Gly Tyr Ser Gly Arg
            180                 185                 190

Ile Glu Pro Lys Phe Gly Trp Trp Val Gly Trp Val Glu Leu Asp Asp
        195                 200                 205

Asn Val Trp Phe Phe Ala Met Asn Met Asp Met Pro Thr Ser Asp Gly
    210                 215                 220

Leu Gly Leu Arg Gln Ala Ile Thr Lys Glu Val Leu Lys Gln Glu Lys
225                 230                 235                 240

Ile Ile Pro
```

<210> 53
<211> 243
<212> PRT
<213> Shewanella xiamenensis

<400> 53

Arg Glu Trp Gln Glu Asn Lys Ser Trp Asn Val His Phe Thr Glu His
1 5 10 15

Lys Ser Gln Gly Val Val Val Leu Trp Asn Glu Asn Lys Gln Gln Gly
20 25 30

Phe Thr Asn Asn Leu Lys Arg Ala Asn Gln Ala Phe Leu Pro Ala Ser
35 40 45

Thr Phe Lys Ile Pro Asn Ser Leu Ile Ala Leu Asp Leu Gly Val Val
50 55 60

Lys Asp Glu His Gln Val Phe Lys Trp Asp Gly Gln Thr Arg Asp Ile
65 70 75 80

Ala Thr Trp Asn Arg Asp His Asn Leu Ile Thr Ala Met Lys Tyr Ser
85 90 95

Val Val Pro Val Tyr Gln Glu Phe Ala Arg Gln Ile Gly Glu Ala Arg
100 105 110

Met Ser Lys Met Leu His Ala Phe Asp Tyr Gly Asn Glu Asp Ile Ser
115 120 125

Gly Asn Val Asp Ser Phe Trp Leu Asp Gly Gly Ile Arg Ile Ser Ala
130 135 140

Ile Glu Gln Ile Ser Phe Leu Arg Lys Leu Tyr His Asn Lys Leu His
145 150 155 160

Val Ser Glu Arg Ser Gln Arg Ile Val Lys Gln Ala Met Leu Thr Glu
165 170 175

Ala Asn Gly Asp Tyr Ile Ile Arg Ala Lys Thr Gly Tyr Ser Thr Arg
180 185 190

Ile Glu Pro Lys Ile Gly Trp Trp Val Gly Trp Val Glu Leu Asp Asp
195 200 205

Asn Val Trp Phe Phe Ala Met Asn Met Asp Met Pro Thr Ser Asp Gly
210 215 220

Leu Gly Leu Arg Gln Ala Ile Thr Lys Glu Val Leu Lys Gln Glu Lys
225 230 235 240

Ile Ile Pro

<210> 54
<211> 243
<212> PRT
<213> Shewanella xiamenensis

<400> 54

Lys Glu Trp Gln Glu Asn Lys Ser Trp Asn Ala His Phe Thr Glu His
1 5 10 15

Lys Ser Gln Gly Val Val Ala Leu Trp Asn Glu Asn Lys Gln Gln Gly
20 25 30

Phe Thr Asn Asn Leu Lys Arg Ala Asn Gln Ala Phe Leu Pro Ala Ser
35 40 45

Thr Phe Lys Ile Pro Asn Ser Leu Ile Ala Leu Asp Leu Gly Val Val
50 55 60

Lys Asp Glu His Gln Val Phe Lys Trp Asp Gly Gln Thr Arg Asp Ile
65 70 75 80

Ala Thr Trp Asn Arg Asp His Asn Leu Ile Thr Ala Met Lys Tyr Ser
85 90 95

Val Val Pro Val Tyr Gln Glu Phe Ala Arg Gln Ile Gly Glu Ala Arg
100 105 110

Met Ser Lys Met Leu His Ala Phe Asp Tyr Gly Asn Glu Asp Ile Ser
115 120 125

Gly Asn Val Asp Ser Phe Trp Leu Asp Gly Gly Ile Arg Ile Ser Ala
130 135 140

Ile Glu Gln Ile Ser Phe Leu Arg Lys Leu Tyr His Asn Lys Leu His
145 150 155 160

Val Ser Glu Arg Ser Gln Arg Ile Val Lys Gln Ala Met Leu Thr Glu
165 170 175

Ala Asn Gly Asp Tyr Ile Ile Arg Ala Lys Thr Gly Tyr Ser Thr Arg
180 185 190

```
Ile Glu Pro Lys Ile Gly Trp Trp Val Gly Trp Val Glu Leu Asp Asp
        195                 200                 205

Asn Val Trp Phe Phe Ala Met Asn Met Asp Met Pro Thr Ser Asp Gly
    210                 215                 220

Leu Gly Leu Arg Gln Ala Ile Thr Lys Glu Val Leu Lys Gln Glu Lys
225                 230                 235                 240

Ile Ile Pro



<210>  55
<211>  243
<212>  PRT
<213>  Escherichia coli

<400>  55

Lys Glu Trp Gln Glu Asn Lys Ser Trp Asn Ala His Phe Thr Glu His
1               5                   10                  15

Lys Ser Gln Gly Val Val Val Leu Trp Asn Glu Asn Lys Gln Gln Gly
            20                  25                  30

Phe Thr Asn Asn Leu Lys Arg Ala Asn Gln Ala Phe Leu Pro Ala Ser
        35                  40                  45

Thr Phe Lys Ile Pro Asn Ser Leu Ile Ala Leu Asp Leu Gly Val Val
    50                  55                  60

Lys Asp Glu His Gln Val Phe Lys Trp Asp Gly Gln Thr Arg Asp Ile
65                  70                  75                  80

Ala Thr Trp Asn Arg Asp His Asn Leu Ile Thr Ala Met Lys Tyr Ser
                85                  90                  95

Val Val Pro Val Tyr Gln Glu Phe Ala Arg Gln Ile Gly Glu Ala Arg
            100                 105                 110

Met Ser Lys Met Leu His Asp Phe Asp Tyr Gly Asn Glu Asp Ile Ser
        115                 120                 125

Gly Asn Val Asp Ser Phe Trp Leu Asp Gly Gly Ile Arg Ile Ser Ala
    130                 135                 140

Thr Glu Gln Ile Ser Phe Leu Arg Lys Leu Tyr His Asn Lys Leu His
145                 150                 155                 160
```

Val Ser Glu Arg Ser Gln Arg Ile Val Lys Gln Ala Met Leu Thr Glu
165 170 175

Ala Asn Gly Asp Tyr Ile Ile Arg Ala Lys Thr Gly Tyr Ser Thr Arg
180 185 190

Ile Glu Pro Lys Ile Gly Trp Trp Val Gly Trp Val Glu Leu Asp Asp
195 200 205

Asn Val Trp Phe Phe Ala Met Asn Met Asp Met Pro Thr Ser Asp Gly
210 215 220

Leu Gly Leu Arg Gln Ala Ile Thr Lys Glu Val Leu Lys Gln Glu Lys
225 230 235 240

Ile Ile Pro

<210> 56
<211> 243
<212> PRT
<213> Shewanella sp. FDAARGOS_354

<400> 56

Lys Glu Trp Gln Glu Asn Lys Ser Trp Asn Ala His Phe Thr Glu His
1 5 10 15

Lys Ser Gln Gly Val Val Ala Leu Trp Asn Glu Asn Lys Gln Gln Gly
20 25 30

Phe Thr Asn Asn Leu Lys Arg Ala Asn Gln Ala Phe Leu Pro Ala Ser
35 40 45

Thr Phe Lys Ile Pro Asn Ser Leu Ile Ala Leu Asp Leu Gly Val Val
50 55 60

Lys Asp Glu His Gln Val Phe Lys Trp Asp Gly Gln Thr Arg Asp Ile
65 70 75 80

Ala Thr Trp Asn Arg Asp His Asn Leu Ile Thr Ala Met Lys Tyr Ser
85 90 95

Val Val Pro Val Tyr Gln Glu Phe Ala Arg Gln Ile Gly Glu Ala Arg
100 105 110

Met Ser Lys Met Leu His Ala Phe Asp Tyr Gly Asn Glu Asp Ile Ser
115 120 125

```
Gly Asn Val Asp Ser Phe Trp Leu Asp Gly Gly Ile Arg Ile Ser Ala
    130                 135                 140

Thr Glu Gln Ile Ser Phe Leu Arg Lys Leu Tyr His Asn Lys Leu His
145                 150                 155                 160

Val Ser Glu Arg Ser Gln Arg Ile Val Lys Gln Ala Met Leu Thr Glu
                165                 170                 175

Ala Asn Gly Asp Tyr Ile Ile Arg Ala Lys Thr Gly Tyr Ser Thr Arg
            180                 185                 190

Ile Glu Pro Lys Ile Gly Trp Trp Val Gly Trp Val Glu Leu Asp Asp
        195                 200                 205

Asn Val Trp Phe Phe Ala Met Asn Met Asp Met Pro Thr Ser Asp Gly
    210                 215                 220

Leu Gly Leu Arg Gln Ala Ile Thr Lys Glu Val Leu Lys Gln Glu Lys
225                 230                 235                 240

Ile Ile Pro
```

## Description

### FIELD OF THE INVENTION

The invention relates to methods for measuring carbapenem antibiotics. The invention is particularly important to the monitoring and adjusting of a carbapenem antibiotic dosage provided to a subject suffering from an infection treatable with carbapenem antibiotics.

### BACKGROUND OF THE INVENTION

Antibacterial drugs are facing increasing limitations in terms of effectiveness not only due to emergence of high-level resistance but also due to a moderate but significant decrease in bacterial susceptibility (e.g. low level resistance). These expose the patients to a risk of sub-therapeutic dosages that explains treatment failures and further emergence of drug resistance. To mitigate these risks, clinicians tend to increase drug dosages and/or to combine antibiotics, which then expose the patients to potential toxicity while not necessarily minimizing efficiently drug resistance.

One way to improve this situation is to refine clinical treatment interventions, in terms of dosages and duration. Real-time therapeutic drug monitoring of the antimicrobials in individual patients should allow fine-tuning drug regimens (including dosages and schedules of administration) to meet patient-specific pharmacological requirements for activity (pharmacodynamics) while, at the same time decreasing the risk of emergence of drug resistance and controlling toxicity (toxicodynamics). What is still missing today for implementing beta-lactam therapeutic drug monitoring is the possibility for the clinicians to obtain a rapid assessment of drug levels. All methods available so far rely on complex technologies (mainly HPLC and LC-MS-MS) that take several hours before results can be communicated. Since the patient's situation is quickly changing over time, results that come late tend to be ignored. It is essential to provide the clinician with grounds for dosage adjustments as early as possible in the treatment process and at the level of the individual patient. Therefore, there is a clear unmet need to provide the clinicians with a direct, rapid and/or real-time information about free β-lactam actual blood levels both at initiation and during therapy.

WO2013053953 describes a method for measuring beta-lactam antibiotics that involves contacting a class C beta-lactamase with a biological sample. The beta-lactam antibiotic present in the sample will form a covalent adduct with the class C beta-lactamase. It has further been disclosed that the hydrolysis rate of the formed adduct is slow enough to allow the measurement of this complex by spectroscopic methods but rapid enough to allow the self-regeneration of the active enzyme which is advantageous to perform successive assays. These particular kinetic features are in contrast with class A, class B and class D beta-lactamases.

The problem with such method is that in presence of more than one beta-lactam antibiotic, there is a need to use a mixture of two or more enzymes to quantify the individual concentration of two or more beta-lactam antibiotics. In the case of patients in intensive care units, such cases are frequent.

A beta-lactam antibiotic that is frequently encountered in such patients, is meropenem. It is the most widely used antibiotic in nosocomial broncho-pulmonary infections due to multidrug-resistant Gram-negative bacilli including, in particular, broad-spectrum beta-lactamase producing enterobacteria or Amp-C, *Pseudomonas aeruginosa,* and *Acinetobacter baumannii.* Because these infections are difficult to treat, there is a need to optimize the modalities of antibiotic treatment and therefore to be able to determine the meropenem antibiotic concentration even in presence of other beta-lactam antibiotics.

Accordingly, a need exists to develop further and improved methods for the quantification of a carbapenem antibiotics in samples, such as complex biological samples.

### SUMMARY OF THE INVENTION

The invention provides technology adapted to the measurement, including quantitative analysis, of a carbapenem antibiotic, in particular meropenem, in a biological sample, e.g. in the presence of other beta-lactam antibiotics and/or other pharmaceutical substances.

In a first aspect, the invention provides a method for determining the concentration of a carbapenem antibiotic in a biological sample, comprising the steps of:

(a) providing a blaOXA-48 class D beta-lactamase (blaOXA-48) or a functionally active variant or fragment thereof;

(b) providing a biological sample;

(c) contacting the blaOXA-48 or functionally active variant or fragment thereof with the biological sample; and

(d) determining the concentration of the carbapenem antibiotic in the biological sample.

As illustrated in the example section, the inventors have found that the beta-lactamase class D OXA-48 is an efficient biosensor for the determination of carbapenem-type antibiotic concentration in serum. This biosensor allows the monitoring of carbapenem-type antibiotic in the presence of large excess of other beta-lactam antibiotics as well as other drugs, commonly used in intensive care units, due to a high affinity of beta-lactamase class D OXA-48 for meropenem (i.e. low K or $K_m$) combined with a slow formation of the covalent adduct during catalysis (i.e. low $k_2$). These characteristics are not encountered for the other commonly used beta-lactam antibiotics. This unique property was shown after extensive evaluation of the kinetic properties of more than fifteen other different beta-lactamases which were not suitable for selectively measuring a carbapenem antibiotic, in particular meropenem, in the presence of other beta-lactam antibiotics.

In the prior art, it was shown that the hydrolysis rate of the beta-lactam antibiotic/class C beta-lactamase complex (acyl-enzyme complex) can be slow enough (i.e. low $k_3$) to allow the measurement of this complex for instance by spectroscopic methods, but rapid enough to allow the self-regeneration of the active enzyme. In the case where several beta-lactam antibiotics are present, there are as many possible substrates for the active site of the class C beta-lactamase as there are different beta-lactam antibiotics, and hence there are several possible reactions. It is therefore problematic to determine directly the concentration of a carbapenem antibiotic in the presence of other beta-lactam antibiotics with a class C beta-lactamase biosensor.

With a blaOXA-48 class D beta-lactamase, no competition with other beta-lactam antibiotics was observed in determining the concentration of the carbapenem antibiotic, in particular meropenem. This is surprising as based on the known kinetic constants from the prior art, it would be expected that, while determining the carbapenem antibiotic, in particular meropenem, a competition with another beta-lactam antibiotics, for instance ampicillin, would be present. The experimental result herein show that this was not the case: the difference of absorbance as measured by a spectroscopic method was only due to the carbapenem antibiotic, in particular meropenem.

The present method allows a rapid and simple quantitative analysis of free carbapenem antibiotics in biological samples. Furthermore, the present method advantageously allows for the quantification of carbapenem antibiotics that is applicable to clinical practice. For instance, the present method allows real-time monitoring of carbapenem antibiotics in biological samples such as biological fluids, in particular serum. The present method allows repeated blood level monitoring and rapid delivery of the results to the clinician in order to ensure optimal treatment of a subject.

The above and other characteristics, features and advantages of the present invention will become apparent from the following detailed description, which illustrate, by way of example, the principles of the invention.

## DETAILED DESCRIPTION OF THE INVENTION

As used herein, the singular forms "a", "an", and "the" include both singular and plural referents unless the context clearly dictates otherwise.

The terms "comprising", "comprises" and "comprised of' as used herein are synonymous with "including", "includes" or "containing", "contains", and are inclusive or open-ended and do not exclude additional, non-recited members, elements or method steps. The terms also encompass "consisting of" and "consisting essentially of", which enjoy well-established meanings in patent terminology.

The recitation of numerical ranges by endpoints includes all numbers and fractions subsumed within the respective ranges, as well as the recited endpoints.

The terms "about" or "approximately" as used herein when referring to a measurable value such as a parameter, an amount, a temporal duration, and the like, are meant to encompass variations of and from the specified value, such as variations of ± 10% or less, preferably ± 5% or less, more preferably ± 1% or less, and still more preferably ± 0.1% or less of and from the specified value, insofar such variations are appropriate to perform in the disclosed invention. It is to be understood that the value to which the modifier "about" refers is itself also specifically, and preferably, disclosed.

Whereas the terms "one or more" or "at least one", such as one or more members or at least one member of a group of members, is clear per se, by means of further exemplification, the term encompasses inter alia a reference to any one of said members, or to any two or more of said members, such as, e.g., any 3 or more, 4 or more, 5 or more, 6 or more, or 7 or more etc. of said members, and up to all said members. In another example, "one or more" or "at least one" may refer to 1, 2, 3, 4, 5, 6, 7 or more.

The discussion of the background to the invention herein is included to explain the context of the invention. This is not to be taken as an admission that any of the material referred to was published, known, or part of the common general knowledge in any country as of the priority date of any of the claims.

Throughout this disclosure, various publications, patents and published patent specifications are referenced by an identifying citation. All documents cited in the present specification are hereby incorporated by reference in their entirety. In particular, the teachings or sections of such documents herein specifically referred to are incorporated by

reference.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the invention. When specific terms are defined in connection with a particular aspect of the invention or a particular embodiment of the invention, such connotation is meant to apply throughout this specification, i.e., also in the context of other aspects or embodiments of the invention, unless otherwise defined.

In the following passages, different aspects or embodiments of the invention are defined in more detail. Each aspect or embodiment so defined may be combined with any other aspect(s) or embodiment(s) unless clearly indicated to the contrary. In particular, any feature indicated as being preferred or advantageous may be combined with any other feature or features indicated as being preferred or advantageous.

Reference throughout this specification to "one embodiment", "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the claimed embodiments can be used in any combination.

The present invention relates to an improved method for measuring and/or monitoring the level of free carbapenem antibiotics in a biological sample. By extensive experimental testing, the present inventors realised that the beta-lactamase class D OXA-48 is an efficient biosensor for the determination of a carbapenem antibiotic concentration in a biological sample such as serum, thereby allowing the monitoring of a carbapenem antibiotic in presence of large excess of other beta-lactam antibiotics as well as other drugs, commonly used in intensive care units (ICUs).

In a first aspect, the present invention relates to a method for measuring a carbapenem antibiotic in a biological sample, comprising the steps of:

(a) providing a blaOXA-48 class D beta-lactamase (blaOXA-48) or a functionally active variant or fragment thereof;

(b) providing a biological sample;

(c) contacting the blaOXA-48 or functionally active variant or fragment thereof with the biological sample; and

(d) measuring the carbapenem antibiotic in the biological sample.

The recitation "measuring a carbapenem antibiotic" generally refers to determining the presence, amount, quantity or concentration of the carbapenem antibiotic.

Preferably, the present invention relates to a method for determining the concentration of a free carbapenem antibiotic in a biological sample, comprising the steps of:

(a) providing a blaOXA-48 class D beta-lactamase (blaOXA-48) or a functionally active variant or fragment thereof;

(b) providing a biological sample;

(c) contacting the blaOXA-48 or functionally active variant or fragment thereof with the biological sample; and

(d) determining the concentration of the free carbapenem antibiotic in the biological sample.

The recitation "determining the concentration of a carbapenem antibiotic in a biological sample" may encompass quantifying and/or monitoring the concentration of a carbapenem antibiotic in a biological sample. The term "quantifying" refers to measuring and/or expressing the concentration of a carbapenem antibiotic. The term "monitoring" generally refers to quantifying the concentration of the carbapenem antibiotic over time. For instance, monitoring a carbapenem antibiotic in a biological sample may be performed by measuring the carbapenem antibiotic concentration at one or more successive time points.

In certain embodiments, the concentration of carbapenem antibiotic is a relative concentration. In a certain preferred embodiments, the concentration of carbapenem antibiotic is an absolute concentration. Advantageously, as illustrated in the example section, the methods embodying the principles of the present invention allow the determination

of absolute concentrations of a carbapenem antibiotic in a biological sample.

The recitation "determining the relative concentration" of a substance in a sample refers to the qualitative determination of the substance in the sample, i.e. determining whether the substance is present or absent in the sample.

The recitation "determining the absolute concentration" of a substance in a sample refers to the quantitative determination of the substance in the sample, i.e. determining the amount of the substance per volume of the sample. The determination of the absolute concentration of a carbapenem antibiotic in a sample may be performed by comparison with one or more standards of known concentration.

In certain embodiments of the methods or uses as taught herein, the concentration may be an absolute concentration.

The term "carbapenem antibiotic" or "carbapenem" refers to a class of beta-lactam antibiotics comprising the structure of Formula (I).

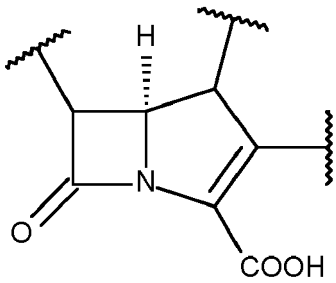

(I)

The terms "free" or "unbound" denote that the carbapenem antibiotic is not bound or attached to other molecules, particularly biological molecules such as proteins, present in the biological sample. Advantageously, the present method is adapted for measuring carbapenem antibiotic unbound to other biological molecules in biological samples. The present method is therefore advantageous in that it allows the measurement and/or monitoring of the biologically active fraction of the carbapenem antibiotic in a biological sample.

The term "beta-lactam antibiotic" as used herein refers to an antibiotic agent that comprises a beta-lactam ring in its molecular structure.

The term "beta-lactam ring" refers to a lactam or cyclic amide structure, as shown in Formula (II).

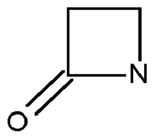

(II)

The reference to "a carbapenem antibiotic" encompasses one carbapenem antibiotic or a combination of two or more or three or more of thereof.

In certain embodiments of the methods or uses as taught herein, the carbapenem antibiotic may be one or more of meropenem, ertapenem, doripenem, or imipenem. In certain embodiments, the carbapenem antibiotic may be one or more of meropenem, ertapenem, or doripenem. In the clinic, the utilisation of two or more carbapenem antibiotics in the same patient is not common or recommended because the spectra of the carbapenem antibiotics is similar. Hence, in certain embodiments, the carbapenem antibiotic may be one of meropenem, ertapenem, doripenem, or imipenem. In certain embodiments, the carbapenem antibiotic may be one of meropenem, ertapenem, or doripenem. Preferably, the carbapenem antibiotic is meropenem.

In some embodiments, the carbapenem antibiotic may be active against multidrug-resistant (MDR) bacteria such as broad-spectrum beta-lactamase producing enterobacteria or Amp-C, *Pseudomonas aeruginosa,* and *Acinetobacter baumannii.* In some embodiments, the carbapenem antibiotic may be active against bacteria resistant to one or more of penicillin antibiotics, cephalosporin antibiotics, penem antibiotics or monobactam antibiotics, such as against beta-lactamase-producing bacteria.

The biological sample can be derived from a biological origin, such as from humans or non-human animals, preferably warm-blooded animals, even more preferably mammals, such as, e.g., non-human primates, rodents, canines, felines, equines, ovines, porcines, and the like. The term "non-human animals" includes all vertebrates, e.g., mammals, such as non-human primates, (particularly higher primates), sheep, dog, rodent (e.g. mouse or rat), guinea pig, goat, pig, cat, rabbits, cows, and non-mammals such as chickens, amphibians, reptiles etc. Preferably, the biological sample is derived from human origin.

The biological sample can be a biological fluid or a non-fluid biological sample. It should be understood that sample preparation before or during the method as taught herein can release the carbapenem antibiotic, for instance from a non-fluid biological sample. Hence, the method as taught herein may comprise the step of releasing the carbapenem antibiotic from a non-fluid biological sample such as a stool specimen.

Preferably, the biological sample is a biological fluid. The biological fluid can be selected from the group comprising serum, blood, urine, interstitial fluid, saliva, tears, exudates, fluid collected from deep tissues, fluid collected from subcutaneous tissues and other human fluids susceptible of containing carbapenem antibiotic. In certain embodiments of the methods or uses as taught herein, the biological sample may be selected from the group consisting of serum, blood, urine, interstitial fluid, saliva, tears, exudates, fluid collected from deep tissues, and fluid collected from subcutaneous tissues. Preferably, the biological sample is serum, blood, urine, or interstitial fluid, more preferably the biological sample is serum.

In certain embodiments of the methods or uses as taught herein, the biological sample may comprise a carbapenem antibiotic and one or more other beta-lactam antibiotics. In certain embodiments, the biological sample may comprise a carbapenem antibiotic and one or more other pharmaceutical substances. In certain embodiments, the biological sample may comprise a carbapenem antibiotic, one or more other beta-lactam antibiotics, and one or more other pharmaceutical substances. Advantageously, the present methods allow to determine the concentration of a carbapenem antibiotic in a biological sample, for instance in a microdialysate from an intensive care unit patient, in the presence of other beta-lactam antibiotics, such as other beta-lactam antibiotics commonly used in ICUs, and/or in the presence of other pharmaceutical substances, such as pharmaceutical substances commonly used in ICUs.

Hence, certain embodiments provide a method for determining the concentration of a carbapenem antibiotic in a biological sample comprising a carbapenem antibiotic and one or more other beta-lactam antibiotics. Certain further embodiments provide a method for determining the concentration of a carbapenem antibiotic in a biological sample comprising a carbapenem antibiotic and one or more other pharmaceutical substances.

In certain embodiments, the other beta-lactam antibiotics may be beta-lactam antibiotics commonly used in ICUs.

In certain embodiments, the other beta-lactam antibiotic may be selected from the group consisting of a penicillin, a cephalosporin, a penem, and a monobactam.

The term "penicillin", "penicillin antibiotic" or "penam" refers to a beta-lactam antibiotic comprising a thiazolidine ring.

The term "cephalosporin", "cephalosporin antibiotic" or "cephem" refers to a beta-lactam antibiotic comprising a 3,6-dihydro-2H-1,3-thiazine ring.

The term "penem" or "penem antibiotic" refers to a beta-lactam antibiotic comprising a 2,3-dihydrothiazole ring.

The term "monobactam" or "monobactam antibiotic" refers to a beta-lactam antibiotic comprising a beta-lactam ring not fused to any other ring.

Non-limiting examples of other beta-lactam antibiotics may be benzylpenicillin, phenoxymethylpenicillin, methicillin, oxacillin, nafcillin, cloxacillin, dicloxacillin, flucloxacillin, mezlocillin, piperacillin, ticarcillin, temocillin, amoxicillin, ampicillin, azlocillin, carbenicillin, cephalexin, cephalothin, cefadroxil, cefprozil, cefdinir, cefditoren, ceftibuten, cefoperazone, ceftizoxime, ceftobiprole, cefaclor, cefuroxime, cefamandole, cefotetan, cefoxitin, ceftaroline, ceftriaxone, cefotaxime, cefpodoxime, cefixime, ceftazidime, faropenem, aztreonam, tigemonam, or anocardicin A, or combination thereof.

The other beta-lactam antibiotics are commercially available as Augmentin, Azactam, Cefamandole, Cefotaxim (Sandoz), Ceftria, Claventin, Kefzol, Penicilline, Penstapho, Pentrexyl, Ticarpen, Zinacef, Cefepime, Ceftazidime, or Tazocin.

In certain embodiments, the other pharmaceutical substances may be pharmaceutical substances commonly used in ICUs.

In certain embodiments, the other pharmaceutical substances may be selected form the group consisting of a glucoregulator, an analgesic, an anesthetic, an antiarythmic, an antiasthmatic, an anticoagulant, an antiepileptic, an antifungal, an antihypertensive, an antiulcereus, an anxiolytic, a cardiotonic, a diuretic, an immunosuppressive, a regulator of hypocalcemia, a sedative, an anticonvulsive, a vasodilator, and an anti-ischemic.

In certain embodiments, the other pharmaceutical substances may be one or more of Actrapid, Adrenaline, Anidulafungin, Calciclo, Caspofungin, Clonazepam, Cordarone, Dapakine, Diazepam, Diphantoïne, Dobutrex, Dopamine, Fluconazole, Heparine Leo, Keppra, Lasix, Liposomial amphotericin B, Midazolam Braun, Milrinone, Morphine, Nimbex, Nimotop, Noradrenaline, Pantomed, Prograft, Propolipid, Protamine, Rydene, Salbutamol, Sandimmum,

Solu-cortef, Solu-Medrol, Sufentanil, Thiobarbital, Ultiva, or Voriconazole.

In certain embodiments, the biological sample may be a biological fluid that can be processed through microdialysis. In certain embodiments, the biological sample may be a microdialysate obtained from a subject receiving an antibiotherapy.

In certain embodiments of the methods or uses as taught herein, the biological sample may be a biological sample (obtained) from a subject receiving an antibiotherapy. In certain embodiments of the methods or uses as taught herein, the biological sample may be a biological sample (obtained) from a subject being treated for or in need of treatment of an infection caused by Gram-negative bacteria, Gram-positive bacteria and/or MDR bacteria, in particular broad-spectrum beta-lactamase producing enterobacteria or Amp-C, *Pseudomonas aeruginosa,* or *Acinetobacter baumannii.* In certain embodiments, the biological sample may be a biological sample (obtained) from a subject receiving an antibiotherapy and being treated for or in need of treatment of an infection caused by Gram-negative bacteria, Gram-positive bacteria and/or MDR bacteria.

In certain embodiments of the methods or uses as taught herein, the biological sample may be a biological sample (obtained) from a critically ill subject presenting at or present in an intensive care unit (ICU) or emergency department (ED). Critically ill subjects tend to be treated immediately by a combination of two or more beta-lactam antibiotics including a carbapenem. The present method advantageously allows real-time determination of the concentration of the carbapenem antibiotic in the presence of other beta-lactam antibiotics in a biological sample from a critically ill subject. Thereby the present method allows real-time therapeutic drug monitoring and fine-tuning drug regimens (e.g. dosage and schedule of administration).

The present method is particularly useful, for determining the concentration, in particular for measuring and/or monitoring the concentration of a carbapenem antibiotic in the treatment of an infection caused by Gram-negative bacteria, Gram-positive bacteria and/or MDR bacteria in a subject in need thereof.

The present method is based on the interaction between a blaOXA-48 class D beta-lactamase and a carbapenem antibiotic, in particular meropenem.

Scheme 1 shows the kinetic models of the reaction of the enzyme or biosensor (E) with the reporter substrate ($S_R$) and the beta-lactam analyte (A) (Frère et al Eur. J. Biochem. (1975) 57: 343-351).

$$E + S_R \overset{K}{\rightleftarrows} E.S_R \overset{k_2}{\longrightarrow} E - S_R \overset{k_3}{\longrightarrow} E + P_R$$

$$E + A \overset{K'}{\rightleftarrows} E.A \overset{k'_2}{\longrightarrow} E - A \overset{k'_3}{\longrightarrow} E + P_A$$

$$K = k_1/k_{-1},$$

$$K' = k'_1/k'_{-1}.$$

Scheme 1 wherein E is the enzyme (blaOXA-48 class D beta-lactamase), $S_R$ is the reporter substrate, A is the carbapenem antibiotic (analyte) acting as competitive inhibitor, $E.S_R$ and E.A are Michaelis-Menten complexes (non-covalent interactions), $E\text{-}S_R$ and E-A are acyl-enzyme adducts (covalent adduct), $P_R$ and $P_A$ are products of reaction (here hydrolysed carbapenem antibiotic), $k_2$ and $k'_2$ are the constants for acylation steps, $k_3$ and $k'_3$ are the constants of the deacylation steps. K and K' are constants of non-covalent complex formation.

The rate equation describing the kinetic model can be resumed to the following equation:

$$\frac{v_{max}^{S_R}}{v_0^A} - 1 = \alpha . A \quad \text{(equation 1)}$$

Where $v_{max}^{S_R}$ is the initial velocity of hydrolysis of $S_R$ by the biosensor in absence of analyte; $v_0^A$ is the initial velocity of hydrolysis of $S_R$ by the biosensor in presence of analyte; *a* is a linear coefficient; A is the analyte to monitor.

The blaOXA-48 class D beta-lactamase when contacted with a carbapenem antibiotic, features kinetic parameters such that the enzyme remains mostly in the first non-covalent complex (E.A in Scheme 1), due to a high affinity of blaOXA-48 for a carbapenem antibiotic (low K' in Scheme 1) combined with a slow formation of the covalent adduct during catalysis (low $k_2$' in Scheme 1). These characteristics are not encountered for the other commonly used beta-lactam antibiotics.

The particular kinetic features of blaOXA-48 and carbapenem antibiotic (i.e. high affinity of blaOXA-48 for carbapenem antibiotic such as meropenem and a slow formation of the covalent adduct during catalysis) results in a temporary immobilisation of the enzyme in a non-covalent complex (E.A in Scheme 1). The inventors have surprisingly found that the non-covalent complex (Michaelis-Menten complex) lasts long enough to allow its measurement for instance by spectroscopic methods, but the enzyme still hydrolyses the carbapenem antibiotic fast enough to allow the self-regeneration of the enzyme which is advantageous to perform successive assays.

The term "self-regeneration" as used herein refers to the capacity of the enzyme (blaOXA-48 class D beta-lactamase) to repeatedly perform its function i.e. bind, process and release a carbapenem antibiotic.

The present method can take advantage of the kinetic features of blaOXA-48 class D beta-lactamases to allow the determination of the concentration of a carbapenem antibiotic, as illustrated in the example section. Thereby, the present method advantageously allows the label-free measurement of unbound carbapenem antibiotic in a biological sample.

In certain embodiments, the method as taught herein comprises the step (a) of providing a blaOXA-48 class D beta-lactamase (blaOXA-48) or a functionally active variant or fragment thereof.

The terms "beta-lactamases" "beta-lactamase enzymes", beta-lactamase polypeptides" or "beta-lactamase proteins" may be used interchangeably herein and generally refer to a class of proteins, in particular to a class of enzymes (EC 3.5.2.6) which are able to open a beta-lactam ring. Typically, beta-lactamases are produced by bacteria and provide multi-resistance to beta-lactam antibiotics.

As used herein, the term "class D", "OXA" or "group 2d" beta-lactamase, refers to a class of beta-lactamases which (i) in the Ambler primary structural classification belong to the molecular class D (Ambler, Philos. Trans. R. Soc. Lond. B. Biol. Sci., 1980, 289: 321-31) and (ii) in the functional classification of Bush et al. belong to group 2d (Antimicrob. Agents Chemother., 1995, 39, 1211-33).

The terms "blaOXA-48", blaOXA-48 beta-lactamase", "blaOXA-48 class D beta-lactamase" or "OXA-48 family class D beta-lactamase" can be used interchangeably herein and refer to OXA-48 family carbapenem-hydrolysing class D beta-lactamases (EC:3.5.2.6). The term "blaOXA-48" as used herein encompasses beta-lactamase class D OXA-48 and functional orthologs thereof.

Beta-lactamase class D OXA-48 from *Klebsiella pneumoniae* (strain: Kp11978) may be defined as an OXA beta-lactamase isolated in Turkey in 2011 and found to be resistant to all beta-lactams, including carbapenems. Beta-lactamase class D OXA-48 is remotely related to the other oxacillinases. Beta-lactamase class D OXA-48 hydrolyzes penicillins and imipenem but not expanded-spectrum cephalosporins (Poirel et al., Antimicrob. Agents Chemother., 2004, 48:15-22).

The terms "beta-lactamase class D OXA-48", "beta-lactamase class D OXA-48 from *Klebsiella pneumoniae* (strain: Kp11978)", *"Klebsiella pneumoniae* beta-lactamase class D OXA-48", *"Klebsiella pneumoniae* OXA-48" or "OXA-48" can be used interchangeably herein.

Exemplary beta-lactamase class D OXA-48 protein sequence may be as annotated under U.S. government's National Center for Biotechnology Information (NCBI) NCBI Reference Sequence (http://www.ncbi.nlm.nih.gov/) WP_015059991.1 (sequence version 1), or Swissprot/Uniprot (http://www.uniprot.org/) accession number Q6XEC0-1 (sequence version 1) or entry Q6XEC0_KLEPN. Exemplary beta-lactamase class D OXA-48 genomic sequence may be as annotated under NCBI Reference Sequence NG 049762.1 (sequence version 1).

The OXA-48 amino acid sequence annotated under NCBI Reference Sequence WP_015059991.1 is reproduced below:

MRVLALSAVFLVASIIGMPAVAKEWQENKSWNAHFTEHKSQGVVVLWNENKQQGFTNNLKRA
NQAFLPASTFKIPNSLIALDLGVVKDEHQVFKWDGQTRDIATWNRDHNLITAMKYSVVPVYQEF
ARQIGEARMSKMLHAFDYGNEDISGNVDSFWLDGGIRISATEQISFLRKLYH**NKLHVSE**RSQRIV
KQAMLTEANGDYIIRAKTGYSTRIEPKIGWWVGWVELDDNVWFFAMNMDMPTSDGLGLRQAI TKEVLKQEKIIP (SEQ ID NO: 1, underlined: signal peptide, bold underlined: conserved sequence for modification).

The above representative *Klebsiella pneumoniae* OXA-48 polypeptide sequence is that of a OXA-48 precursor, including an N-terminal signal peptide. During processing of *Klebsiella pneumoniae* OXA-48, the signal peptide, corresponding to amino acids 1 to 22 in SEQ ID NO: 1, is processed away to form the mature *Klebsiella pneumoniae* OXA-48 protein, corresponding to amino acids 23 to 265 of SEQ ID NO: 1, which is thus 243 amino acids long.

Hence, the amino acid sequence of an exemplary mature *Klebsiella pneumoniae* OXA-48 is reproduced below:

KEWQENKSWNAHFTEHKSQGVVVLWNENKQQGFTNNLKRANQAFLPASTFKIPNSLIALDLGV
VKDEHQVFKWDGQTRDIATWNRDHNLITAMKYSVVPVYQEFARQIGEARMSKMLHAFDYGNE

DISGNVDSFWLDGGIRISATEQISFLRKLYH**NKLHVSE**RSQRIVKQAMLTEANGDYIIRAKTGYST RIEPKIGWWVGWVELDDNVWFFAMNMDMPTSDGLGLRQAITKEVLKQEKIIP (SEQ ID NO: 29, bold underlined: conserved sequence for modification).

The qualifier *"Klebsiella pneumoniae"* as used herein in connection with the OXA-48 polypeptide relates to the primary amino acid sequence of the OXA-48 polypeptide, rather than to its origin or source. For example, the *Klebsiella pneumoniae* OXA-48 polypeptide may be obtained by technical means, e.g., by recombinant expression, cell-free translation, or non-biological peptide synthesis.

The terms "peptide", "polypeptide" or "protein" can be used interchangeably and relate to any natural, synthetic, or recombinant molecule comprising amino acids joined together by peptide bonds between adjacent amino acid residues. A "peptide bond", "peptide link" or "amide bond" is a covalent bond formed between two amino acids when the carboxyl group of one amino acid reacts with the amino group of the other amino acid, thereby releasing a molecule of water. The polypeptide can be from any source, e.g., a naturally occurring polypeptide, a chemically synthesized polypeptide, a polypeptide produced by recombinant molecular genetic techniques, or a polypeptide from a cell or translation system. Preferably, the polypeptide is a polypeptide produced by recombinant molecular genetic techniques. The polypeptide may be a linear chain or may be folded into a globular form. Preferably, the blaOXA-48 class D beta-lactamases are folded into a globular form. The terms "amino acid" or "amino acid residue" may be used interchangeably herein.

The term "recombinant" is generally used to indicate that the material (e.g., a nucleic acid, a genetic construct or a protein) has been altered by technical means (i.e., non-naturally) through human intervention. The term "recombinant nucleic acid" can commonly refer nucleic acids comprised of segments joined together using recombinant DNA technology. As used herein, the term may preferably denote material (e.g., a nucleic acid, a genetic construct or a protein) that has been altered by technical means of mutagenesis. As used herein the term "recombinant protein or polypeptide" refers to a protein or polypeptide that can result from the expression of recombinant nucleic acid such as recombinant DNA.

By "nucleic acid" is meant oligomers and polymers of any length composed essentially of nucleotides, e.g., deoxyribonucleotides and/or ribonucleotides. Nucleic acids can comprise purine and/or pyrimidine bases and/or other natural (e.g., xanthine, inosine, hypoxanthine), chemically or biochemically modified (e.g., methylated), non-natural, or derivatised nucleotide bases. The backbone of nucleic acids can comprise sugars and phosphate groups, as can typically be found in RNA or DNA, and/or one or more modified or substituted sugars and/or one or more modified or substituted phosphate groups. Modifications of phosphate groups or sugars may be introduced to improve stability, resistance to enzymatic degradation, or some other useful property. A "nucleic acid" can be for example doublestranded, partly double stranded, or single-stranded. Where single-stranded, the nucleic acid can be the sense strand or the antisense strand. In addition, nucleic acid can be circular or linear. The term "nucleic acid" as used herein preferably encompasses DNA and RNA, specifically including RNA, genomic RNA, cDNA, DNA, provirus, pre-mRNA and mRNA.

The functional orthologues (orthologs) of beta-lactamase class D OXA-48 may be as annotated under KEGG Annotation (KO-based annotation for linking genomes to phenotypes) (https://www.genome.jp/kegg/annotation/). Exemplary functional orthologs of beta-lactamase class D OXA-48 may be as annotated under KO (KEGG Orthology) database Entry K18976 (https://www.kegg.jp/dbget-bin/www_bget?ko:K18976). Exemplary functional orthologs of beta-lactamase class D OXA-48 may be as annotated under KO database Addendum Entry (or NCBI-ProteinID) (Gene name between brackets): AAP70012 (OXA-48), AAR89917 (OXA-54), ADG27454 (OXA-162), ADY06444 (OXA-163), AEP16366 (OXA-181), AFC95894 (OXA-199), AFU91598 (OXA-204), AGC60012 (OXA-244), AGC60013 (OXA-245), AGC70814 (OXA-247), AGD91915 (OXA-232), AHF71363 (OXA-370), AJA30430 (OXA-405), AKH90740 (OXA-416), AKL59521 (OXA-438), AKR53961 (OXA-439), ALI16502 (OXA-484), ALN39132 (OXA-436), ALZ40809 (OXA-505), AMN85820 (OXA-514), AMN85821 (OXA-515), AMO66558 (OXA-517), ANI25017 (OXA-519), APB92846 (OXA-538), AQW34754 (OXA-547), AQX43454 (OXA-546), ASC55290 (OXA-566), and ASF15667 (OXA-252) (https://www.genome.jp/kegg/seq/tree/br01553d.html).

Exemplary protein sequences as taught herein may be as annotated under NCBI Genbank (http://www.ncbi.nlm.nih.gov/) or Swissprot/Uniprot (http://www.uniprot.org/) as given below. A skilled person will appreciate that although only one or more isoforms may be listed below, all isoforms are intended. The entries in Table 1 are presented in the form: Gene, KO Entry (or NCBI-ProteinID), Organism, Swissprot/Uniprot accession number followed by the sequence version, Swissprot/Uniprot entry name, NCBI Reference Sequence for a representative amino acid sequence followed by the sequence version, SEQ ID NO of the representative amino acid sequence, and NCBI Reference Sequence for a representative genomic sequence followed by the sequence version.

Table 1: Exemplary sequences of bla-OXA48 class D beta-lactamases

| Gene | KO Entry | Organism | UniProt Accession number | UniProt Entry name | NCBI RefSeq Protein | SEQ ID NO: | NCBI RefSeq Genomic |
|---|---|---|---|---|---|---|---|
| OXA-48 | AAP70012 | Klebsiella pneumoniae | Q6XEC0-1 | Q6XEC0_KLEPN | WP_015059991.1 | 1 | NG_049762.1 |
| OXA-54 | AAR89917 | Shewanella oneidensis | Q6RFZ0-1 | Q6RFZ0_SHEOE | WP_011071128.1 | 2 | NG_049794.1 |
| OXA-162 | ADG27454 | Klebsiella pneumoniae | D6QY24-1 | D6QY24_KLEPN | WP_060613455.1 | 3 | NG_049461.1 |
| OXA-163 | ADY06444 | Enterobacter cloacae | F6KZJ2-1 | F6KZJ2_ENTCL | WP_063131934.1 | 4 | NG_049462.1 |
| OXA-181 | AEP16366 | Klebsiella pneumoniae | G5CKK8-1 | G5CKK8_KLEPN | WP_015060052.1 | 5 | NG_049482.1 |
| OXA-199 | AFC95894 | Shewanella xiamenensis | H9A1B4-1 | H9A1B4_9GAMM | WP_063861505.1 | 6 | NG_049495.1 |
| OXA-204 | AFU91598 | Klebsiella pneumoniae | K4JY39-1 | K4JY39_KLEPN | WP_032495449.1 | 7 | NG_049502.1 |
| OXA-244 | AGC60012 | Klebsiella pneumoniae | L7UXE1-1 | L7UXE1_KLEPN | WP_032495517.1 | 8 | NG_049539.1 |
| OXA-245 | AGC60013 | Klebsiella pneumoniae | L7V1I2-1 | L7V1I2_KLEPN | WP_032495518.1 | 9 | NG_049540.1 |
| OXA-247 | AGC70814 | Klebsiella pneumoniae | L7VTN5-1 | L7VTN5_KLEPN | WP_032495595.1 | 10 | NG_049542.1 |
| OXA-232 | AGD91915 | Escherichia coli | M4JTK1-1 | M4JTK1_ECOLX | WP_043907054.1 | 11 | NG_049528.1 |
| OXA-370 | AHF71363 | Enterobacter sp. 87F-2 | WOHE56-1 | W0HE56_9ENTR | WP_032495789.1 | 12 | NG_049661.1 |
| OXA-405 | AJA30430 | Serratia marcescens | A0A0F6P2I5-1 | A0A0F6P2I5_SERMA | WP_063862762.1 | 13 | NG_049694.1 |
| OXA-416 | AKH90740 | Shewanella xiamenensis | AOAOF7NPZ6-1 | A0A0F7NPZ6_9GAMM | WP_063862782.1 | 14 | NG_049704.1 |
| OXA-438 | AKL59521 | Escherichia coli | A0A0H3WBB3-1 | A0A0H3WBB3_ECOLX | WP_063864080.1 | 15 | NG_049724.1 |

(continued)

| Gene | KO Entry | Organism | UniProt Accession number | UniProt Entry name | NCBI RefSeq Protein | SEQ ID NO: | NCBI RefSeq Genomic |
|---|---|---|---|---|---|---|---|
| OXA-439 | AKR53961 | Escherichia coli | A0A0K0TQH6-1 | A0A0KQTQH6_ECOLX | WP_063864081.1 | 16 | NG_049725.1 |
| OXA-484 | ALI16502 | Klebsiella pneumoniae | A0A0N9X3M9-1 | A0A0N9X3M9_KLEPN | WP_063864110.1 | 17 | NG_049766.1 |
| OXA-436 | ALN39132 | Enterobacter asburiae | AOAOS2C593-1 | A0A0S2C593_ENTAS | WP_058842180.1 | 18 | NG_049722.1 |
| OXA-505 | ALZ40809 | Klebsiella pneumoniae | A0A0U4UUJ9-1 | A0A0U4UUJ9_KLEPN | WP_063864118.1 | 19 | NG_049783.1 |
| OXA-514 | AMN85820 | Shewanella sp. 10A | A0A1C8FIV5-1 | A0A1C8FIV5_9GAMM | WP_094009803.1 | 20 | NG_055475.1 |
| OXA-515 | AMN85821 | Shewanella sp. W17 | A0A1C8FMF1-1 | A0A1C8FMF1_9GAMM | WP_094009804.1 | 21 | NG_055476.1 |
| OXA-517 | AM066558 | Klebsiella pneumoniae | A0A1U8YI81-1 | A0A1U8YI81_KLEPN | WP_085562384.1 | 22 | NG_054666.1 |
| OXA-519 | ANI25017 | Klebsiella pneumoniae | AOA218KGB2-1 | A0A218KGB2_KLEPN | WP_094009808.1 | 23 | NG_055490.1 |
| OXA-538 | APB92846 | Shewanella xiamenensis | A0A1J0CZ62-1 | A0A1J0CZ62_9GAMM | WP_071593227.1 | 24 | NG_052051.1 |
| OXA-547 | AQW34754 | Shewanella xiamenensis | A0A1S6QLQI-1 | A0A1S6QLQ1_9GAMM | WP_085562403.1 | 25 | NG_054693.1 |
| OXA-546 | AQX43454 | Shewanella xiamenensis | A0A1W5RS27-1 | A0A1W5RS27_9GAMM | WP_087587945.1 | 26 | NG_054959.1 |
| OXA-566 | ASC55290 | Escherichia coli | A0A1Z3GBB3-1 | A0A1Z3GBB3_ECOLX | WP_094009811.1 | 27 | NG_055499.1 |
| OXA-252 | ASF15667 | Shewanella sp. FDAARGOS_354 | A0A1Z4ACK8-1 | A0A1Z4ACK8_9GAMM | WP_037428895.1 | 28 | NG_050608.1 |

Each of the above blaOXA-48 class D beta-lactamase polypeptide sequences e.g., a blaOXA-48 class D beta-lactamase as set forth in SEQ ID NO: 1 to 28, includes an N-terminal signal peptide (as indicated in the sequence listing). During processing of blaOXA-48 class D beta-lactamase polypeptide, the signal peptide, corresponding to amino acids 1 to 22 in each of SEQ ID NO: 1 to 28, is processed away to form the mature blaOXA-48 class D beta-lactamase polypeptide, corresponding to the respective blaOXA-48 class D beta-lactamase as set forth in SEQ ID NO: 29 to 56.

In other words, the amino acid sequence of the blaOXA-48 class D beta-lactamase as set forth in SEQ ID NO: 1 to 28 without the N-terminal signal peptide is provided as set forth in SEQ ID NO: 29 to 56 respectively.

In certain embodiments of the methods or uses as taught herein, the blaOXA-48 may be selected from the group consisting of beta-lactamase class D OXA-48, beta-lactamase class D OXA-54, beta-lactamase class D OXA-162, beta-lactamase class D OXA-163, beta-lactamase class D OXA-181, beta-lactamase class D OXA-199, beta-lactamase class D OXA-204, beta-lactamase class D OXA-244, beta-lactamase class D OXA-245, beta-lactamase class D OXA-247, beta-lactamase class D OXA-232, beta-lactamase class D OXA-370, beta-lactamase class D OXA-405, beta-lactamase class D OXA-416, beta-lactamase class D OXA-438, beta-lactamase class D OXA-439, beta-lactamase class D OXA-484, beta-lactamase class D OXA-436, beta-lactamase class D OXA-505, beta-lactamase class D OXA-514, beta-lactamase class D OXA-515, beta-lactamase class D OXA-517, beta-lactamase class D OXA-519, beta-lactamase class D OXA-538, beta-lactamase class D OXA-547, beta-lactamase class D OXA-546, beta-lactamase class D OXA-566, and beta-lactamase class D OXA-252. Preferably, the blaOXA-48 is beta-lactamase class D OXA-48.

In certain embodiments of the methods or uses as taught herein, the blaOXA-48 is an OXA-48 family class D beta-lactamase comprising an amino acid sequence as set forth in SEQ ID NO: 1, 2, 3, 4, 5, 6, 7, 8, 9, 10, 11, 12, 13, 14, 15, 16, 17, 18, 19, 20, 21, 22, 23, 24, 25, 26, 27, or 28.

In certain embodiments of the methods or uses as taught herein, the blaOXA-48 is a mature OXA-48 family class D beta-lactamase (without the N-terminal signal peptide) comprising an amino acid sequence as set forth in SEQ ID NO: 29, 30, 31, 32, 33, 34, 35, 36, 37, 38, 39, 40, 41, 42, 43, 44, 45, 46, 47, 48, 49, 50, 51, 52, 53, 54, 55, or 56.

The blaOXA-48 class D beta-lactamase or functionally active variant or fragment thereof as disclosed herein may be conveniently denoted as "modified", or as "mutated" or "mutant", or as comprising one or more mutations, i.e., comprising one or more amino acid sequence changes compared to the amino acid sequence of the blaOXA-48 class D beta-lactamase that has not been so-mutated, such as, particularly, compared to the amino acid sequence of wild-type blaOXA-48 class D beta-lactamase. For instance, the blaOXA-48 class D beta-lactamase may be modified, e.g. by nucleic acid deletions, insertions, and/or substitutions, to introduce a cysteine residue, e.g. in order to be able to immobilize the blaOXA-48 class D beta-lactamase on a surface of a device or to add a biotin moiety on the blaOXA-48 class D beta-lactamase.

As used herein, the term "wild-type" as applied to a nucleic acid or polypeptide refers to a nucleic acid or a polypeptide that occurs in, or is produced by, a biological organism as that biological organism exists in nature. The term "wild-type" may to some extent be synonymous with "native", the latter encompassing nucleic acids or polypeptides having a native sequence, i.e., ones of which the primary sequence is the same as that of the nucleic acids or polypeptides found in or derived from nature. A skilled person understands that native sequences may differ between or within different individuals of the same species due to normal genetic diversity (variation) within a given species. Also, native sequences may differ between or within different individuals of the same species due to post-transcriptional or post-translational modifications. Any such variants or isoforms of nucleic acids or polypeptides are encompassed herein as being "native". Accordingly, all sequences of nucleic acids or polypeptides found in or derived from nature are considered "native". The term "native" encompasses the nucleic acids or polypeptides when forming a part of a living organism, organ, tissue or cell, when forming a part of a biological sample, as well as when at least partly isolated from such sources. The term also encompasses the nucleic acids or polypeptides when produced by recombinant or synthetic means.

In certain embodiments, the blaOXA-48 class D beta-lactamase polypeptide or functionally active variant or fragment thereof may be modified during chemical polypeptide synthesis (e.g., by chemically building in the desired amino acids), or during production of the polypeptide by recombinant molecular genetic techniques, or by cell-free translation.

In certain embodiments, the blaOXA-48 class D beta-lactamase polypeptide, e.g., a blaOXA-48 class D beta-lactamase as set forth in SEQ ID NO: 1 to 56, or functionally active variant or fragment thereof may be modified so as to replace one or more amino acids (such as two or more consecutive amino acids) in the conserved sequence NKLHVSE by cysteine (such as by one cysteine). In certain embodiments, the blaOXA-48 class D beta-lactamase polypeptide, e.g., a blaOXA-48 class D beta-lactamase as set forth in SEQ ID NO: 1 to 56, or functionally active variant or fragment thereof may be modified so as to insert cysteine between any two consecutive amino acids in the conserved sequence NKLHVSE. Preferably, the blaOXA-48 class D beta-lactamase polypeptide, e.g., a blaOXA-48 class D beta-lactamase as set forth in SEQ ID NO: 1 to 56, or functionally active variant or fragment thereof may be modified so as to replace the serine in the conserved sequence NKLHVSE by cysteine.

Certain embodiments provide a blaOXA-48 class D beta-lactamase polypeptide or functionally active variant or fragment thereof, wherein one or more amino acids (such as two or more consecutive amino acids) corresponding

to asparagine 179, lysine 180, leucine 181, histidine 182, valine 183, serine 184 or glutamic acid 185 of *Klebsiella pneumoniae* OXA-48 as set forth in SEQ ID NO: 1 are replaced by cysteine (such as by one cysteine). Certain embodiments provide a blaOXA-48 class D beta-lactamase polypeptide or functionally active variant or fragment thereof, wherein cysteine is inserted between any two consecutive amino acids corresponding to asparagine 179, lysine 180, leucine 181, histidine 182, valine 183, serine 184 or glutamic acid 185 of *Klebsiella pneumoniae* OXA-48 as set forth in SEQ ID NO: 1. Preferred embodiments provide a blaOXA-48 class D beta-lactamase polypeptide or functionally active variant or fragment thereof, wherein the amino acids corresponding to serine 184 of *Klebsiella pneumoniae* OXA-48 as set forth in SEQ ID NO: 1 is replaced by cysteine.

Certain embodiments provide a blaOXA-48 class D beta-lactamase polypeptide or functionally active variant or fragment thereof, wherein one or more amino acids (such as two or more consecutive amino acids) corresponding to asparagine 157, lysine 158, leucine 159, histidine 160, valine 161, serine 162 or glutamic acid 163 of mature *Klebsiella pneumoniae* OXA-48 as set forth in SEQ ID NO: 29 are replaced by cysteine (such as by one cysteine). Certain embodiments provide a blaOXA-48 class D beta-lactamase polypeptide or functionally active variant or fragment thereof, wherein cysteine is inserted between any two consecutive amino acids corresponding to asparagine 157, lysine 158, leucine 159, histidine 160, valine 161, serine 162 or glutamic acid 163 of mature *Klebsiella pneumoniae* OXA-48 as set forth in SEQ ID NO: 29. Preferred embodiments provide a blaOXA-48 class D beta-lactamase polypeptide or functionally active variant or fragment thereof, wherein the amino acids corresponding to serine 162 of mature *Klebsiella pneumoniae* OXA-48 as set forth in SEQ ID NO: 29 is replaced by cysteine.

In certain embodiments, blaOXA-48 class D beta-lactamase, e.g., a blaOXA-48 class D beta-lactamase as set forth in SEQ ID NO: 1 to 56, or a functionally active variant or fragment thereof may be fused with its N-terminus and/or C-terminus to a further polypeptide or may be inserted into (the amino acid sequence of) a further polypeptide. Preferably, the further polypeptide is an enzyme. For instance, blaOXA-48 class D beta-lactamase, e.g., a blaOXA-48 class D beta-lactamase as set forth in SEQ ID NO: 1 to 56, or a functionally active variant or fragment thereof may be inserted into the loop of a carrier protein such as in the loop of another enzyme (also referred to as "an internalized blaOXA-48 class D beta-lactamase").

The present disclosure also relates to "functionally active variants or fragments" of the blaOXA-48 class D beta-lactamases disclosed herein. The expression comprises functionally active variants of the blaOXA-48 class D beta-lactamases, functionally active fragments of the blaOXA-48 class D beta-lactamases, as well as functionally active variants of fragments of the blaOXA-48 class D beta-lactamases.

In certain embodiments, the method may comprise (a) providing a blaOXA-48 class D beta-lactamase or a functionally active variant thereof having at least about 90% sequence identity to the amino acid sequence of the blaOXA-48 class D beta-lactamase, e.g. the corresponding wild-type blaOXA-48 class D beta-lactamase. In certain embodiments, the method may comprise (a) providing a blaOXA-48 class D beta-lactamase or a functionally active variant thereof having at least about 91%, at least 92%, at least 93%, at least 94%, at least 95%, at least 96%, at least 97%, at least 98%, or at least 99% sequence identity to the amino acid sequence of the blaOXA-48 class D beta-lactamase, e.g. the corresponding wild-type blaOXA-48 class D beta-lactamase. In certain embodiments, the method may comprise (a) providing a blaOXA-48 class D beta-lactamase or a functionally active variant thereof having about 90% to about 99% or about 95% to about 99% sequence identity to the amino acid sequence of the blaOXA-48 class D beta-lactamase, e.g. the corresponding wild-type blaOXA-48 class D beta-lactamase.

The term "variant" of a protein, polypeptide or peptide generally refers to proteins, polypeptides or peptides the amino acid sequence of which is substantially identical (i.e., largely but not wholly identical) to the sequence of the protein, polypeptide, or peptide, e.g., at least about 80% identical or at least about 85% identical, e.g., preferably at least about 90% identical, e.g., at least 91% identical, 92% identical, more preferably at least about 93% identical, e.g., at least 94% identical, even more preferably at least about 95% identical, e.g., at least 96% identical, yet more preferably at least about 97% identical, e.g., at least 98% identical, and most preferably at least 99% identical to the sequence of the protein, polypeptide, or peptide, e.g., to the sequence of a corresponding blaOXA-48 class D beta-lactamase, e.g., a blaOXA-48 class D beta-lactamase as set forth in SEQ ID NO: 1 to 56. Preferably, a variant may display such degrees of identity to a recited protein, polypeptide or peptide when the whole sequence of the recited protein, polypeptide or peptide is queried in the sequence alignment (i.e., overall sequence identity). Sequence identity may be determined using suitable algorithms for performing sequence alignments and determination of sequence identity as know *per se.* Exemplary but non-limiting algorithms include those based on the Basic Local Alignment Search Tool (BLAST) originally described by Altschul et al. 1990 (J Mol Biol 215: 403-10), such as the "Blast 2 sequences" algorithm described by Tatusova and Madden 1999 (FEMS Microbiol Lett 174: 247-250), for example using the published default settings or other suitable settings (such as, e.g., for the BLASTN algorithm: cost to open a gap = 5, cost to extend a gap = 2, penalty for a mismatch = -2, reward for a match = 1, gap x_dropoff = 50, expectation value = 10.0, word size = 28; or for the BLASTP algorithm: matrix = Blosum62 (Henikoff et al., 1992, Proc. Natl. Acad. Sci., 89:10915-10919), cost to open a gap = 11, cost to extend a gap = 1, expectation value = 10.0, word size = 3).

An exemplary procedure to determine the percent identity between a particular amino acid sequence and the

amino acid sequence of a query polypeptide (e.g., a blaOXA-48 class D beta-lactamase, e.g., a blaOXA-48 class D beta-lactamase as set forth in SEQ ID NO: 1 to 56) will entail aligning the two amino acid sequences using the Blast 2 sequences (Bl2seq) algorithm, available as a web application or as a standalone executable programme (BLAST version 2.2.31+) at the NCBI web site (www.ncbi.nlm.nih.gov), using suitable algorithm parameters. An example of suitable algorithm parameters include: matrix = Blosum62, cost to open a gap = 11, cost to extend a gap = 1, expectation value = 10.0, word size = 3). If the two compared sequences share homology, then the output will present those regions of homology as aligned sequences. If the two compared sequences do not share homology, then the output will not present aligned sequences. Once aligned, the number of matches will be determined by counting the number of positions where an identical amino acid residue is presented in both sequences. The percent identity is determined by dividing the number of matches by the length of the query polypeptide, followed by multiplying the resulting value by 100. The percent identity value may, but need not, be rounded to the nearest tenth. For example, 78.11, 78.12, 78.13, and 78.14 may be rounded down to 78.1, while 78.15, 78.16, 78.17, 78.18, and 78.19 may be rounded up to 78.2. It is further noted that the detailed view for each segment of alignment as outputted by Bl2seq already conveniently includes the percentage of identities.

A variant of a protein, polypeptide, or peptide may be a homologue (e.g., orthologue or paralogue) of said protein, polypeptide, or peptide. As used herein, the term "homology" generally denotes structural similarity between two macromolecules, particularly between two proteins or polypeptides, from same or different taxons, wherein said similarity is due to shared ancestry.

A variant of a protein, polypeptide, or peptide may comprise one or more amino acid additions, deletions, or substitutions relative to (i.e., compared with) the corresponding protein or polypeptide, e.g., a corresponding blaOXA-48 class D beta-lactamase, e.g., a blaOXA-48 class D beta-lactamase as set forth in SEQ ID NO: 1 to 56.

For example, a variant (substitution variant) of a protein, polypeptide, or peptide may comprise up to 50 (e.g., not more than one, two, three, four, five, six, seven, eight, nine, ten, 12, 15, 20, 25, 30, 35, 40, or 50) conservative amino acid substitutions relative to (i.e., compared with) the corresponding protein or polypeptide, e.g., a corresponding blaOXA-48 class D beta-lactamase, e.g., a blaOXA-48 class D beta-lactamase as set forth in SEQ ID NO: 1 to 56.

A conservative amino acid substitution is a substitution of one amino acid for another with similar characteristics. Conservative amino acid substitutions include substitutions within the following groups: valine, alanine and glycine; leucine, valine, and isoleucine; aspartic acid and glutamic acid; asparagine and glutamine; serine, cysteine, and threonine; lysine and arginine; and phenylalanine and tyrosine. The nonpolar hydrophobic amino acids include alanine, leucine, isoleucine, valine, proline, phenylalanine, tryptophan and methionine. The polar neutral amino acids include glycine, serine, threonine, cysteine, tyrosine, asparagine and glutamine. The positively charged (i.e., basic) amino acids include arginine, lysine and histidine. The negatively charged (i.e., acidic) amino acids include aspartic acid and glutamic acid. Any substitution of one member of the above-mentioned polar, basic, or acidic groups by another member of the same group can be deemed a conservative substitution. By contrast, a non-conservative substitution is a substitution of one amino acid for another with dissimilar characteristics.

Alternatively or in addition, for example, a variant (deletion variant) of a protein, polypeptide, or peptide may lack up to 20 amino acid segments (e.g., one, two, three, four, five, six, seven, eight, nine, ten, 11, 12, 13, 14, 15, 16, 17, 18, 19, or 20 segments) relative to (i.e., compared with) the corresponding protein or polypeptide, e.g., a corresponding blaOXA-48 class D beta-lactamase, e.g., a blaOXA-48 class D beta-lactamase as set forth in SEQ ID NO: 1 to 56. The deletion segment(s) may each independently consist of one amino acid, two contiguous amino acids or three contiguous amino acids. The deletion segments may be non-contiguous, or two or more or all of the deletion segments may be contiguous.

In certain embodiments, the method may comprise (a) providing a blaOXA-48 class D beta-lactamase or a functionally active fragment thereof representing at least about 90% of the amino acid sequence of the blaOXA-48 class D beta-lactamase, e.g. the corresponding wild-type blaOXA-48 class D beta-lactamase. In certain embodiments, the method may comprise (a) providing a blaOXA-48 class D beta-lactamase or a functionally active fragment thereof representing at least about 91%, at least 92%, at least 93%, at least 94%, at least 95%, at least 96%, at least 97%, at least 98%, or at least 99% of the amino acid sequence of the blaOXA-48 class D beta-lactamase, e.g. the corresponding wild-type blaOXA-48 class D beta-lactamase. In certain embodiments, the method may comprise (a) providing a blaOXA-48 class D beta-lactamase or a functionally active fragment thereof representing about 90% to about 99% or about 95% to about 99% of the amino acid sequence of the blaOXA-48 class D beta-lactamase, e.g. the corresponding wild-type blaOXA-48 class D beta-lactamase.

The term "fragment" of a protein, polypeptide, or peptide generally refers to N-terminally and/or C-terminally deleted or truncated forms of said protein, polypeptide or peptide. The term encompasses fragments arising by any mechanism, such as, without limitation, by alternative translation, exo- and/or endo-proteolysis and/or degradation of said peptide, polypeptide or protein, such as, for example, *in vivo* or *in vitro,* such as, for example, by physical, chemical and/or enzymatic proteolysis. Without limitation, a fragment of a protein, polypeptide, or peptide may represent at least about 5% (by amino acid number), or at least about 10%, e.g., 20% or more, 30% or more, or 40% or more, such as preferably 50% or more, e.g., 60% or more, 70% or more, 80% or more, 90% or more, 91% or more, 92% or more, 93%

or more, 94% or more, 95% or more, 96% or more, 97% or more, 98% or more, or 99% or more of the amino acid sequence of said protein, polypeptide, or peptide, e.g., a corresponding blaOXA-48 class D beta-lactamase as defined herein, e.g., a blaOXA-48 class D beta-lactamase as set forth in SEQ ID NO: 1 to 56. The reference herein to "SEQ ID NO: 1 to 56" denotes SEQ ID NO: 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, or 56.

For example, a fragment of a protein, polypeptide, or peptide may include a sequence of 5 or more consecutive amino acids, 10 or more consecutive amino acids, 20 or more consecutive amino acids, 30 or more consecutive amino acids, e.g., 40 or more consecutive amino acids, such as for example 50 or more consecutive amino acids, 60 or more, 70 or more, 80 or more, 90 or more, 100 or more, 150 or more, 200 or more, 210 or more, 220 or more, 230 or more, 240 or more, 250 or more, or 260 or more consecutive amino acids of the corresponding full-length protein or polypeptide, e.g., a corresponding blaOXA-48 class D beta-lactamase, e.g., a blaOXA-48 class D beta-lactamase as set forth in SEQ ID NO: 1 to 56.

In an embodiment, a fragment of a protein, polypeptide, or peptide may be N-terminally and/or C-terminally truncated by between 1 and about 20 amino acids, such as by between 1 and about 15 amino acids, or by between 1 and about 10 amino acids, or by between 1 and about 5 amino acids, compared with the corresponding full-length protein or polypeptide, e.g., a corresponding blaOXA-48 class D beta-lactamase, e.g., a blaOXA-48 class D beta-lactamase as set forth in SEQ ID NO: 1 to 56.

In certain embodiments, the method may comprise (a) providing a blaOXA-48 class D beta-lactamase or a functionally active variant thereof having at least about 90% sequence identity to the amino acid sequence of the blaOXA-48 class D beta-lactamase, e.g. the corresponding wild-type blaOXA-48 class D beta-lactamase, or a functionally active fragment thereof representing at least about 90% of the amino acid sequence of the blaOXA-48 class D beta-lactamase, e.g. the corresponding wild-type blaOXA-48 class D beta-lactamase. In certain embodiments, the method may comprise (a) providing a blaOXA-48 class D beta-lactamase or a functionally active variant thereof having about 90% to about 99% sequence identity to the amino acid sequence of the blaOXA-48 class D beta-lactamase, e.g. the corresponding wild-type blaOXA-48 class D beta-lactamase, or a functionally active fragment thereof representing about 90% to about 99% or about 95% to about 99% of the amino acid sequence of the blaOXA-48 class D beta-lactamase, e.g. the corresponding wild-type blaOXA-48 class D beta-lactamase.

As disclosed herein, blaOXA-48 class D beta-lactamase, e.g., a blaOXA-48 class D beta-lactamase as set forth in SEQ ID NO: 1 to 56, may also be fused with one or more internal and/or terminal (i.e., N- and/or C-terminal) irrelevant or heterologous amino acid sequences (i.e., fusion protein). A heterologous sequence can be, for example a sequence used for purification of the recombinant protein (e.g., FLAG, polyhistidine (e.g., hexahistidine), hemagluttanin (HA), glutathione-S-transferase (GST), or maltose-binding protein (MBP)). Heterologous sequences can also be proteins useful as diagnostic or detectable markers, for example, luciferase, green fluorescent protein (GFP), or chloramphenicol acetyl transferase (CAT). In some embodiments, the fusion protein may contain a signal sequence from another protein.

Where the present specification refers to or encompasses variants and/or fragments of proteins, polypeptides or peptides, this denotes variants or fragments which are functionally active or functional, i.e., which at least partly retain the biological activity or intended functionality of the respective or corresponding proteins, polypeptides, or peptides. By means of an example and not limitation, a functionally active variant or fragment of blaOXA-48 class D beta-lactamase as disclosed herein shall at least partly retain the biological activity of blaOXA-48 class D beta-lactamase. For example, it may retain one or more aspects of the biological activity of blaOXA-48 class D beta-lactamase, such as its beta-lactam hydrolysing activity. Preferably, a functionally active variant or fragment may retain at least about 20%, e.g., at least about 25%, or at least 30%, or at least about 40%, or at least about 50%, e.g., at least 60%, more preferably at least about 70%, e.g., at least 80%, yet more preferably at least about 85%, still more preferably at least about 90%, and most preferably at least about 95% or even about 100% or higher of the intended biological activity or functionality compared with the corresponding protein, polypeptide, or peptide. Reference to the "activity" of a protein, polypeptide, or peptide such as blaOXA-48 class D beta-lactamase may generally encompass any one or more aspects of the biological activity of the protein, polypeptide, or peptide, such as without limitation any one or more aspects of its biochemical activity, enzymatic activity, signalling activity, interaction activity, ligand activity, and/or structural activity, e.g., within a cell, tissue, organ or an organism. By means of an example and not limitation, reference to the activity of blaOXA-48 class D beta-lactamase or functionally active variant or fragment thereof may particularly denote its activity as a beta-lactamase, i.e., its ability to hydrolyse a beta-lactam ring. Where the activity of a given protein, polypeptide, or peptide such as blaOXA-48 class D beta-lactamase can be readily measured in an established assay, e.g., an enzymatic assay (such as, for example, by a colorimetric assay), a functionally active variant or fragment of the protein, polypeptide, or peptide may display activity in such assays, which is at least about 20%, e.g., at least about 25%, or at least 30%, or at least about 40%, or at least about 50%, e.g., at least 60%, more preferably at least about 70%, e.g., at least 80%, yet more preferably at least about 85%, still more preferably at least about 90%, and most preferably at least about 95% or even about 100% or higher of the activity of the respective or corresponding protein, polypeptide, or peptide.

For example, the blaOXA-48 class D beta-lactamase activity of blaOXA-48 class D beta-lactamase or functionally active variant or fragment thereof can be measured in an enzymatic assay, such as by a colorimetric assay with Nitrocefin (SKU: N005) (TOKU-E, Washington, USA) as a substrate. The activity can be measured, for instance with a spectrophotometer, e.g., a computer controlled AMS-Ellipse analyser (AMS SpA, Rome, Italy), e.g. at 482 nm after excitation at 390 nm.

In certain examples, a functionally active variant or fragment of blaOXA-48 class D beta-lactamase may have at least 25% (e.g., at least 30%, at least 40%, at least 50%, at least 60%, at least 70%, at least 75%, at least 80%, at least 85%, at least 90%, at least 95%, at least 98%, at least 99%, at least 100%, or even greater than 100%) of the blaOXA-48 class D beta-lactamase enzymatic activity of the blaOXA-48 class D beta-lactamase polypeptide as set forth in SEQ ID NO: 1 to 56. The functional variant or fragment can generally, but not always, be comprised of a continuous region of the protein, wherein the region has functional activity. The amino acid sequence of the active site of OXA-48 polypeptide has been described in the literature (Docquier et al., 2009, Chem. Biol., 16(5), 540-7). Candidate functional variants or fragments of blaOXA-48 class D beta-lactamase polypeptides can therefore be produced by one skilled in the art using well established methods, such as homology modelling and computational engineering, and tested for the desired enzymatic activity.

Further, unless otherwise apparent from the context, reference herein to any nucleic acid, peptide, polypeptide or protein and variants or fragments thereof may generally also encompass altered forms of said nucleic acid, peptide, polypeptide or protein and variants or fragments such as bearing post-expression modifications including, for example, phosphorylation, glycosylation, lipidation, methylation, cysteinylation, sulphonation, glutathionylation, acetylation, oxidation of methionine to methionine sulphoxide or methionine sulphone, and the like.

Conveniently, certain amino acid(s) of the blaOXA-48 class D beta-lactamase or functionally active variant or fragment thereof as disclosed herein may be referred to herein as "corresponding to" certain amino acid(s) of a reference blaOXA-48 class D beta-lactamase, usually the blaOXA-48 class D beta-lactamase polypeptide as set forth in SEQ ID NO: 1 to 56.

The skilled person will have an immediate understanding of the correspondence between amino acid(s) of two forms of blaOXA-48 class D beta-lactamase polypeptide. By means of example, such corresponding amino acids may be located at the same position in an alignment of the primary amino acid sequences of the two forms of blaOXA-48 class D beta-lactamase polypeptide. The sequence alignment may be generated as explained elsewhere in the specification, in connection with the determination of the extent of sequence identity. Such corresponding amino acids may also co-locate in the secondary and/or tertiary structures of the two forms of blaOXA-48 class D beta-lactamase polypeptide.

In certain embodiments, the method as taught herein comprises the step (b) of providing a biological sample.

In certain embodiments, the method as taught herein comprises the step (b) of providing a biological sample obtained from a subject. In other words, in certain embodiments, the biological sample is obtained from the subject prior to step (b).

In certain embodiments, the method as taught herein comprises the step (c) of contacting the blaOXA-48 or functionally active variant or fragment thereof with the biological sample.

The biological samples, e.g. serum, blood, urine, interstitial fluid, saliva, tears, exudates, fluid collected from deep tissues, fluid collected from subcutaneous tissues or other mammal fluids, preferably human fluids, preferably serum, blood, urine, interstitial fluids, more preferably serum susceptible of containing a carbapenem antibiotic, may be analysed at regular intervals. Thereby, the present method advantageously allows to monitor the concentration of the carbapenem antibiotic in the biological sample.

In an embodiment, the method comprises prior to step (c), the step of treating the biological sample with an aqueous solution. According to this embodiment, the biological sample may be treated with an aqueous solution, prior to contacting the blaOXA-48 or functionally active variant or fragment thereof with the biological sample.

The aqueous solution may be a buffer such as a phosphate buffer. The aqueous solution may comprise at least one salt and/or at least one organic compound such as a carbohydrate, a protein, a peptide, an amino acid, an organic acid (such as lactate), a polyol (such as mannitol or glycerol), a colloid (such as hydroxyethyl starch) or an infusion molecule, or any other compound capable of creating an osmotic pressure similar to that of the biological sample, such as serum, preferably at concentrations that preserve the chemical and physical intactness of the carbapenem antibiotic to be measured.

By "phosphate" it is meant compounds from the group $M^1H_2PO_4$, $M^1{}_2HPO_4$, $M^1{}_3PO_4$, $M^2(H_2PO_4)_2$, $M^2{}_3(PO_4)_2$ and $M^2HPO_4$, wherein $M^1$ is Na, Li, or K, and $M^2$ is Ca or Mg Representatives of this group are $NaH_2PO_4$, $KH_2PO_4$, $Na_2HPO_4$, $K_2HPO_4$, $Mg(H_2PO_4)_2$.

By "salt" it is meant compounds of formula $M^3X^2$ or $M^4X^2{}_2$, wherein $X^2$ is selected from F, Cl, Br, I and $M^3$ is selected from Li, Na, K, $NH_4$ and alkyl ammonium; $M^4$ is selected from Ca, Mg, Zn, Cu, Fe, Ni, Co.

The term "carbohydrate" as used herein includes monosaccharides, oligosaccharides and polysaccharides as well as substances derived from monosaccharides by reduction of the carbonyl group such as alditols, by oxidation of one or more terminal groups to carboxylic acids, or by replacement of one or more hydroxy group(s) by a hydrogen

atom, an amino group, a thiol group or similar heteroatomic groups. It also includes derivatives of these compounds.

In an embodiment, the aqueous solution is preferably phosphate buffered saline (PBS).

The treatment of the biological sample with an aqueous solution prior to step (c) is preferably a dialysis step. Dialysis, preferably micro-dialysis, allows to isolate the unbound fraction of a carbapenem antibiotic for measurement. This is advantageous over the available commercial methods that do not separate free from bound fraction without an additional step such as centrifugation through membranes. Advantageously, the present method allows that the biologically active carbapenem antibiotic can be determined directly and without any additional step.

In certain embodiments, the method as taught herein comprises the step (d) of determining the concentration of the carbapenem antibiotic in the biological sample.

In certain embodiments of the methods or uses as taught herein, the measuring step (d) may be performed by Ultraviolet-visible (UV-VIS) spectroscopy, fluorescence spectroscopy, fluorescence resonance energy transfer (FRET) spectroscopy, Fourier Transform Infrared (FTIR) spectroscopy, plasmon resonance, electrochemical detection (ECD), or by surface sensitive wave based technique, such as quartz crystal microbalance (QCM).

The ECD method allows to detect an electrical signal emitted by a proton, for example a proton released after hydrolysis of the reporter substrate. Also, it is possible to use reporter substrates that after hydrolysis release particular compounds, such as an -SH group, that can be oxidized onto an electrode.

In certain embodiments of the methods or uses as taught herein, the method may comprise, prior to step (c), contacting the blaOXA-48 or functionally active variant or fragment thereof with a reporter substrate.

Hence, in certain embodiments, the method as taught herein may comprise the steps of:

(a) providing a blaOXA-48 or a functionally active variant or fragment thereof;

(b) providing a biological sample;

(b') contacting the blaOXA-48 or functionally active variant or fragment thereof with a reporter substrate;

(c) contacting the blaOXA-48 or functionally active variant or fragment thereof with the biological sample; and

(d) determining the concentration of the carbapenem antibiotic in the biological sample.

Using a reporter substrate, the present method advantageously allows the online and real-time measurement of a carbapenem antibiotic in a biological sample. Furthermore, the present method using a reporter substrate allows automated measurement and/or monitoring of a carbapenem antibiotic in biological fluids. The reporter substrate may advantageously allow to measure an optical signal, a spectrophotometric signal or an electrical signal.

In certain embodiments of the methods or uses as taught herein, the method may comprise the steps of contacting the biological sample with a reporter substrate and the blaOXA-48 or functionally active variant or fragment thereof, and measuring the amount of a spectrophotometric signal, an optical signal and/or an electrical signal in the biological sample in comparison with a standard, thereby determining the concentration of the carbapenem antibiotic in the biological sample.

In certain embodiments, the reporter substrate may comprise a beta-lactam ring.

In certain embodiments of the methods or uses as taught herein, the reporter substrate may be a chromogenic substrate.

For instance, the reporter substrate may be a chromogenic substrate, such as the compound CENTA, as shown in Formula (IIIA) or nitrocefin, as shown in Formula (IIIB).

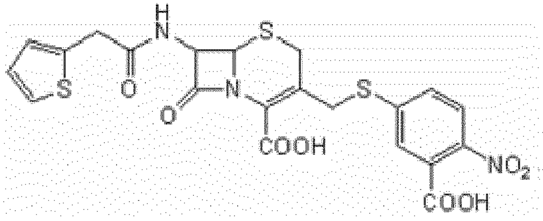

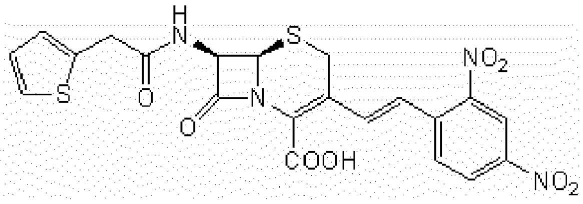

(IIIA) (IIIB)

In certain embodiments, the reporter substrate may be a fluorescent substrate. Non-limiting examples of suitable fluorescent substrates include commercially available fluorescent substrates, such as CCF2 fluorescent substrate (Invitrogen, San Diego, California) or Fluorocillin TM Green beta-lactamase substrate (Molecular Probes, Invitrogen, San Diego, California).

In certain embodiments of the methods or uses as taught herein, the method may comprise the steps of contacting the biological sample with a chromogenic substrate and the blaOXA-48 or functionally active variant or fragment thereof, and measuring the amount of colour developed in the biological sample in comparison with a standard, thereby determining the concentration of the carbapenem antibiotic in the biological sample. In certain embodiments, the method may comprise the steps of contacting the biological sample with a fluorescent substrate and the blaOXA-48 or functionally active variant or fragment thereof, and measuring the amount of fluorescence developed in the biological sample in comparison with a standard, thereby determining the concentration of the carbapenem antibiotic in the biological sample. The standard may represent a known amount, quantity or concentration of a carbapenem antibiotic.

Without being bound to a theory, this embodiment of the present method is based on a competition between a reporter substrate, acting as ’a substrate’ and a carbapenem antibiotic in the biological sample, acting as ’an inhibitor substrate’ towards the catalytic site of the blaOXA-48 class D beta-lactamase. In absence of carbapenem antibiotic, the biosensor will hydrolyse the reporter substrate. This will lead to a reaction product that may be measured by a spectroscopic or colorimetric method. In presence of carbapenem antibiotic, also referred to as analyte, the biosensor is less available to hydrolyse the reporter substrate. Accordingly, the higher the concentration of carbapenem antibiotic, the lower is the response measured by the spectroscopic or colorimetric method.

In certain embodiments, the method as taught herein may be considered as a colorimetric assay.

In certain embodiments, the blaOXA-48 or functionally active variant or fragment thereof may be immobilized. The blaOXA-48 or functionally active variant or fragment thereof may be immobilized by impregnation on an inert material with sufficient porosity and wettability to allow movement of a liquid biological sample and to allow the blaOXA-48 or functionally active variant or fragment thereof to rehydrate easily and completely when the liquid biological sample reaches the enzyme. The inert material may be glass fibres or polyester or any other physically and/or chemically inert material. The reporter substrate (e.g., a chromogenic substrate) may also be immobilized. When the liquid biological sample is in contact with at least one rehydrated blaOXA-48 or functionally active variant or fragment thereof and a reporter substrate (e.g., a chromogenic substrate), the signal (e.g., colour development) will depend on the concentration of the carbapenem antibiotic in the biological sample. In certain embodiments, the blaOXA-48 or functionally active variant or fragment thereof may be immobilized on a test strip.

In certain embodiments of the methods or uses as taught herein, the blaOXA-48 or functionally active variant or fragment thereof may be grafted on at least one surface of a device. In certain embodiments of the methods or uses as taught herein, the blaOXA-48 or functionally active variant or fragment thereof may be grafted on at least one surface of a device, wherein said at least one surface is chemically activated. In certain embodiments, the blaOXA-48 or functionally active variant or fragment thereof may be grafted on at least one surface of a device, wherein said at least one surface is a metal plated surface.

Accordingly, in certain embodiments, the method as taught herein may comprise the steps of:

(a) providing a blaOXA-48 or a functionally active variant or fragment thereof, wherein the blaOXA-48 or functionally active variant or fragment thereof is grafted on at least one surface of a device, preferably wherein said at least one surface is chemically activated or is a metal plated surface;

(b) providing a biological sample;

(c) contacting the blaOXA-48 or functionally active variant or fragment thereof with the biological sample; and

(d) determining the concentration of the carbapenem antibiotic in the biological sample.

Hence, the method as taught herein may use a device on which the blaOXA-48 or functionally active variant or fragment thereof is grafted. The terms "grafted", "coupled" or "bound" may be used herein interchangeably and refer to the covalent incorporation of the blaOXA-48 or functionally active variant or fragment thereof on a device.

The device may also be referred herein as "biosensor-based device". By the term "biosensor-based device" it is meant a device based on the specific interaction of an analyte of interest and a target, such as a biological component, for example a receptor, an antibody, an enzyme, a membrane, a cell or cell containing media, a molecule, and the subsequent transformation of this interaction into an electrical, optical, or other signal.

Using a device on which the blaOXA-48 or functionally active variant or fragment thereof is grafted, the present method is particularly useful for online and real-time measurement and/or monitoring of a carbapenem antibiotic in a biological sample. Using a device on which the blaOXA-48 or functionally active variant or fragment thereof is grafted, the present method is particularly useful for automated measurement and/or monitoring of a carbapenem antibiotic in biological fluids, such as serum. Using a device on which the blaOXA-48 or functionally active variant or fragment thereof

is grafted, the present method allows self-regeneration of the device or provides the device with a self-regenerative capacity, i.e. the capacity to be re-used without the need to regenerate the device. The self-regenerative capacity of the present method allows monitoring and adjustment of the concentration of a carbapenem antibiotic in a biological sample, such as serum, in an efficient, and hence economical way.

In certain embodiments of the methods or uses as taught herein, the device may comprise an attenuated total internal reflection element, transparent in the infrared, or the device may be a quartz crystal microbalance.

In certain embodiments, the device may comprise an attenuated total internal reflection element, transparent in the infrared. In this embodiment, the carbapenem antibiotic may be measured with surface sensitive optical methods. In this embodiment, the concentration of the carbapenem antibiotic may be measured by Fourier Transform Infrared (FTIR)-ATR spectroscopy. FTIR-ATR spectroscopy is a rapid, selective and efficient label-free analytical method. In certain embodiments, the device may be based on FTIR-ATR technology. Fourier transform Infrared (FTIR) spectroscopy is an extremely powerful analytical technique, particularly well adapted to the characterization of organic molecules and biological systems. Quantitative structural and conformational information can be recorded.

According to an embodiment of the invention, the device may comprises an attenuated total internal reflection (ATR) element, transparent in the infrared of which at least one surface is chemically activated and covalently grafted with the blaOXA-48 or functionally active variant or fragment thereof. The ATR configuration allows the study of analytes such as biological components and molecules or proteins, on surfaces in contact with a sample.

Hence, in certain embodiments, the method as taught herein may comprise the steps of: (a) providing a blaOXA-48 or a functionally active variant or fragment thereof, wherein the blaOXA-48 or functionally active variant or fragment thereof is grafted on at least one chemically activated surface of an ATR element, transparent in the infrared; (b) providing a biological sample; (c) contacting the blaOXA-48 or functionally active variant or fragment thereof with the biological sample; and (d) determining the concentration of the carbapenem antibiotic in the biological sample.

In certain further embodiments, the method as taught herein may comprise the steps of: (a) providing a blaOXA-48 or a functionally active variant or fragment thereof, wherein the blaOXA-48 or functionally active variant or fragment thereof is grafted on at least metal plated surface of an ATR element, transparent in the infrared; (b) providing a biological sample; (c) contacting the blaOXA-48 or functionally active variant or fragment thereof with the biological sample; and (d) determining the concentration of the carbapenem antibiotic in the biological sample.

A purposely modified ATR element can be provided to study carbapenem antibiotic/blaOXA-48 class D beta-lactamase interactions occurring at the solvent ATR element interface, particularly, at the water-containing media-ATR element interface, by using attenuated total internal reflection (ATR) infrared (IR) spectroscopy, preferably Fourier transform infrared spectroscopy (FTIR). Advantageously, using an ATR element of which at least one surface is chemically activated and covalently grafted with the blaOXA-48 or functionally active variant or fragment thereof, the present method allows automated and online monitoring and adjustment of a carbapenem antibiotic in a biological sample.

In one embodiment, the ATR element may be made of a material selected from the group consisting of germanium, silicon, ZnS, ZnSe, and diamond. Preferably, the ATR element is made of germanium or silicon and more preferably the ATR element is made of germanium.

In another embodiment, the ATR element may have any shape as long as it allows internal reflection of a radiation within said ATR element. Preferably, the ATR element is a crystal having a trapezoidal, hemicylindrical, rectangular or triangular polyhedral form, rectangular prism, or a triangular prism (prism with triangular basis). More preferably, the ATR element is a triangular prism with a right triangular basis (also called "right-angled triangle" or "rectangled triangle"). More preferably, the ATR element is a triangular prism with 45-45-90 triangle basis (right triangle with the two other angles at 45°).

In certain embodiments, the device may be a Surface Plasmon Resonance sensor, such as the BIACORE/Surface Plasmon Resonance sensor.

In certain embodiments, the device may be a quartz crystal microbalance (QCM). In this embodiment, the carbapenem antibiotic may be measured with surface sensitive waveguide techniques.

The recitation "quartz crystal microbalance" (QCM) refers to a mass-sensing device. An electrical signal is sent through a quartz crystal, producing a vibration at a resonance frequency. Changes in frequency are related to changes in mass on the surface of the crystal.

In an embodiment, the QCM may function as a biosensor-based device. In an embodiment the blaOXA-48 or functionally active variant or fragment thereof may be associated with the QCM surface. Subsequent binding of the carbapenem antibiotic results in a measurable change in the resonance frequency. In an embodiment, the blaOXA-48 or functionally active variant or fragment thereof associated with the QCM allows determining the concentration of the carbapenem antibiotic and provides self-regenerative capacity to the QCM.

Hence, in certain embodiments, the method as taught herein may comprise the steps of: (a) providing a blaOXA-48 or a functionally active variant or fragment thereof, wherein the blaOXA-48 or functionally active variant or fragment thereof is grafted on at least one chemically activated surface or metal plated surface of a quartz crystal microbalance; (b) providing a biological sample; (c) contacting the blaOXA-48 or functionally active variant or fragment thereof with the

biological sample; and (d) determining the concentration of the carbapenem antibiotic in the biological sample.

In an embodiment, the surface of the ATR element or the QCM may be coated or plated with metal films such as gold films. Preferably, such metal films may have a thickness of 3 nm to 15 nm, such as 5 nm to 10 nm. Metals other than gold are e.g. Ag, Cu, Pt, Au/Pt, alloys, particularly gold comprising alloys, multilayers, particularly bilayers of metals such as gold on chromium or titanium. Such plated device can be produced by coating an ATR element or a QCM with a thin metal layer on at least one face. Such coating is performed by means of known methods for the preparation of thin metallic films, e.g. physical vapour deposition (PVD).

According to an embodiment, the device comprises an ATR element or a gold-coated quartz crystal, of which at least one surface is chemically activated and covalently grafted with the blaOXA-48 or functionally active variant or fragment thereof.

The surface can be activated by wet chemistry using oxidation / hydroxylation / reduction in an acid or alkaline environment. The activation results from the surface oxidation or hydroxylation by any available technique (physical or chemical), preferably by the wet-chemistry technique using a solution of an oxidant in acidic or basic media, such as $H_2O_2/H_2SO_4$, $H_2O_2$/TFA, $H_2O_2$/HF, $K_2Cr_2O_7/H_2SO_4$, oxone/$H_2SO_4$, $H_2O_2/NH_4OH$, or in organic media, such as an organic peracid, $Br_2$ in solution. The activation may also be carried out by dipping the crystals in sequences of solutions of an oxidant in acidic or basic media. Suitable solutions of an oxidant in acidic or basic media, are e.g. $H_2O_2/H_2SO_4$, $H_2O_2$/TFA, $H_2O_2$/HF, $K_2Cr_2O_7/H_2SO_4$, oxone/$H_2SO_4$, $H_2O_2/NH_4OH$, or in organic media, such as an organic peracid, $Br_2$ in a suitable solution, or a combination of these solutions in specific sequences, such as HF in water followed by $H_2O_2$ in water, which can be iterated for several times e.g. number of repetitions: 2, 3, 4, 5... or $NH_4OH/H_2O_2$ in water followed by HCl/$H_2O_2$ in water e.g. number of repetitions: 1. The temperature can be comprised between -15°C and +150°C. The duration of the treatment can be comprised between a few seconds to several hours.

In an embodiment, the blaOXA-48 or functionally active variant or fragment thereof can be grafted on at least one chemically activated or metal plated surface of the ATR element or on the gold-plated quartz crystal, via an organic molecule of Formula (III), (IV), (V), (VI), (VII), (VIII), (IX), (X), (XI) or (XII), as in the table here below,

| Formula | Organic molecule |
|---|---|
| III | $HS-(CH_2-CH_2-O)_w-CH_3$ |
| IV | $X^1{}_3Si-(CH_2)_q-NH-COO-(CH_2)_s-X^1$ |
| V | $CH{\equiv}CR^6$ |
| VI | $CH_2{=}CHR^6$ |
| VII | $X^1{}_3Si-(CH_2)_q-(CF_2)_s-Y^1$ |
| VIII | $X^1{}_2(R^3)Si-(CH_2)_q-(CF_2)_s-Y^1$ |
| IX | $X^1(R^3)(R^4)Si-(CH_2)_q-(CF_2)_s-Y^1$ |
| X | $X^1{}_3Si-(L^1)_n-NH-COO-(L^2-O)_m-L^3-X^1$ |
| XI | $X^1{}_2(R^3)Si-(L^1)_n-NH-COO-(L^2-O)_m-L^3-X^1$ |
| XII | $X_1(R^3)(R^4)Si-(L^1)_n-NH-COO-(L^2-O)_m-L^3-X^1$ |

wherein

$X^1$ is halogen or $C_{1-6}$alkoxy; $Y^1$ is Me, $CF_3$, $CHF_2$, $CH_2F$, $CH{=}CH_2$, CN, CH=O, epoxy, halogen, SH, $NH_2$ or *N*-maleimide or *N*-succinimide derivative thereof, OH, N=C=O, N=C=S, $CO_2H$ or *N*-hydroxysuccinimide ester thereof; $R^3$ and $R^4$ are each independently $C_{1-6}$alkyl; $R^6$ is selected from $C_wH_{2w+1}$, $C_wF_{2w+1}$, or $-(CH_2)_u-(O-CH_2-CH_2)_p-OR^5$; wherein $R^5$ is selected from $C_{1-4}$alkyl, $C_{1-6}$alkylarylsulfoxide, heteroaryloxycarbonyl$C_{1-6}$alkyl,

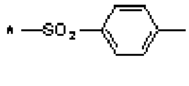,

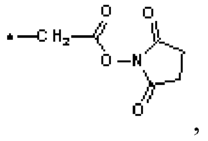,

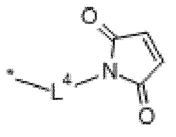,
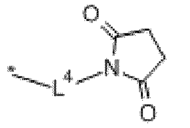
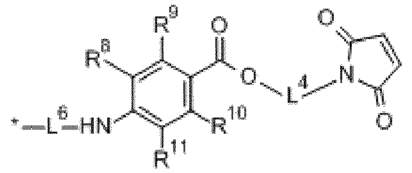,

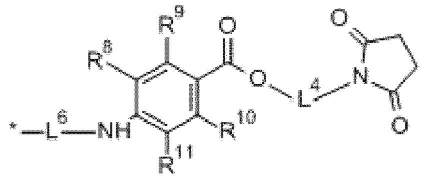,

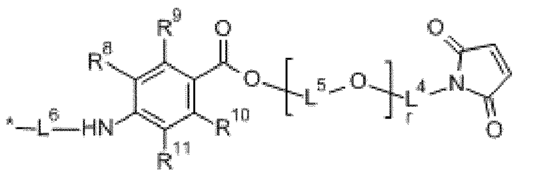,
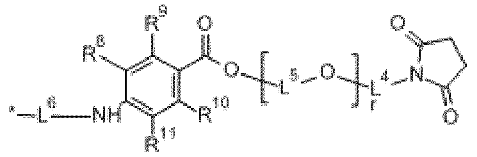,

or mixture thereof; $L^1$ is $C_{1-6}$alkylene, optionally substituted by halogen; $L^2$ is $C_{1-6}$alkylene; $L^3$ is $C_{1-6}$alkylene, optionally substituted with $R^7$, wherein $R^7$ is

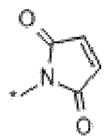,
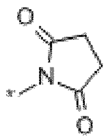,

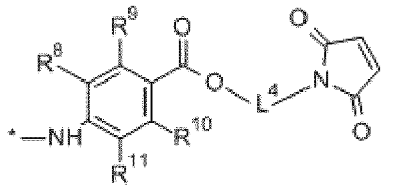,
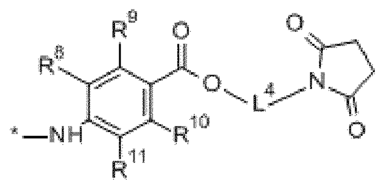,

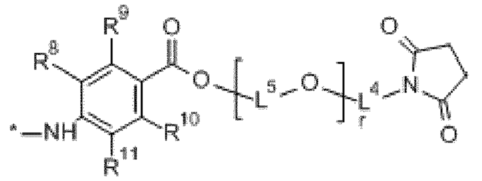, or
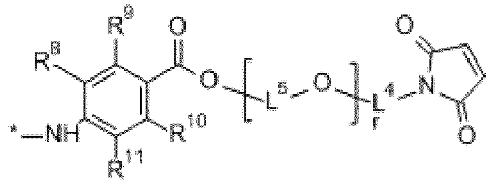;

$L^4$ is $C_{1-20}$alkylene; $L^5$ is $C_{1-6}$alkylene; $L^6$ is $C_{1-6}$alkylene, optionally substituted with $C_{1-6}$alkoxy; each of $R^8$, $R^9$, $R^{10}$ and $R^{11}$ are independently selected from hydrogen, $C_{1-6}$alkyl, $C_{1-6}$alkoxy or halogen;

w is an integer selected from 3 to 50, for instance, w is 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, or 50,

q is an integer selected from 1 to 20, for instance, q is 1, 2, 3, 4, 5, 6, 7, 8, 9, 10, 11, 12, 13, 14, 15, 16, 17, 18, 19, or 20;

s is an integer selected from 0 to 20, for instance, s is 0, 1, 2, 3, 4, 5, 6, 7, 8, 9, 10, 11, 12, 13, 14, 15, 16, 17, 18, 19, or 20;

n is an integer selected from 1 to 10; for instance, n is 1, 2, 3, 4, 5, 6, 7, 8, 9, or 10;

m is an integer selected from 1 to 20; for instance, m is 1, 2, 3, 4, 5, 6, 7, 8, 9, 10, 11, 12, 13, 14, 15, 16, 17, 18, 19, or 20;

u is an integer selected from 0 to 20; for instance, u is 0, 1, 2, 3, 4, 5, 6, 7, 8, 9, 10, 11, 12, 13, 14, 15, 16, 17, 18, 19, or 20;

p is an integer selected from 3 to 20; for instance, p is 3, 4, 5, 6, 7, 8, 9, 10, 11, 12, 13, 14, 15, 16, 17, 18, 19, or 20;

r is an integer selected from 1 to 20, for instance, r is 1, 2, 3, 4, 5, 6, 7, 8, 9, 10, 11, 12, 13, 14, 15, 16, 17, 18, 19, or 20.

As used herein, the term "alkyl" by itself or as part of another substituent, refers to a straight or branched saturated hydrocarbon group joined by single carbon-carbon bonds having 1 to 10 carbon atoms, for example 1 to 8 carbon atoms, for example 1 to 6 carbon atoms or for example 1 to 4 carbon atoms. When a subscript is used herein following a carbon atom, the subscript refers to the number of carbon atoms that the named group may contain. Thus, for example, $C_{1-6}$alkyl means an alkyl of one to six carbon atoms. Examples of alkyl groups are methyl, ethyl, propyl, isopropyl, butyl, isobutyl, sec-butyl, *tert*-butyl, 2-methylbutyl, pentyl iso-amyl and its isomers, hexyl and its isomers, heptyl and its isomers and octyl and its isomers. Where alkyl groups as defined are divalent, i.e., with two single bonds for attachment to two other groups, they are termed "alkylene" groups. Non-limiting examples of alkylene groups includes methylene, ethylene, methylmethylene, trimethylene, propylene, tetramethylene, ethylethylene, 1,2-dimethylethylene, pentamethylene and hexamethylene.

The term "aryl" as used herein by itself or as part of another group refers but is not limited to 5 to 14 carbon-atom homocyclic (i.e., hydrocarbon) monocyclic, bicyclic or tricyclic aromatic rings or ring systems containing 1 to 4 rings which are fused together or linked covalently, typically containing 5 to 10 atoms; at least one of which is aromatic. Where aryl groups as defined are divalent, i.e., with two single bonds for attachment to two other groups, they are termed "arylene" groups.

The term "$C_{1-6}$alkylcarbonyl" as used herein refers to a group of general formula $C_{1-6}$alkyl-CO, wherein $C_{1-6}$alkyl is as defined above.

The term "amino" refers to the group $-NH_2$.

The term "halo" or "halogen" as a group or part of a group is generic for fluoro, chloro, bromo or iodo.

The term "epoxy" as used herein refers to cyclic ether with only three ring atoms.

The term "$C_{1-6}$alkylarylsulfoxide" as used herein refers to a compound with a $C_{1-6}$alkyl moiety, an aryl moiety and a group of general formula $-SO_2$.

The term "heteroaryl" as used herein refers to a group of five to about a 14-membered aromatic monocyclic or multicyclic hydrocarbon ring system, including fused and spiro rings, in which one or more of the elements in the ring system is an element other than carbon and is selected from nitrogen, oxygen, silicon, or sulfur and wherein an N atom may be in the form of an N-oxide.

The term "heteroaryloxycarbonyl$C_{1-6}$alkyl" as used herein refers to a group of general formula heteroaryl-O-C(O)-$C_{1-6}$alkyl group where R is a $C_{1-6}$alkyl is a group as previously described heteroaryl group as previously described.

The term "$C_{1-6}$alkoxy" as used herein refers to a group of general formula $C_{1-6}$alkyl-O-.

The term "thio" or "thiol" as used herein refers to the -SH group. The term "isocyano" as used herein refers to the group of formula -N=C=O. The term "isothiocyano" as used herein refers to a group of formula -N=C=S.

In an embodiment, said organic molecule of Formula (III), (IV), (V), (VI), (VII), (VIII), (IX), (X), (XI) or (XII) can be optionally coupled to the blaOXA-48 or functionally active variant or fragment thereof using a multifunctional arm-spacer of Formula (XIII), (XIV), (XV), or (XVI),

| Formula | Organic molecule |
|---|---|
| XIII | $Z^1-(CH_2)_v-Z^2$ |
| XIV | $Z^1-CH_2-(O-CH_2-CH_2)_d-OCH_2_Z^2$ |
| XV | $W^1-COO-L^4-W^2$ |
| XVI | $W^1-COO-(L^5-O)_t-L^4-W^2$ |

wherein

v is an integer selected from 2 to 12, for example, v is 2, 3, 4, 5, 6, 7, 8, 9, 10, 11, or 12;
d is an integer selected from 0 to 5, for example, d is 0, 1, 2, 3, 4, or 5;

t is an integer selected from 1 to 10, for example, t is 1, 2, 3, 4, 5, 6, 7, 8, 9, or 10;

$Z^1$, $Z^2$ are each independently selected from $N_3$-aryl, or $N_3$-aryl-$CH_2$-NH-CO-, optionally substituted with halogen, $C_{1-6}$alkyl or $C_{1-6}$alkoxy; diazirinyl; -COOH and *N*-hydroxysuccinimidyl ester thereof; -$CH_2$-$NH_2$ and *N*-maleimide or *N*-succinimide derivative thereof; -$CH_2OH$ and tosylates thereof; -$CH_2$-SH and dithiane derivatives thereof; -$CH_2N$=C=O; -$CH_2N$=C=S;

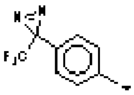 ; 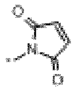 , 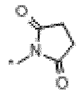 or 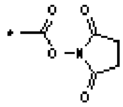 ;

$W^1$ is selected from $N_3$-aryl, optionally substituted with halogen, $C_{1-6}$alkyl or $C_{1-6}$alkoxy; diazirinyl;

$W^2$ is selected from -COOH and *N*-hydroxysuccinimidyl ester thereof; -$CH_2$-$NH_2$ and *N*-maleimide or *N*-succinimide derivative thereof; -$CH_2OH$ and tosylates thereof; -$CH_2$-SH and dithiane derivatives thereof; -$CH_2N$=C=O; -$CH_2N$=C=S;

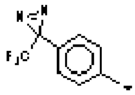 ; 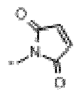 , 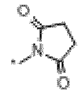 or 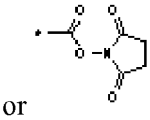 ;

and

$L^4$ and $L^5$ are defined as described above.

In an embodiment, the organic molecule of Formula (III), (IV), (V), (VI), (VII), (VIII), (IX), (X), (XI) or (XII) can be covalently coupled to a multifunctional arm-spacer of Formula (XIII), (XIV), (XV), or (XVI).

In an embodiment, for the binding of the blaOXA-48 or functionally active variant or fragment thereof to the organic molecule of Formula (III), (IV), (V), (VI), (VII), (VIII), (IX), (X), (XI) or (XII) or to the multifunctional arm-spacer of Formula (XIII), (XIV), (XV), or (XVI) on the surface of the ATR element or the quartz crystal, *N*-maleimide or *N*-succinimide derivatives are preferred. For example, the *N*-maleimide derivative can be selected from

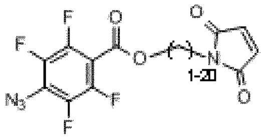 or 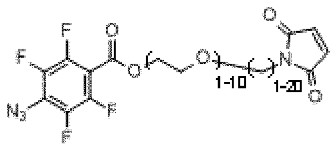 .

For example, the *N*-maleimide derivative can be

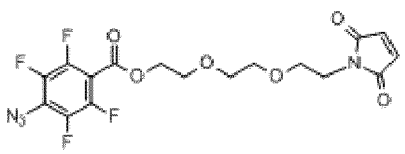 .

In an embodiment, the organic molecule of Formula (III), (IV), (V), (VI), (VII), (VIII), (IX), (X), (XI) or (XII) or the multifunctional arm-spacer of Formula (XIII), (XIV), (XV), or (XVI) is able to covalently couple the blaOXA-48 or functionally active variant or fragment thereof by reacting with a functional group of the blaOXA-48 or functionally active variant or fragment thereof.

The functional group of the blaOXA-48 or functionally active variant or fragment thereof may be any group which allows coupling of the blaOXA-48 or functionally active variant or fragment thereof with the organic molecule of Formula (III), (IV), (V), (VI), (VII), (VIII), (IX), (X), (XI) or (XII), or with the multifunctional arm-spacer of Formula (XIII), (XIV), (XV), or (XVI). The functional group may be a thiol group, a carboxyl group, an amine group, a hydroxyl group of a carbonyl group. Preferably, the functional group is a thiol group of the enzyme which can react with the organic molecule

of Formula (III), (IV), (V), (VI), (VII), (VIII), (IX), (X), (XI) or (XII), or with the multifunctional arm-spacer of Formula (XIII), (XIV), (XV), or (XVI), such as maleimidyl, isothiocyanate or succinimidyl of said organic molecule of Formula (III), (IV), (V), (VI), (VII), (VIII), (IX), (X), (XI) or (XII), or of said multifunctional arm-spacer of Formula (XIII), (XIV), (XV), or (XVI). In a particular embodiment, the functional group is a thiol group present in a variant of the blaOXA-48 or functionally active variant or fragment thereof comprising a cysteine in replacement of a native amino acid. More preferably, the functional group is a thiol group present in a variant of a wild type blaOXA-48 class D beta-lactamase as described herein which has been modified so as to replace one or more amino acids (such as two or more consecutive amino acids) in the conserved sequence NKLHVSE by a cysteine (such as by one cysteine); to insert a cysteine between any two consecutive amino acids in the conserved sequence NKLHVSE; preferably so as to replace the serine in the conserved sequence NKLHVSE by a cysteine.

Using a blaOXA-48 class D beta-lactamase comprising a cysteine is advantageous for coupling the blaOXA-48 class D beta-lactamase with the organic molecule of Formula (III), (IV), (V), (VI), (VII), (VIII), (IX), (X), (XI) or (XII) or with the multifunctional arm-spacer of Formula (XIII), (XIV), (XV), or (XVI), because the thiol group present in said cysteine is available in the opposite direction of the active site and thus the coupling does not disturb the enzymatic activity of the blaOXA-48 class D beta-lactamase.

In an embodiment, the construction of a device comprising a blaOXA-48 or a functionally active variant or fragment thereof grafted on the surface comprises the steps of:

(a1) chemically activating at least part of an ATR element's surface by oxidation, hydroxylation or reduction in acid or alkaline environment, or metal plating at least part of an ATR element's surface, or

(a2) chemically activating at least part of a gold-plated quartz crystal surface by oxidation, hydroxylation or reduction in acid or alkaline environment, and

(b) covalently grafting on said chemically activated or metal plated surface of step (a1) or (a2) an organic molecule of Formula (III), (IV), (V), (VI), (VII), (VIII), (IX), (X), (XI) or (XII).

In an embodiment, said organic molecule of Formula (III), (IV), (V), (VI), (VII), (VIII), (IX), (X), (XI) or (XII) can be optionally further covalently coupled with a multifunctional arm-spacer of Formula (XIII), (XIV), (XV), or (XVI), prior to being reacted with a the blaOXA-48 or functionally active variant or fragment thereof.

In certain embodiments, the method may be performed *in vitro* or *ex vivo*. In certain embodiments, the method may be performed *in vivo*.

In certain embodiments, the method as taught herein may be used for determining, e.g. measuring and/or monitoring, the concentration of a carbapenem antibiotic in a microdialysate. In certain embodiments of the methods as taught herein, the method may be used for determining, e.g. measuring and/or monitoring, the concentration of a carbapenem antibiotic in a microdialysate (obtained) from a subject receiving an antibiotherapy, e.g. a beta-lactam antibiotic therapy.

Microdialysis probes can be used to this effect. Preferably, the microdialysate is derived from a subject receiving an antibiotherapy, e.g. a beta-lactam antibiotic therapy, by *in vivo* microdialysis. In certain embodiments, the method as taught herein may be used for determining, e.g. measuring and/or monitoring, the concentration of a carbapenem antibiotic in a microdialysate from a subject receiving an antibiotherapy, e.g. a beta-lactam antibiotic therapy, by *in vivo* microdialysis. This has the advantage that the number of sample preparation steps can be reduced and/or that the measurement and/or monitoring of a carbapenem antibiotic level in the biological sample can be obtained in a short time interval.

In certain embodiments, the method for determining, e.g. measuring and/or monitoring, the concentration of a carbapenem antibiotic in a biological sample, such as in a microdialysate, from a subject receiving an antibiotherapy may be performed at two or more successive time points. The certain embodiments, the respective outcomes at the two or more successive time points may be compared, whereby the presence or absence of a change between the two or more successive time points is determined. Thereby, the present method allows to monitor a change in the quantity or concentration of a carbapenem antibiotic in biological samples of a subject over time.

In certain embodiments, the microdialysis step may comprise inserting a probe into tissue *in vivo,* such that one side of a semi-permeable membrane is in contact with tissue and extracellular liquid and the other side is flushed or rinsed with a dialysis liquid (perfusate) which takes up substances from the extra cellular liquid through the membrane. Pharmaceutical substances, such as antibiotics, can also be distributed locally to the extracellular liquid through the perfusion liquid. These substances can then be analysed in the microdialysate on or after exiting the probe. Probes are often made in the form of an inner and an outer tube, where the outer tube exhibits a membrane, and the dialysate and the perfusate is entering and exiting the tube at one end and the other end of the tubes are fused or plugged.

Microdialysis has the advantage of allowing the determination of the amounts of pharmaceutical substances

present or missing in patients. It also allows monitoring changes in the status of pharmaceutical substances.

In certain embodiments, the method as taught herein is used for monitoring of a subject being treated with at least one carbapenem antibiotic, e.g. undergoing antibiotherapy. Advantageous, the method increases treatment accuracy and reliability.

The terms "subject", "individual" or "patient" are used interchangeably and refer to animals, preferably warm-blooded animals, more preferably vertebrates, even more preferably mammals, still more preferably primates, and specifically includes human patients and non-human mammals and primates. Preferred patients are human subjects.

The term "mammal" includes any animal classified as such, including, but not limited to, humans, domestic and farm animals, zoo animals, sport animals, pet animals, companion animals and experimental animals, such as, for example, mice, rats, hamsters, rabbits, dogs, cats, guinea pigs, gerbils, cattle, cows, sheep, horses, pigs and primates, e.g., monkeys and apes (e.g., chimpanzee, baboon, or monkey). Particularly preferred are human subjects, including both genders and all age categories thereof.

In certain embodiments of the methods as taught herein, the method may be used for determining, e.g. measuring and/or monitoring, the concentration of a carbapenem antibiotic in a biological sample obtained from a subject to refine clinical treatment intervention on the subject, preferably wherein refining the clinical treatment intervention comprises refining one or more of the dosage, the duration, or the schedule of administration of the carbapenem antibiotic.

In certain embodiments of the methods taught herein, the method may be used for determining, e.g. measuring and/or monitoring, the concentration of a carbapenem antibiotic in a biological sample obtained from a subject being treated for or in need of treatment of an infection caused by Gram-negative bacteria, Gram-positive bacteria and/or MDR bacteria such as broad-spectrum beta-lactamase producing enterobacteria or Amp-C, *Pseudomonas aeruginosa,* and *Acinetobacter baumannii.*

Hence, related aspects provide the use of a blaOXA-48 class D beta-lactamase or a functionally active variant or fragment thereof:

- for determining, e.g. measuring and/or monitoring, the concentration of a carbapenem antibiotic in a biological sample;
- for determining, e.g. measuring and/or monitoring, the concentration of a carbapenem antibiotic in a microdialysate obtained from a subject receiving an antibiotherapy;
- for determining, e.g. measuring and/or monitoring, the concentration of a carbapenem antibiotic in a biological sample obtained from a subject to refine clinical treatment intervention on the subject, preferably wherein refining the clinical treatment intervention comprises refining one or more of the dosage, the duration, or the schedule of administration of the carbapenem antibiotic; and/or
- for determining, e.g. measuring and/or monitoring, the concentration of carbapenem antibiotic in a biological sample obtained from a subject being treated for or in need of treatment of an infection caused by Gram-negative bacteria, Gram-positive bacteria and/or MDR bacteria.

As illustrated herein, the present inventors found that the blaOXA-48 class D beta-lactamase or functionally active variant or fragment can act as an efficient biosensor for the determination of the concentration of a carbapenem antibiotic in a biological sample such as serum. The blaOXA-48 class D beta-lactamase or functionally active variant or fragment thereof allows measuring and/or monitoring of a carbapenem antibiotic in the presence of other beta-lactam antibiotics as well as other pharmaceutical substances commonly used in intensive care units due to a high affinity of the beta-lactamase for the carbapenem antibiotic combined with a slow formation of the covalent beta-lactamase-carbapenem adduct during catalysis.

The present application also provides aspects and embodiments as set forth in the following Statements:

Statement 1. Method for determining the concentration of a carbapenem antibiotic in a biological sample, comprising the steps of:

(a) providing a blaOXA-48 class D beta-lactamase (blaOXA-48) or a functionally active variant or fragment thereof;

(b) providing a biological sample;

(c) contacting the blaOXA-48 or functionally active variant or fragment thereof with the biological sample; and

(d) determining the concentration of the carbapenem antibiotic in the biological sample.

Statement 2. The method according to statement 1, wherein the blaOXA-48 or functionally active variant or fragment thereof is fused with its N-terminus and/or C-terminus to a further polypeptide, or is inserted into a further polypeptide; preferably wherein the further polypeptide is an enzyme.

Statement 3. The method according to statement 1 or 2, wherein the method comprises, prior to step (c), contacting the blaOXA-48 or functionally active variant or fragment thereof with a reporter substrate.

Statement 4. The method according to any one of statements 1 to 3, wherein the measuring step (d) is performed by Ultraviolet-visible (UV-VIS) spectroscopy, fluorescence spectroscopy, fluorescence resonance energy transfer (FRET) spectroscopy, Fourier Transform Infrared (FTIR) spectroscopy, plasmon resonance, electrochemical detection (ECD), or by surface sensitive wave based technique such as quartz crystal microbalance (QCM).

Statement 5. The method according to any one of statements 1 to 4, wherein the method comprises the steps of contacting the biological sample with a reporter substrate and the blaOXA-48 or functionally active variant or fragment thereof, and measuring the amount of a spectrophotometric signal, an optical signal and/or an electrical signal in the biological sample in comparison with a standard, thereby determining the concentration of the carbapenem antibiotic in the biological sample.

Statement 6. The method according to any one of statements 3 to 5, wherein the reporter substrate is a chromogenic substrate.

Statement 7. The method according to any one of statements 1 to 6, wherein the concentration is an absolute concentration.

Statement 8. The method according to any one of statements 1 to 7, wherein the carbapenem antibiotic is one or more of meropenem, ertapenem, doripenem, or imipenem, preferably wherein the carbapenem antibiotic is meropenem.

Statement 9. The method according to any of statements 1 to 8, wherein the biological sample is selected from the group consisting of serum, blood, urine, interstitial fluid, saliva, tears, exudates, fluid collected from deep tissues, and fluid collected from subcutaneous tissues, preferably the biological sample is serum.

Statement 10. The method according to any of statements 1 to 9, wherein the biological sample comprises:

- a carbapenem antibiotic, and
- one or more other beta-lactam antibiotics and/or one or more other pharmaceutical substances.

Statement 11. The method according to any of statements 1 to 10, wherein the biological sample is a biological sample obtained from a subject receiving an antibiotherapy and/or wherein the biological sample is a biological sample obtained from a subject being treated for or in need of treatment of an infection caused by Gram-negative bacteria, Gram-positive bacteria and/or multidrug-resistant (MDR) bacteria.

Statement 12. The method according to any one of statements 1 to 11, wherein the blaOXA-48 or functionally active variant or fragment thereof is grafted on at least one surface of a device, preferably wherein said at least one surface is chemically activated.

Statement 13. The method according to statement 12, wherein the device comprises an attenuated total internal reflection element, transparent in the infrared, or the device is a quartz crystal microbalance.

Statement 14. The method according to any one of statements 1 to 13, wherein the blaOXA-48 is selected from the group consisting of beta-lactamase class D OXA-48, beta-lactamase class D OXA-54, beta-lactamase class D OXA-162, beta-lactamase class D OXA-163, beta-lactamase class D OXA-181, beta-lactamase class D OXA-199, beta-lactamase class D OXA-204, beta-lactamase class D OXA-244, beta-lactamase class D OXA-245, beta-lactamase class D OXA-247, beta-lactamase class D OXA-232, beta-lactamase class D OXA-370, beta-lactamase class D OXA-405, beta-lactamase class D OXA-416, beta-lactamase class D OXA-438, beta-lactamase class D OXA-439,

beta-lactamase class D OXA-484, beta-lactamase class D OXA-436, beta-lactamase class D OXA-505, beta-lactamase class D OXA-514, beta-lactamase class D OXA-515, beta-lactamase class D OXA-517, beta-lactamase class D OXA-519, beta-lactamase class D OXA-538, beta-lactamase class D OXA-547, beta-lactamase class D OXA-546, beta-lactamase class D OXA-566, and beta-lactamase class D OXA-252; preferably wherein the blaOXA-48 is beta-lactamase class D OXA-48.

Statement 15. The method according to any one of statements 1 to 14, wherein the method is used for:

- determining the concentration of a carbapenem antibiotic in a microdialysate obtained from a subject receiving an antibiotherapy;

- for determining the concentration of a carbapenem antibiotic in a biological sample obtained from a subject to refine clinical treatment intervention on the subject, preferably wherein refining the clinical treatment intervention comprises refining one or more of the dosage, the duration, or the schedule of administration of the carbapenem antibiotic; and/or

- for determining the concentration of a carbapenem antibiotic in a biological sample obtained from a subject being treated for or in need of treatment of an infection caused by Gram-negative bacteria, Gram-positive bacteria and/or multidrug-resistant (MDR) bacteria.

While the invention has been described in conjunction with specific embodiments thereof, it is evident that many alternatives, modifications, and variations will be apparent to those skilled in the art in light of the foregoing description. Accordingly, it is intended to embrace all such alternatives, modifications, and variations as follows in the spirit and broad scope of the appended claims.

The herein disclosed aspects and embodiments of the invention are further supported by the following non-limiting examples.

**Sequence listing**

Throughout the description and Examples, reference is made to the following sequences:

| | |
|---|---|
| SEQ ID NO: 1: | OXA-48 polypeptide annotated under NCBI Reference Sequence WP_015059991.1 |
| SEQ ID NO: 2: | OXA-54 polypeptide annotated under NCBI Reference Sequence WP_011071128.1 |
| SEQ ID NO: 3: | OXA-162 polypeptide annotated under NCBI Reference Sequence WP_060613455.1 |
| SEQ ID NO: 4: | OXA-163 polypeptide annotated under NCBI Reference Sequence WP_063131934.1 |
| SEQ ID NO: 5: | OXA-181 polypeptide annotated under NCBI Reference Sequence WP_015060052.1 |
| SEQ ID NO: 6: | OXA-199 polypeptide annotated under NCBI Reference Sequence WP 063861505.1 |
| SEQ ID NO: 7: | OXA-204 polypeptide annotated under NCBI Reference Sequence WP_032495449.1 |
| SEQ ID NO: 8: | OXA-244 polypeptide annotated under NCBI Reference Sequence WP_032495517.1 |
| SEQ ID NO: 9: | OXA-245 polypeptide annotated under NCBI Reference Sequence WP_032495518.1 |
| SEQ ID NO: 10: | OXA-247 polypeptide annotated under NCBI Reference Sequence WP_032495595.1 |
| SEQ ID NO: 11: | OXA-232 polypeptide annotated under NCBI Reference Sequence WP_043907054.1 |
| SEQ ID NO: 12: | OXA-370 polypeptide annotated under NCBI Reference Sequence WP 032495789.1 |
| SEQ ID NO: 13: | OXA-405 polypeptide annotated under NCBI Reference Sequence WP_063862762.1 |
| SEQ ID NO: 14: | OXA-416 polypeptide annotated under NCBI Reference Sequence WP_063862782.1 |
| SEQ ID NO: 15: | OXA-438 polypeptide annotated under NCBI Reference Sequence WP_063864080.1 |
| SEQ ID NO: 16: | OXA-439 polypeptide annotated under NCBI Reference Sequence WP_063864081.1 |
| SEQ ID NO: 17: | OXA-484 polypeptide annotated under NCBI Reference Sequence WP_063864110.1 |
| SEQ ID NO: 18: | OXA-436 polypeptide annotated under NCBI Reference Sequence WP_058842180.1 |
| SEQ ID NO: 19: | OXA-505 polypeptide annotated under NCBI Reference Sequence WP_063864118.1 |
| SEQ ID NO: 20: | OXA-514 polypeptide annotated under NCBI Reference Sequence WP_094009803.1 |
| SEQ ID NO: 21: | OXA-515 polypeptide annotated under NCBI Reference Sequence WP_094009804.1 |
| SEQ ID NO: 22: | OXA-517 polypeptide annotated under NCBI Reference Sequence WP_085562384.1 |
| SEQ ID NO: 23: | OXA-519 polypeptide annotated under NCBI Reference Sequence WP_094009808.1 |
| SEQ ID NO: 24: | OXA-538 polypeptide annotated under NCBI Reference Sequence WP_071593227.1 |

(continued)

| | |
|---|---|
| SEQ ID NO: 25: | OXA-547 polypeptide annotated under NCBI Reference Sequence WP_085562403.1 |
| SEQ ID NO: 26: | OXA-546 polypeptide annotated under NCBI Reference Sequence WP_087587945.1 |
| SEQ ID NO: 27: | OXA-566 polypeptide annotated under NCBI Reference Sequence WP_094009811.1 |
| SEQ ID NO: 28: | OXA-252 polypeptide annotated under NCBI Reference Sequence WP_037428895.1 |
| SEQ ID NO: 29: | Mature OXA-48 polypeptide |
| SEQ ID NO: 30: | Mature OXA-54 polypeptide |
| SEQ ID NO: 31: | Mature OXA-162 polypeptide |
| SEQ ID NO: 32: | Mature OXA-163 polypeptide |
| SEQ ID NO: 33: | Mature OXA-181 polypeptide |
| SEQ ID NO: 34: | Mature OXA-199 polypeptide |
| SEQ ID NO: 35: | Mature OXA-204 polypeptide |
| SEQ ID NO: 36: | Mature OXA-244 polypeptide |
| SEQ ID NO: 37: | Mature OXA-245 polypeptide |
| SEQ ID NO: 38: | Mature OXA-247 polypeptide |
| SEQ ID NO: 39: | Mature OXA-232 polypeptide |
| SEQ ID NO: 40: | Mature OXA-370 polypeptide |
| SEQ ID NO: 41: | Mature OXA-405 polypeptide |
| SEQ ID NO: 42: | Mature OXA-416 polypeptide |
| SEQ ID NO: 43: | Mature OXA-438 polypeptide |
| SEQ ID NO: 44: | Mature OXA-439 polypeptide |
| SEQ ID NO: 45: | Mature OXA-484 polypeptide |
| SEQ ID NO: 46: | Mature OXA-436 polypeptide |
| SEQ ID NO: 47: | Mature OXA-505 polypeptide |
| SEQ ID NO: 48: | Mature OXA-514 polypeptide |
| SEQ ID NO: 49: | Mature OXA-515 polypeptide |
| SEQ ID NO: 50: | Mature OXA-517 polypeptide |
| SEQ ID NO: 51: | Mature OXA-519 polypeptide |
| SEQ ID NO: 52: | Mature OXA-538 polypeptide |
| SEQ ID NO: 53: | Mature OXA-547 polypeptide |
| SEQ ID NO: 54: | Mature OXA-546 polypeptide |
| SEQ ID NO: 55: | Mature OXA-566 polypeptide |
| SEQ ID NO: 56: | Mature OXA-252 polypeptide |

## EXAMPLES

### Example 1: Screening of beta-lactamases for measuring the concentration of a carbapenem antibiotic

In a bibliographic search, several beta-lactamases were screened for their capacity to quantify by competition a carbapenem antibiotic, meropenem. The beta-lactamase collection covered all beta-lactamase classes: A, C and D beta-lactamases, that are serine enzymes, and class B beta-lactamases that are metallo-enzymes. Some beta-lactamases were recognized as poor enzymes to hydrolyse carbapenem antibiotics. A list of beta-lactamases was selected and the activity of the selected enzymes was determined with nitrocefin as a reporter substrate (see Example 2) (Table 2).

Beta-lactamases that were not capable to quantify by competition meropenem were rapidly eliminated without further characterization.

Table 2: Different beta-lactamases for testing, and results of screening of beta-lactamase enzymes. NA means not determined. Symbols are correlated with hydrolysis rate following Table 3

| **Beta-lactamase** | **Bla P** | **CTX M15** | **Kpc2** | **Per2** | **Imp 1** | **Imp 4** | **Ndm 1** | **Vim 1** | **Amp CHD** | **P99** | **Oxa4 8** |
|---|---|---|---|---|---|---|---|---|---|---|---|
| Class type | A | A | A | A | B | B | B | B | C | C | D |

(continued)

| Beta-lactamase | Bla P | CTX M15 | Kpc2 | Per2 | Imp 1 | Imp 4 | Ndm 1 | Vim 1 | Amp CHD | P99 | Oxa4 8 |
|---|---|---|---|---|---|---|---|---|---|---|---|
| Activity loss in 1 h | 6% | 0% | 2% | 0% | 0% | 19% | 0% | 16% | 0% | 0% | 0% |
| Augmentin® P | NA | + | NA | ++ | NA | - | - | NA | +++ | ++ | - |
| Azactam® | NA | NA | NA | + | NA | NA | NA | NA | - | +++ | NA |
| Cefamandole® | NA | NA | - | + | ++ | ++ | + | NA | ++ | ++ | - |
| Cefotaxim® Sandoz | NA | NA | NA | - | + | + | - | NA | + | ++++ | - |
| Ceftria® | NA | NA | NA | - | NA | - | - | NA | - | +++ | NA |
| Claventin® | NA | ++ | NA | +++ | NA | NA | - | NA | + | ++++ | - |
| Kefzol® | NA | NA | NA | - | - | + | + | NA | NA | NA | NA |
| Penicilline | NA | - | NA | ++ | NA | - | - | NA | ++ | ++ | + |
| Penstapho® | NA | - | + | ++ | NA | NA | - | - | ++ | ++++ | - |
| Pentrexyl® | NA | - | NA | + | NA | - | - | NA | +++ | ++ | - |
| Ticarpen® | NA | ++ | NA | ++ | NA | NA | - | NA | + | ++++ | + |
| Tienam® | NA | + | + | +++ + | NA | NA | - | + | ++ | +++ | ++ |
| Zinacef® | NA | NA | NA | - | + | NA | - | NA | + | ++++ | NA |
| Ceftazidime | NA | NA | NA | NA | NA | NA | NA | NA | - | + | NA |
| Tazocin® | ++ + | +++ | NA | +++ | NA | NA | NA | - | ++ | ++ | + |
| Meropenem | + | +++ | + | +++ | + | + | NA | NA | + | +++ | +++ |

Table 3: Explanation of hydrolysis rate symbols used in Table 2

| | | | | | |
|---|---|---|---|---|---|
| (Vmax/V0)-1 | <0.1 | 0.1-1 | 1-10 | 10-100 | >100 |
| Symbol | - | + | ++ | +++ | ++++ |

**Example 2: Method for quantitative measurement of carbapenem antibiotic using a colorimetric assay according to an embodiment of the present invention**

The principle of a determination method according to an embodiment of the invention is based on a competition between a reporter substrate and a substrate of interest for a same enzyme. The selected enzyme is used as a biosensor. In absence of carbapenem antibiotic, the biosensor hydrolyses the reporter substrate. This leads to a reaction product that may be measured for instance by a spectroscopic or colorimetric method. In presence of carbapenem substrate, also referred to as analyte, the biosensor is less available to hydrolyse the reporter substrate. Accordingly, the higher the concentration of carbapenem analyte, the lower is the response measured by the spectroscopic or colorimetric measure.

Scheme 1 shows the kinetic models of the reaction of biosensor (E) with the reporter substrate ($S_R$) and the beta-lactam analyte (A) (Frère et al, Eur. J. Biochem., 1975, 57: 343-351).

The rate equation describing the kinetic model can be resumed to equation 1 as described herein.

The present inventors surprisingly found that for the beta-lactamase class D OXA-48 remained mostly immobilized in the first complex E.A in the case of binding with carbapenem antibiotics. For this, it requires a low K' (also refer to as $K_m$ in the literature). The difference in the velocity of the hydrolysis of the $S_R$ by the biosensor in the absence and presence of the analyte relied upon the respective non-covalent complex formation (K and K') and acylation rate ($k_2$ and $k'_2$) and resulted in the immobilisation of the enzyme at E.A step.

With the enzyme OXA-48, the present inventors did not observe any competition between meropenem and other beta-lactam antibiotics. Indeed, the difference of absorbance as measured by a spectroscopic method was only

due to the meropenem antibiotic.

The reporter substrate ($S_R$) used was nitrocefin, a commercially available chromogenic cephalosporin (O'Callaghan et al., Antimicrob. Agents and Chemother., 1972, 1: 283-288). In the absence of carbapenem antibiotic, the biosensor rapidly hydrolysed the $S_R$ and the resulting reaction product exhibited a deep red coloration (maximum absorbance at 482 nm). In presence of the carbapenem analyte, the biosensor was less available to hydrolyse the reporter substrate. Accordingly, the higher the concentration of carbapenem analyte, the lower was the coloration of the solution. In this enzymatic competitive kinetic assay, the variation of absorbance at 482 nm per min ($\Delta A^{482nm}$/min) was inversely proportional to the concentration of carbapenem analyte.

The test was carried out using a computer controlled AMS Ellipse analyser (AMS Spa, Rome Italy). 10$\mu$l of sample, i.e. quality control samples (QC samples) or ultrafiltrated spiked serum, corresponding to meropenem free-fraction, were mixed with 190 $\mu$l of $S_R$ (nitrocefin (110 $\mu$M) in buffer (pH 7.4) comprising $KH_2PO_4$ (1.76 mM), $Na_2HPO_4.2H_2O$ (10 mM), NaCl (137 mM), $KCl_2$ (7 mM)) by the Ellipse analyser.

Then, 10 $\mu$l ofbeta-lactamase class D OXA-48 (also referred to herein as "M4S-Biosensor-2") (14 nM or $\pm$ 8 $10^{-4}$ U/$\mu$l in nitrocefin (110 $\mu$M) in buffer (pH 7.4) comprising $KH_2PO_4$ (1.76 mM), $Na_2HPO_4.2H_2O$ (10 mM), NaCl (137 mM), $KCl_2$ (7mM), $NaHCO_3$ (100mM), ethylene glycol (8%)) was automatically added and mixed with the other reactants. The enzymatic hydrolysis of $S_R$ at 37°C, was spectrophotometrically and automatically recorded during 91 seconds by measuring the increase of the absorbance at 482 nm. These raw data were then transferred to another computer to be analysed by in-house developed software to calculate the initial velocity expressed as $\Delta A^{482nm}$/min.

QC samples were automatically generated by successive dilutions from a meropenem stock solution (20mg/L) prepared daily, to obtain analyte concentrations ranging from lower limit of quantification to upper limit of quantification.

The Blank sample was as a QC sample in which the volume of meropenem solution is replaced by the same volume of reaction buffer.

After analysis, the initial velocities obtained for blank and QC samples were used to automatically generate the standard curve following equation 1.

Tests have shown that the lower limit of quantification (LLOQ) was 0.5 mg/L of meropenem and upper limit of quantification (ULOQ) was 20 mg/L of meropenem.

**Example 3: Specificity of beta-lactamase class D OXA-48 for carbapenem antibiotic in the presence of other beta-lactam antibiotics in a method for quantitative measurement of the carbapenem antibiotic according to an embodiment of the present invention**

The specificity (or selectivity) of the method according to an embodiment of the invention is defined as the ability of the method to measure the concentration of a carbapenem antibiotic (such as meropenem) in presence of an excess of another beta-lactam antibiotic (second beta-lactam) currently used in ICUs (see Table 4).

To determine the specificity of the method illustrating the invention for carbapenem antibiotics, in particular meropenem, versus other beta-lactam antibiotics, stock-solutions of second beta-lactam antibiotics were prepared in water.

Two meropenem concentrations were selected: 1.2 and 20 mg/L, which covers the range of quantification. An excess of a second beta-lactam concentration of up to 10 times the concentration of meropenem was used.

The results are presented in Tables 4 and 5. The accuracy lies within the range of 90 to 120 %.

Table 4: Meropenem concentrations and average accuracies obtained for $QC^{SUS}$ samples contained 1.2 mg/L of meropenem and an excess of second β-lactam antibiotic fixed to 10.0 mg/L (8.4 times excess)

| Interfering antibiotics | **Meropenem 1.2 mg/L** | | | | | | | | | | | | |
|---|---|---|---|---|---|---|---|---|---|---|---|---|---|
| | [IA] (mg/L) | [Meropenem] (mg/L) | | | | | | Accuracy (%) | | | | | |
| | | Replicates | | | | | <> | Replicates | | | | | <> |
| | | 1 | 2 | 3 | 4 | 5 | | 1 | 2 | 3 | 4 | 5 | |
| Augmentin | 10.0 | 1.3 | 1.3 | 1.3 | 1.1 | 1.3 | **1.3** | 114 | 114 | 111 | 97 | 113 | **110** |
| Azactam | 10.0 | 1.2 | 1.2 | 1.2 | 1.3 | 1.2 | **1.2** | 102 | 106 | 101 | 113 | 101 | **104** |
| Cefamandole | 10.0 | 1.3 | 1.3 | 1.3 | 1.4 | 1.3 | **1.3** | 112 | 116 | 114 | 123 | 112 | **115** |
| Cefotaxim | 10.0 | 1.3 | 1.3 | 1.2 | 1.3 | 1.3 | **1.3** | 109 | 116 | 107 | 110 | 115 | **111** |
| Ceftria | 10.0 | 1.2 | 1.3 | 1.3 | 1.3 | 1.3 | **1.3** | 105 | 111 | 112 | 116 | 113 | **112** |
| Claventin | 10.0 | 1.2 | 1.2 | 1.1 | 1.2 | 1.1 | **1.1** | 100 | 99 | 96 | 104 | 94 | **99** |
| Kefzol | 10.0 | 1.2 | 1.1 | 1.4 | 1.3 | 1.1 | **1.2** | 107 | 91 | 117 | 111 | 97 | **105** |
| Penicilline | 10.0 | 1.2 | 1.3 | 1.3 | 1.3 | 1.2 | **1.3** | 107 | 110 | 109 | 112 | 106 | **109** |
| Penstapho | 10.0 | 1.3 | 1.4 | 1.4 | 1.4 | 1.3 | **1.4** | 115 | 123 | 121 | 122 | 116 | **119** |
| Pentrexyl | 10.0 | 1.4 | 1.3 | 1.3 | 1.3 | 1.3 | **1.3** | 122 | 112 | 110 | 109 | 108 | **112** |
| Ticarpen | 10.0 | 1.4 | 1.4 | 1.2 | 1.3 | 1.2 | **1.3** | 121 | 122 | 102 | 111 | 105 | **112** |
| Zinacef | 10.0 | 1.3 | 1.1 | 1.2 | 1.2 | 1.3 | **1.2** | 108 | 97 | 100 | 104 | 111 | **104** |
| Cefepime | 10.0 | 1.2 | 1.2 | 1.2 | 1.3 | 1.4 | **1.2** | 103 | 102 | 99 | 108 | 118 | **106** |
| Ceftazidime | 10.0 | 1.3 | 1.2 | 1.3 | 1.3 | 1.4 | **1.3** | 108 | 105 | 108 | 112 | 118 | **111** |
| Tazocin | 10.0 | 1.4 | 1.3 | 1.1 | 1.1 | 1.2 | **1.2** | 117 | 110 | 97 | 95 | 102 | **104** |
| Blank | 0.0 | 0.0 | 0.02 | -0.01 | -0.09 | 0.08 | **0.0** | - | - | - | - | - | - |
| Meropenem | 1.2 | 1.1 | 1.2 | 1.1 | 1.3 | 1.1 | **1.2** | 98 | 100 | 97 | 108 | 97 | **100** |

Table 5: Meropenem concentrations and average accuracies obtained for $QC^{SUS}$ samples contained 20 mg/L of meropenem and an excess of second β-lactam antibiotic fixed to 200.0 mg/L (10 times excess)

| Interfering antibiotics | Meropenem 20.0 mg/L | | | | | | | | | | | | |
|---|---|---|---|---|---|---|---|---|---|---|---|---|---|
| | [IA] (mg/L) | [Meropenem] (mg/L) | | | | | | Accuracy (%) | | | | | |
| | | Replicates | | | | | <> | Replicates | | | | | <> |
| | | 1 | 2 | 3 | 4 | 5 | | 1 | 2 | 3 | 4 | 5 | |
| Augmentin | 200.0 | 21.2 | 21.3 | 21.5 | 21.2 | 20.4 | **21.1** | 95 | 95 | 97 | 95 | 91 | **95** |
| Azactam | 200.0 | 20.1 | 20.7 | 21.9 | 20.9 | 21.3 | **21.0** | 90 | 93 | 98 | 94 | 95 | **94** |
| Cefamandole | 200.0 | 21.5 | 21.9 | 22.2 | 23.4 | 21.0 | **22.0** | 97 | 98 | 99 | 105 | 94 | **99** |
| Cefotaxim | 200.0 | 20.8 | 20.9 | 20.2 | 20.2 | 20.2 | **20.5** | 93 | 94 | 91 | 91 | 91 | **92** |
| Ceftria | 200.0 | 22.3 | 21.4 | 21.4 | 20.0 | 20.4 | **21.1** | 100 | 96 | 96 | 90 | 91 | **95** |
| Claventin | 200.0 | 21.3 | 24.0 | 22.7 | 22.4 | 22.7 | **22.6** | 95 | 108 | 102 | 101 | 102 | **101** |
| Kefzol | 200.0 | 21.2 | 21.8 | 21.4 | 21.5 | 21.5 | **21.5** | 95 | 98 | 96 | 97 | 97 | **96** |
| Penicilline | 200.0 | 21.4 | 21.8 | 21.9 | 21.8 | 22.2 | **21.8** | 96 | 98 | 98 | 98 | 99 | **98** |
| Penstapho | 200.0 | 21.8 | 23.2 | 23.1 | 23.8 | 22.7 | **22.9** | 98 | 104 | 104 | 107 | 102 | **103** |
| Pentrexyl | 200.0 | 20.5 | 20.8 | 21.4 | 21.3 | 21.8 | **21.1** | 92 | 93 | 96 | 95 | 98 | **95** |
| Ticarpen | 200.0 | 22.7 | 23.4 | 23.1 | 21.9 | 21.9 | **22.6** | 102 | 105 | 104 | 98 | 98 | **101** |
| Zinacef | 200.0 | 21.8 | 21.9 | 21.2 | 22.4 | 22.0 | **21.9** | 98 | 98 | 95 | 101 | 99 | **98** |
| Cefepime | 200.0 | 19.9 | 19.9 | 20.0 | 21.6 | 22.0 | **20.7** | 98 | 98 | 99 | 107 | 109 | **102** |
| Ceftazidime | 200.0 | 20.6 | 19.6 | 20.1 | 20.2 | 20.0 | **20.1** | 102 | 97 | 100 | 100 | 99 | **100** |
| Tazocin | 200.0 | 20.9 | 20.8 | 19.5 | 19.5 | 19.8 | **20.1** | 104 | 103 | 97 | 97 | 98 | **100** |
| Blank | 0.0 | 0.03 | -0.02 | -0.02 | 0.00 | -0.02 | **-0.01** | - | - | - | - | - | - |
| Meropenem | 20.0 | 20.7 | 20.9 | 19.8 | 19.9 | 19.6 | 20.2 | 103 | 104 | 98 | 98 | 97 | **100** |

Tables 4 and 5 show that the method illustrating the invention allowed to specifically determine the concentration of a carbapenem antibiotic, meropenem (1.2 mg/L and 20.0 mg/L), in the presence of other beta-lactam antibiotics.

**Example** 4: **Specificity of beta-lactamase class D OXA-48 for carbapenem antibiotic versus non beta-lactam drugs in a method for quantitative measurement of the carbapenem antibiotic according to an embodiment of the present invention**

The specificity (or selectivity) of the method according to an embodiment of the invention for a carbapenem antibiotic (such as meropenem) in presence of other drugs is defined as the ability of the method to quantify and differentiate the carbapenem antibiotic, in particular meropenem, in the presence of another drug (second drug) currently used in ICUs and listed below.

The stock-solutions of potentially interfering drugs were prepared following the dilutions as shown in Table 6. The final drug concentration in ultra-filtrated serum was fixed to 10 times the generally recommended concentration in blood. Non-beta-lactam compounds were Actrapid (Novo Nordisk, 4548); Adrenaline (Aguettant, 4301381); Anidulafungin (Pfizer, A09109); Calciclo (Sterop Belgium, 140245); Caspofungin (Merck-Sharp & Dohme Ltd, 2159760); Clonazepam (Roche, F0096); Cordarone (Sterop Belgium, 4YO30); Dapakine (Sanofi, A4521); Diazepam (Roche, F1032); Diphantoïne (Kela Pharma, 140172); Dobutrex (Mylan bvba, F1072); Dopamine (Renaudin, 201591); Fluconazole (Fresenius Kabi, 15HB12R1); Heparine (Leo Pharma, DH6929); Keppra (UCB, 14097); Lasix (Sanofi, AY008); Liposomial amphotericin B (Gilead, 042501AD); Midazolam (B. Braun, 14247011); Milrinone (Sanofi-Aventis, 3Y013A); Morphine (Sterop Belgium, 140232); Nimbex (GSK, 4548); Nimotop (Bayer, KP09XNU); Noradrenaline (Aguettant, 4301308); Pantomed (Takeda, 282119); Prograft (Astellas Pharma, 5A3281D); Propolipid (Fresinus Kabi, 16IB0271); Protamine (Leo Pharma, F2026); Rydene (Astellas Pharma, 14C75/101); Salbutamol (Mylan, D1016); Sandimmum (Novartis, S0072); Solu-Cortef (Pfizer, L65966); Solu-Medrol (Pfizer, L09838); Sufentanil (Mylan, H2171); Thiobarbital (B. Braun, 1440511); Ultiva (GSK, V037); and Voriconazole (Pfizer, Z325005).

Table 6: Sample preparation of non-beta-lactam compounds

| N° | Product | Initial Conc | Final Conc | Dilution | Product Volume | Serum phi Volume |
|---|---|---|---|---|---|---|
| 1 | Actrapid | 100 UI/ml | 0.6 UI/ml | 166.7 | 6 µl | 994 µl |
| 2 | Calciclo | 1.1 mEQ/ml | 0.022 mEQ/ml | 50.0 | 20 µl | 980 µl |
| 3 | Cordarone | 50 mg/ml | 0.3 mg/ml | 166.7 | 6 µl | 994 µl |
| 4 | Dapakine | 100 mg/ml | 0.8 mg/ml | 125.0 | 8 µl | 992 µl |
| 5 | Diphantoïne | 50 mg/ml | 0.5 mg/ml | 100.0 | 10 µl | 990 µl |
| 6 | Dobutrex | 12.5 mg/ml | 0.5 mg/ml | 25.0 | 40 µl | 960 µl |
| 7 | Dopamine | 40 mg/ml | 0.4 mg/ml | 100.0 | 10 µl | 990 µl |
| 8 | Heparine Leo | 5000 UI/ml | 50 UI/ml | 100.0 | 10 µl | 990 µl |
| 9 | Keppra | 100 mg/ml | 1 mg/ml | 100.0 | 10 µl | 990 µl |
| 10 | Lasix | 10 mg/ml | 0.04 mg/ml | 250.0 | 4 µl | 996 µl |
| 11 | Noradrenaline | 1 mg/ml | 0.008 mg/ml | 125.0 | 8 µl | 992 µl |
| 12 | Midazolam Braun | 5 mg/ml | 0.1 mg/ml | 50.0 | 20 µl | 980 µl |
| 13 | Morphine | 10 mg/ml | 0.02 mg/ml | 500.0 | 2 µl | 998 µl |
| 14 | Nimbex | 2 mg/ml | 0.02 mg/ml | 100.0 | 10 µl | 990 µl |
| 15 | Nimotop | 0.2 mg/ml | 0.02 mg/ml | 10.0 | 100 µl | 900 µl |
| 16 | Pantomed | 4 mg/ml | 0.08 mg/ml | 50.0 | 20 µl | 980 µl |
| 17 | Prograft | 5 mg/ml | 0.01 mg/ml | 500.0 | 2 µl | 998 µl |
| 18 | Propolipid | 20 mg/ml | 2 mg/ml | 10.0 | 100 µl | 900 µl |
| 19 | Protamine | 1400 UI/ml | 14 UI/ml | 100.0 | 10 µl | 990 µl |
| 20 | Rydene | 1 mg/ml | 0.01 mg/ml | 100.0 | 10 µl | 990 µl |
| 21 | Sandimmum | 50 mg/ml | 0.5 mg/ml | 100.0 | 10 µl | 990 µl |

(continued)

| N° | Product | Initial Conc | Final Conc | Dilution | Product Volume | Serum phi Volume |
|---|---|---|---|---|---|---|
| **22** | Solu-cortef | 50 mg/ml | 0.2 mg/ml | 250.0 | 4 μl | 996 μl |
| **23** | Solu-Medrol | 40 mg/ml | 0.08 mg/ml | 500.0 | 2 μl | 998 μl |
| **24** | Thiobarbital | 100 mg/ml | 0.3 mg/ml | 333.3 | 3 μl | 997 μl |
| **25** | Ultiva | 2 mg/ml | 0.004 mg/ml | 500.0 | 2 μl | 998 μl |
| **26** | Adrenaline | 1 mg/ml | 0.008 mg/ml | 125.0 | 8 μl | 992 μl |
| **27** | Anidulafungin | 5 mg/ml | 0.07 mg/ml | 71.4 | 14 μl | 986 μl |
| **28** | Caspofungin | 5 mg/ml | 0.14 mg/ml | 35.7 | 28 μl | 972 μl |
| **29** | Clonazepam | 1 mg/ml | 0.00075 mg/ml | 1333.3 | 0.75 μl | 999.25 μl |
| **30** | Diazepam | 5 mg/ml | 0.012 mg/ml | 416.7 | 2.4 μl | 997.6 μl |
| **31** | Fluconazole | 2 mg/ml | 0.34 mg/ml | 5.9 | 170 μl | 830 μl |
| **32** | Liposomial amphotericin B | 5 mg/ml | 0.84 mg/ml | 6.0 | 168 μl | 832 μl |
| **33** | Milrinone | 1 mg/ml | 0.003 mg/ml | 333.3 | 3 μl | 997 μl |
| **34** | Salbutamol | 1 mg/ml | 0.0001 mg/ml | 10000.0 | 0.1 μl | 999.9 μl |
| **35** | Sufentanil | 0.005 mg/ml | 0.0002 mg/ml | 25.0 | 40 μl | 960 μl |
| **36** | Voriconazole | 20 mg/ml | 0.04 mg/ml | 500.0 | 2 μl | 998 μl |

For each potentially interfering drug sample assayed by the test, three replicates were carried out and the results were expressed as meropenem concentration, which was theoretically zero. Under these assay conditions, the interference of second drug with the method according to an embodiment using OXA-48 may be highlighted by the determination of a meropenem concentration higher than the limit of detection (in absolute).

Results are presented in Table 7.

Table 7: Mean accuracy values for individual samples of second drugs in ultrafiltrated serum

| **Non-β-lactam compounds** | **Final concentrations** | **[meropenem] (mg/L)** | | | **Average [meropenem] (mg/L)** |
|---|---|---|---|---|---|
| | | Replicates | | | |
| | | 1 | 2 | 3 | |
| Actrapid | 0.6 UI/ml | 0.04 | 0.04 | 0.06 | 0.05 |
| Adrenaline | 0.008 mg/ml | 0.06 | -0.37 | 0.00 | -0.10 |
| Anidulafungin | 0.07 mg/ml | 0.09 | 0.06 | 0.14 | 0.10 |
| Calciclo | 0.022 mEQ/ml | 0.05 | -0.04 | -0.09 | -0.03 |
| Caspofungin | 0.14 mg/ml | 0.03 | 0.06 | 0.07 | 0.05 |
| Clonazepam | 0.00075 mg/ml | 0.06 | 0.06 | 0.01 | 0.05 |
| Cordarone | 0.3 mg/ml | -0.16 | -0.15 | -0.18 | -0.16 |
| Dapakine | 0.8 mg/ml | -0.10 | -0.10 | -0.09 | -0.09 |
| Diazepam | 0.0012 mg/ml | 0.05 | 0.04 | 0.01 | 0.03 |
| Diphantoïne | 0.5 mg/ml | -0.15 | -0.11 | 0.02 | -0.08 |
| Dobutrex | 0.5 mg/ml | -0.07 | -0.11 | -0.14 | -0.11 |
| Dopamine | 0.4 mg/ml | -0.05 | -0.06 | -0.07 | -0.06 |
| Fluconazole | 0.34 mg/ml | 0.02 | 0.10 | 0.11 | 0.07 |
| Heparine Leo | 50 UI/m | 0.02 | -0.05 | -0.08 | -0.04 |
| Keppra | 1 mg/ml | -0.07 | -0.07 | -0.06 | -0.07 |
| Lasix | 0.04 mg/m | -0.13 | -0.15 | -0.09 | -0.12 |
| Liposomial amphotericin B | 0.84 mg/ml | -0.04 | 0.11 | 0.05 | 0.04 |
| Midazolam Braun | 0.1 mg/ml | -0.07 | -0.09 | -0.06 | -0.07 |

(continued)

| Non-β-lactam compounds | Final concentrations | [meropenem] (mg/L) | | | Average [meropenem] (mg/L) |
|---|---|---|---|---|---|
| | | Replicates | | | |
| | | 1 | 2 | 3 | |
| Milrinone | 0.003 mg/ml | 0.14 | 0.06 | 0.06 | 0.09 |
| Morphine | 0.02 mg/ml | -0.12 | -0.15 | -0.10 | -0.13 |
| Nimbex | 0.02 mg/ml | 0.01 | -0.04 | -0.08 | -0.03 |
| Nimotop | 0.02 mg/m | 0.06 | -0.01 | 0.01 | 0.02 |
| Noradrenaline | 0.008 mg/m | -0.12 | -0.12 | -0.08 | -0.11 |
| Pantomed | 0.08 mg/ml | 0.02 | 0.01 | -0.05 | -0.01 |
| Prograft | 0.01 mg/ml | -0.07 | -0.06 | -0.02 | -0.05 |
| Propolipid | 2 mg/ml | -0.07 | -0.01 | 0.05 | -0.01 |
| Protamine | 14 UI/ml | 0.64 | 0.69 | 0.72 | 0.68 |
| Rydene | 0.01 mg/ml | 0.06 | 0.00 | 0.06 | 0.04 |
| Salbutamol | 0.0001 mg/ml | 0.16 | 0.07 | 0.05 | 0.09 |
| Sandimmum | 0.5 mg/ml | 0.09 | 0.11 | -0.02 | 0.06 |
| Solu-cortef | 0.2 mg/ml | -0.10 | 0.02 | 0.00 | -0.03 |
| Solu-Medrol | 0.08 mg/ml | -0.01 | 0.02 | 0.01 | 0.01 |
| Sufentanil | 0.0002 mg/ml | 0.00 | 0.12 | 0.12 | 0.08 |
| Thiobarbital | 0.3 mg/ml | 0.12 | 0.11 | 0.09 | 0.10 |
| Ultiva | 0.004 mg/ml | 0.10 | 0.11 | 0.14 | 0.12 |
| Voriconazole | 0.04 mg/ml | 0.00 | -0.06 | 0.10 | 0.01 |
| $H_2O$ | - | -0.01 | 0.01 | 0.00 | 0.00 |

The results show that the method illustrating the invention provided no interference with other non-beta-lactam drugs, excepted for protamine.

**Claims**

1. Method for determining the concentration of a carbapenem antibiotic in a biological sample, comprising the steps of:

   (a) providing a blaOXA-48 class D beta-lactamase (blaOXA-48) or a functionally active variant or fragment thereof;
   (b) providing a biological sample;
   (c) contacting the blaOXA-48 or functionally active variant or fragment thereof with the biological sample; and
   (d) determining the concentration of the carbapenem antibiotic in the biological sample.

2. The method according to claim 1, wherein the blaOXA-48 or functionally active variant or fragment thereof is fused with its N-terminus and/or C-terminus to a further polypeptide, or is inserted into a further polypeptide; preferably wherein the further polypeptide is an enzyme.

3. The method according to claim 1 or 2, wherein the method comprises, prior to step (c), contacting the blaOXA-48 or functionally active variant or fragment thereof with a reporter substrate.

4. The method according to any one of claims 1 to 3, wherein the measuring step (d) is performed by Ultraviolet-visible (UV-VIS) spectroscopy, fluorescence spectroscopy, fluorescence resonance energy transfer (FRET) spectroscopy, Fourier Transform Infrared (FTIR) spectroscopy, plasmon resonance, electrochemical detection (ECD), or by surface sensitive wave based technique such as quartz crystal microbalance (QCM).

5. The method according to any one of claims 1 to 4, wherein the method comprises the steps of contacting the biological sample with a reporter substrate and the blaOXA-48 or functionally active variant or fragment thereof, and measuring the amount of a spectrophotometric signal, an optical signal and/or an electrical signal in the biological sample in comparison with a standard, thereby determining the concentration of the carbapenem antibiotic in the biological sample.

6. The method according to any one of claims 3 to 5, wherein the reporter substrate is a chromogenic substrate.

7. The method according to any one of claims 1 to 6, wherein the concentration is an absolute concentration.

8. The method according to any one of claims 1 to 7, wherein the carbapenem antibiotic is one or more of meropenem, ertapenem, doripenem, or imipenem, preferably wherein the carbapenem antibiotic is meropenem.

9. The method according to any of claims 1 to 8, wherein the biological sample is selected from the group consisting of serum, blood, urine, interstitial fluid, saliva, tears, exudates, fluid collected from deep tissues, and fluid collected from subcutaneous tissues, preferably the biological sample is serum.

10. The method according to any of claims 1 to 9, wherein the biological sample comprises:

- a carbapenem antibiotic, and
- one or more other beta-lactam antibiotics and/or one or more other pharmaceutical substances.

11. The method according to any of claims 1 to 10, wherein the biological sample is a biological sample obtained from a subject receiving an antibiotherapy and/or wherein the biological sample is a biological sample obtained from a subject being treated for or in need of treatment of an infection caused by Gram-negative bacteria, Gram-positive bacteria and/or multidrug-resistant (MDR) bacteria.

12. The method according to any one of claims 1 to 11, wherein the blaOXA-48 or functionally active variant or fragment thereof is grafted on at least one surface of a device, preferably wherein said at least one surface is chemically activated.

13. The method according to claim 12, wherein the device comprises an attenuated total internal reflection element, transparent in the infrared, or the device is a quartz crystal microbalance.

14. The method according to any one of claims 1 to 13, wherein the blaOXA-48 is selected from the group consisting of beta-lactamase class D OXA-48, beta-lactamase class D OXA-54, beta-lactamase class D OXA-162, beta-lactamase class D OXA-163, beta-lactamase class D OXA-181, beta-lactamase class D OXA-199, beta-lactamase class D OXA-204, beta-lactamase class D OXA-244, beta-lactamase class D OXA-245, beta-lactamase class D OXA-247, beta-lactamase class D OXA-232, beta-lactamase class D OXA-370, beta-lactamase class D OXA-405, beta-lactamase class D OXA-416, beta-lactamase class D OXA-438, beta-lactamase class D OXA-439, beta-lactamase class D OXA-484, beta-lactamase class D OXA-436, beta-lactamase class D OXA-505, beta-lactamase class D OXA-514, beta-lactamase class D OXA-515, beta-lactamase class D OXA-517, beta-lactamase class D OXA-519, beta-lactamase class D OXA-538, beta-lactamase class D OXA-547, beta-lactamase class D OXA-546, beta-lactamase class D OXA-566, and beta-lactamase class D OXA-252; preferably wherein the blaOXA-48 is beta-lactamase class D OXA-48.

15. The method according to any one of claims 1 to 14, wherein the method is used for:

- determining the concentration of a carbapenem antibiotic in a microdialysate obtained from a subject receiving an antibiotherapy;
- for determining the concentration of a carbapenem antibiotic in a biological sample obtained from a subject to refine clinical treatment intervention on the subject, preferably wherein refining the clinical treatment intervention comprises refining one or more of the dosage, the duration, or the schedule of administration of the carbapenem antibiotic; and/or
- for determining the concentration of a carbapenem antibiotic in a biological sample obtained from a subject being treated for or in need of treatment of an infection caused by Gram-negative bacteria, Gram-positive bacteria and/or multidrug-resistant (MDR) bacteria.

Europäisches Patentamt
European Patent Office
Office européen des brevets

# EUROPEAN SEARCH REPORT

Application Number
EP 19 19 3190

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | WO 2013/053953 A1 (UNIV LIEGE [BE]; UNIV LOUVAIN [BE] ET AL.) 18 April 2013 (2013-04-18) * the whole document * ----- | 1-15 | INV. C12Q1/18 G01N33/94 |
| Y | US 2015/160211 A1 (DEVIGNE LAURENCE [FR] ET AL) 11 June 2015 (2015-06-11) * the whole document * ----- | 1-15 | |

TECHNICAL FIELDS SEARCHED (IPC)
G01N
C12Q

The present search report has been drawn up for all claims

3

| Place of search | Date of completion of the search | Examiner |
|---|---|---|
| Munich | 21 October 2019 | Stricker, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 19 19 3190

This annex lists the patent family members relating to the patent documents cited in the above-mentioned European search report.
The members are as contained in the European Patent Office EDP file on
The European Patent Office is in no way liable for these particulars which are merely given for the purpose of information.

21-10-2019

| Patent document cited in search report | Publication date | Patent family member(s) | Publication date |
|---|---|---|---|
| WO 2013053953 A1 | 18-04-2013 | CA 2851984 A1 | 18-04-2013 |
| | | EP 2766735 A1 | 20-08-2014 |
| | | US 2014243241 A1 | 28-08-2014 |
| | | WO 2013053953 A1 | 18-04-2013 |
| US 2015160211 A1 | 11-06-2015 | AU 2013294867 A1 | 29-01-2015 |
| | | CA 2878112 A1 | 30-01-2014 |
| | | CN 104508140 A | 08-04-2015 |
| | | EP 2877591 A1 | 03-06-2015 |
| | | ES 2663235 T3 | 11-04-2018 |
| | | FR 2993900 A1 | 31-01-2014 |
| | | US 2015160211 A1 | 11-06-2015 |
| | | WO 2014016534 A1 | 30-01-2014 |
| | | ZA 201500238 B | 25-05-2016 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013053953 A


### Non-patent literature cited in the description


- **FRÈRE et al.** *Eur. J. Biochem.,* 1975, vol. 57, 343-351
- **AMBLER.** *Philos. Trans. R. Soc. Lond. B. Biol. Sci.,* 1980, vol. 289, 321-31
- **BUSH et al.** *Antimicrob. Agents Chemother.,* 1995, vol. 39, 1211-33
- **POIREL et al.** *Antimicrob. Agents Chemother.,* 2004, vol. 48, 15-22
- **ALTSCHUL et al.** *J Mol Biol,* 1990, vol. 215, 403-10
- **TATUSOVA ; MADDEN.** *FEMS Microbiol Lett,* 1999, vol. 174, 247-250
- **HENIKOFF et al.** *Proc. Natl. Acad. Sci.,* 1992, vol. 89, 10915-10919
- **DOCQUIER et al.** *Chem. Biol.,* 2009, vol. 16 (5), 540-7
- **O'CALLAGHAN et al.** *Antimicrob. Agents and Chemother.,* 1972, vol. 1, 283-288